(19) Europäisches Patentamt
European Patent Office
Office européen des brevets

(11) EP 3 529 275 B1

(12) EUROPEAN PATENT SPECIFICATION

(45) Date of publication and mention of the grant of the patent:
**08.12.2021 Bulletin 2021/49**

(21) Application number: **17794919.5**

(22) Date of filing: **23.10.2017**

(51) Int Cl.:
***C07K 16/24*** *(2006.01)* ***C07K 16/28*** *(2006.01)*

(86) International application number:
**PCT/EP2017/076948**

(87) International publication number:
**WO 2018/077775 (03.05.2018 Gazette 2018/18)**

# (54) PROTEINS AND USES

PROTEINE UND VERWENDUNGEN

PROTÉINES ET UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.10.2016 GB 201617924**

(43) Date of publication of application:
**28.08.2019 Bulletin 2019/35**

(73) Proprietor: **UCB Biopharma SRL**
**1070 Brussels (BE)**

(72) Inventors:
- **WRIGHT, Michael John**
  **Slough**
  **Berkshire SL1 3WE (GB)**
- **ADAMS, Ralph**
  **Slough**
  **Berkshire SL1 3WE (GB)**
- **BURNLEY, Rebecca**
  **Slough**
  **Berkshire SL1 3WE (GB)**
- **ETTORRE, Anna**
  **Slough**
  **Berkshire SL1 3WE (GB)**

(74) Representative: **UCB Intellectual Property**
**c/o UCB Biopharma SRL**
**Intellectual Property Department**
**Allée de la Recherche 60**
**1070 Brussels (BE)**

(56) References cited:
**WO-A1-2016/012363 WO-A2-2004/033485**

- **Katarzyna Domanska ET AL: "Atomic structure of a nanobody-trapped domain-swapped dimer of an amyloidogenic [beta]2-microglobulin variant", Proceedings of the National Academy of Sciences of the United States of America, 25 January 2011 (2011-01-25), pages 1314-1319, XP055213162, DOI: 10.1073/pnas.1008560108 Retrieved from the Internet: URL:http://www.jstor.org/stable/41001858 [retrieved on 2018-01-09]**
- **RUBINSTEIN DANIEL B ET AL: "The MUC1 oncoprotein as a functional target: immunotoxin binding to alpha/beta junction mediates cell killing", INTERNATIONAL JOURNAL OF CANCER, MALDEN, MASS : WILEY-BLACKWELL, 1966-, US, vol. 124, no. 1, 1 January 2009 (2009-01-01), pages 46-54, XP002769039, ISSN: 1097-0215**
- **ALASTAIR D. G. LAWSON: "Antibody-enabled small-molecule drug discovery", NATURE REVIEWS. DRUG DISCOVERY, vol. 11, no. 7, 1 July 2012 (2012-07-01), pages 519-525, XP055440565, GB ISSN: 1474-1776, DOI: 10.1038/nrd3756**
- **RALPH ADAMS ET AL: "Discovery of a junctional epitope antibody that stabilizes IL-6 and gp80 protein:protein interaction and modulates its downstream signaling", SCIENTIFIC REPORTS, vol. 7, 30 January 2017 (2017-01-30), page 37716, XP055440202, DOI: 10.1038/srep37716**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 529 275 B1

## Description

### Field of the invention

The present invention relates to a method of producing an antibody specific for a junctional epitope created by a transient protein:protein interaction, which antibody stabilises the protein:protein interaction.

### Background of the invention

In cancer, most tumour associated antigens are also expressed on normal peripheral tissues and cells, albeit at disparate density. It is commonly believed that the dual targeting of two antigens on the same cell leads to improved target selectivity over normal tissues that express only one or low levels of both antigens.

One means of targeting two antigens on a tumour cell is via a bispecific antibody (i.e. with one arm of the antibody recognising one target antigen and the second arm of the antibody recognising a second target antigen). However, as shown in Mazor et al mAbs, 7, 461-469, dual targeting by bispecific antibodies is not always sufficient to promote efficient target selectivity. Bispecific antibodies may still recognise cells expressing only one of the two target antigens. Mazor *et al* reported that the affinity of the individual arms of the antibody, as well as avidity and valence, play an important role in targeting. For example, a bispecific antibody composed of an anti-CD4 arm and an anti-CD70 arm recognised cells expressing both CD4 and CD70, as well as CD4 alone. Affinity-reduced variants of the CD4 arm were prepared by introducing mutations into the CDRs. These variants exhibited improved selectively; binding to cells expressing CD4 alone was reduced. However, in generating such bispecific antibodies extensive engineering is required. Bispecific antibodies are also inherently specific for their particular target antigens. Different bispecific antibodies are thus needed to target different antigen combinations.

Another technique for recognising multiple antigens on a target cell is described in WO 2013/104804. Here, a targeting moiety for a first antigen "A" is linked to a non-active fragment of a functional domain. A second targeting moiety for antigen "B" is then linked to a second non-active fragment of the functional domain. When both antigens are present on the cell surface, dimerization of the two fragments results in formation of a functional domain.

One fragment may be a $V_L$ domain of an antibody and the other fragment may be a $V_H$ domain of an antibody thus resulting in an scFv when both antigens are present on the target cell. There are, however, also downsides to this approach in that $V_H$ and $V_L$ by themselves can have stability issues and the system may be partially leaky. Separate $V_H$ and $V_L$ domains may also be prone to aggregation.

There exists a need in the art to provide improved means to specifically identify and target cells (e.g. immune cells and tumour cells) with minimal off target effects.

The present inventors have identified a new system with utility, amongst other things, in specifically targeting multiple antigens on cells of interest. The system harnesses transient protein:protein interactions (PPIs), and in particular uses junctional binding proteins which recognise two components of a transient PPI.

An example of a transient PPI, which plays a fundamental role in the patho-physiology of several diseases, is the interaction between IL-6 and its specific receptor gp80, (also known as CD126). IL-6 binds to gp80 to form a heterodimer; this first step is characterized by fast association and dissociation phases (Ward et al. The Journal of biological chemistry 271, 20138-20144 (1996)). The IL-6-gp80 complex then can bind to gp130 to form a heterotrimer, which in turn dimerizes to create the active hexameric complex responsible for key downstream signaling events (Boulanger et al Science 300, 2101-2104 (2003)). IL-6 can signal in *cis* or *trans*, depending on whether gp80 is cell membrane-expressed or in a soluble form generated by shedding (Mullberg et al. European journal of immunology 23, 473-480 (1993)). Both forms of gp80 are active and bind IL-6 (Rose-John et al Journal of leukocyte biology 80, 227-236 (2006)). *In vitro* data suggest that the *trans*-signaling pathway is preferentially activated during inflammation (Rose-John et al. International journal of biological sciences 8, 1237-1247 (2012)). Unlike gp130, which is expressed in a variety of cells, gp80 is expressed mainly on the membrane of hepatocytes and immune cells (Rose-John et al Journal of leukocyte biology 80, 227-236 (2006)). Neither IL-6 nor gp80 binds to gp130, indicating that IL-6 is presented by gp80 in the appropriate conformation to then recruit gp130 (Rose-John et al Journal of leukocyte biology 80, 227-236 (2006)).

IL-6 mediates an array of immune responses including lymphocyte trafficking, proliferation and differentiation of T cells, antibody production from B cells, liver regeneration, expansion/differentiation of bone marrow progenitors and the broad regulation of inflammatory response (Galun, E. & Rose-John, S Methods in molecular biology 982, 59-77 (2013) and Mihara et al Clin Sci (Lond) 122, 143-159 (2012)). IL-6 is a key mediator in a variety of diseases, including cancer and autoimmunity (Hunter, C.A. & Jones, S.A. Nature immunology 16, 448-457 (2015)). As such, IL-6 has been targeted directly (IL-6 blocking antibodies) or indirectly (gp80 blocking antibodies; gp130-Fc) for therapeutic interventions against autoimmune disorders and cancer (Rose-John et al Expert opinion on therapeutic targets 11, 613-624 (2007), Calabrese, L.H. & Rose-John, S. Nature reviews. Rheumatology 10, 720-727 (2014), Shaw, S. et al. Discovery and characterization of olokizumab: a humanized antibody targeting interleukin-6 and neutralizing gp130-signaling. mAbs 6, 774-782 (2014), Atreya, R. et al, Nature medicine 6, 583-588 (2000), Nishimoto, N. et al. Blood 106, 2627-2632 (2005) and Milagre, C.S. et al. Cancer research 75, 1255-1264 (2015)).

Damanska et al Proc Nat Acad Sci USA 1314-1319 (2011) discloses a VHH stabilizing a dimer of a beta2-microglobulin variant.

WO 2004/033485 discloses an antibody which recognizes a discontinuous epitope on a an assembled protein, but not part of the epitope present on each individual polypeptide. WO 2016/012363 discloses an antibody specifically binding to a protein:proptein complex, but not the individual members of the complex.

Rubinstein et al Int J of Cancer 124(1) 46-54 (1996) discloses antibodies binding and stabilizing junctional epitope formed between MUC1 alpha and beta subunits.

L. Hancock PhD Thesis, University of London (2010/2011) discloses several VHH antibodies and a method of generating such, however, it does not disclose how to select an antibody that binds to a junctional epitope and stabilises transient protein:protein interaction.

## Summary of the invention

The present invention provides a method of producing an antibody specific for a junctional epitope created by a transient protein:protein interaction, which antibody stabilises the protein:protein interaction, said method comprising:

(i) immunising an animal with a fusion protein of the stabilised transient protein:protein interaction;

(ii) selecting an antibody which interacts with the stabilised complex of the protein:protein interaction but not with each individual component of the protein:protein interaction in the absence of the other; and

(iii) determining that the antibody stabilises the protein:protein interaction.

## Description of the Figures

Figure 1 exemplifies a two step targeting modality using a radiolabelled junctional antibody (oval attached to a star) with cancer cells expressing antigens A and B (lower diagrams) in comparison with a bispecific antibody (upper diagrams). In the two step targeting modality, the cells are first targeted with antibodies (each represented with four ovals), which are highly specific for the target antigens and which are fused to the components of the interaction recognised by the junctional antibody (the cut out circle and the rectangle). For example, cells are targeted with an antibody targeting antigen A fused to one protein component of the transient protein/protein interaction recognised by the junctional antibody. The cells are also targeted with a second antibody targeting antigen B fused to the second protein component of the transient protein/protein interaction recognised by the junctional antibody. This is followed by administration of the radiolabeled junctional antibody, which cross-links the two components. This system allows for specific targeting of cells expressing both antigen A and antigen B. In contrast, the bispecific antibody as shown in the upper diagrams targets cells expressing antigen A alone or antigen B alone to some extent.

Figure 2 shows how a junctional antibody could be used to switch on/off a molecular bispecific functional effect (using antibody fusions as described for Figure 1). Again, the two-step system with administration of the antibody fusions followed by the junctional antibody allow for a level of flexibility which cannot be achieved with a bispecific antibody. Addition of the junctional antibody switches on the biological effect.

Figure 3 illustrates how a junctional antibody can be used to cross-link two target cells. Once again, the junctional antibody is administered after the two targeting molecules (the antibody fusions), resulting in functional cross linking of the two target cell populations. The two-step targeting approach allows for greater control than with a bispecific antibody.

Figure 4 shows a more complex three-antigen scenario. Such a system could be used to bring two cells together, e.g. target cell with antigens A and B and an effector for cell killing (with the junctional antibody fused to an antibody which targets an antigen on the effector cell). Extra specificity is provided by requiring the two targeting antibodies to recognise the two individual antigens (A and B) on the target cell before the junctional antibody can engage and attract the effector cell. This essentially combines the models of Figures 1 and 3.

Figure 5 shows the specific targeting of e.g. cancer cells (left hand side) by an engineered effector cell (right hand side) benefitting from the junctional antibody high specificity with a dual targeting approach (to antigens A and B on the cancer cell). This example expands the use of the junctional antibody to again only specifically target a cell population with two antigens. The effector cell may be patient derived or an engineered human cell line that expresses the junctional antibody as a cell membrane fusion protein. These cells can be administered to a patient and can be long lived. Only on the administration of the two antibody-fusions would these effector cells find their junctional epitope and lead to cell killing.

Figure 6 shows an example of how a junctional antibody (labelled JEA) can be used to cross-link two antigens (Ag A and Ag B) on the surface of a single cell via antibody fusions (Ab A with component A and Ab B with component B). There are at least four individual protein protein interactions (PPIs) described which will determine how effectively the two antigens are cross-linked. Modulation of the interactions allows for fine tuning of the system.

Figure 7 shows results of the preliminary assays to prove that VHH6 only recognized IL-6-gp80 complex. (a) ELISA: FusionIL-6, IL-6 or gp80 were immobilized overnight on plates. VHH6 binds to FusionIL-6, but not to IL-6 or gp80 alone. When the plates were coated with IL-6 and VHH6 added with gp80, or VHH6 was added with IL-6 (on gp80-coated plates), the absorbance was comparable to FusionIL-6. y-axis: Absorbance 630 nm; x-axis: samples. (b) MicroScale Thermophoresis (MST): IL-6 and gp80 were labelled with NT647. A binding curve was apparent only when different concentrations of VHH6 were incubated with a mixture of IL-6-NT647+gp80 or gp80-NT647+IL-6 (bottom two panels), but not when either IL-6-NT647 or gp80-NT647 alone were probed. y-axis: thermophoresis intensity; x-axis: VHH6 concentration (nM). (c) Bio-Layer Interferometry (Octet): VHH6 was captured through its His-tag onto the Ni-tips and solutions of FusionIL-6, IL-6, gp80 and IL-6+gp80 were probed. A change of the interference pattern for VHH6 was recorded only for FusionIL-6 or IL-6+gp80 solutions. y-axis: wavelength shift (nm); x-axis: time (s). (d) SEC isolation of VHH6 in complex with IL-6 and gp80. Solutions of IL-6, gp80 and VHH6 alone and in combination. SEC on IL-6+VHH6, gp80+VHH6 give two separate peaks corresponding to the elution time of the single loaded components, while the elution time of VHH6-IL-6-gp80 was compatible with the elution of the trimeric complex when compared to the elution of the dimeric complex IL-6-gp80 (respectively, 9.378 min *vs.* 9.556 min). y-axis: Absorbance at 280 nm; x-axis: time (min).

Figure 8 shows the crystal structure of VHH6—IL-6—gp80 complex. (a) Crystal structure of VHH6 bound simultaneously to IL-6 and gp80. The epitope covers a total surface area of 924 $Å^2$, with the interface between VHH6 and IL-6 contributing 414 $Å^2$ and the interface between VHH6 and gp80 contributing 510 $Å^2$. Superimpositions of free IL-6 (1ALU) and free gp80 (1N26) backbone carbon alpha atoms over the equivalent atoms of VHH6-IL-6-gp80 showed no significant shifts in conformation with root mean squared deviation (r.m.s.d.) values of 1.2 Å and 1.5$\pm$0.1 Å, respectively. The Figure on the right shows the complex rotated by 90° around x-axis. (b) CDR1 and CDR3 make contacts with both IL-6 and gp80 whereas CDR2 contacts only gp80. (c) At the gp80 interface, CDR3 residue Ser101 and CDR2 residues Ser30 and Thr31 (both main-chain oxygen atoms) form a hydrogen bond network with Ser228 and Arg231; main chain oxygen atom of CDR2 residue Asn54 forms a hydrogen bond with the main chain nitrogen atom of Asp221; and framework residue Asn74 forms a hydrogen bond with the main chain oxygen atom of His256. (d) At the interface with IL-6, CDR3 residues Ser101 and Ile102 (main-chain nitrogen atom) hydrogen bond with Gln183; CDR3 residue Lys113 forms a salt bridge with Glu80; there is an aliphatic interaction between Tyr27 and Glu23; and CDR1 residue Tyr32 form hydrogen bonds with Ser22 (see also Figure 13 for more details).

Figure 9 shows HDX-MS of VHH6—IL-6—gp80 complex and confirms the junctional epitope identified by crystallography and indicated reduced structural dynamics at the IL-6-gp80 interface. HDX-MS was applied to map the epitope of the VHH by comparing the HDX for a purified IL-6-gp80 complex with that for a purified VHH6-IL-6-gp80 complex. After the exchange reaction, the proteins were digested by pepsin and the resulting peptides analyzed by LC/MS. Sequence coverage of 99.6% was achieved for gp80 and 94.1% for IL-6. No data were obtained for the residues 179-184 (RALRQM; SEQ ID NO: 15) at the C-terminus of IL-6, also expected to interact directly with the VHH. HDX-MS data are represented on (a) the crystal structure of VHH6—IL-6—gp80 and (b) the sequences of IL-6 and gp80. Peptides with differential-deuterium uptake in the presence and absence of the VHH6 with $p<0.01$ (Students' t-test) for $\geq 2$ time-points are defined as significant. (a) Left hand image: arrow indicates loop rigidified upon VHH binding; right hand image: arrows indicate regions involved in gp80-IL-6 interface.

(b) Regions showing a decrease in HDX-MS in the presence of VHH6 are in bold. Regions showing an increase in HDX are lighter in colour.

Figure 10 shows that binding of VHH6 to IL-6-gp80 complex changes the dissociation rate of the complex but does not affect the binding of IL-6-gp80 complex to gp130. (a) Qualitative SPR experiment was performed immobilizing IL-6 on a CM5 chip and injecting gp80. The curves represent a typical sensorgram of gp80 on IL-6; and the sensorgram resulting from gp80 on IL-6 with VHH6 injected at the beginning of the dissociation phase for gp80, showing an inhibition of gp80 dissociation curve (y-axis: RU; x-axis: time (s)). (b) Quantitative SPR was performed immobilizing IL-6 on a CM5 chip and injecting gp80. Different concentrations of gp80 were tested in the absence or in the presence of an excess of VHH6 (see Materials on line). $k_d$($k_{off}$) are reported above each sensorgram (y-axis: RU; x-axis: time (s)) (kd and $k_{off}$ are used interchangeably herein). (c) gp130 was immobilized on a CM5 chip. A fixed excess concentration of IL-6 and differing concentrations of gp80 in the presence or absence of VHH6 were tested. IL-6-gp80 complex binds gp130 with ka (kon) of $3.6\times10^5$ $M^{-1}$ $s^{-1}$and $k$d ($k_{off}$) 0.037 $s^{-1}$ while the VHH6-IL-6-gp80 complex binds gp130 with ka (kon) of $4.7\times10^5$ $M^{-1}$ $s^{-1}$and $k$d ($k_{off}$) 0.029 $s^{-1}$ (y-axis: RU; x-axis: time (s)). The data confirmed that are no significant differences neither in the association or the dissociation phase of IL-6—gp80 complex to gp130 upon addition of VHH6.

Figure 11 shows that stabilization of the complex IL-6-gp80 by VHH6 increases the internalization of IL-6-NT647 over-time and promotes higher and sustained STAT3 phosphorylation signal in HUVEC cells. HUVEC cells were treated with IL-6+gp80 and VHH6+IL-6+gp80 or IL-6-NT647+gp80 and VHH6+IL-6-NT647+gp80. Both pSTAT3 and IL-6-NT647 fluorescent signal were quantified at different time points using the 'spot total intensity per object' parameter. Three different replicates were performed for each time point (statistical significance: *=$p \leq 0.05$; **=$p \leq 0.01$ and ***=$p<0.001$). (a) A representative experiment showing pSTAT3 signal and nuclear translocation at 30 min. (Scale bar = 20 $\mu$m). (b) A representative time-course experiment showing pSTAT3 signal at 30, 180 and 360 min. (Scale bar = 100 $\mu$m). (c) Statistical analysis of pSTAT3 signal over-time showing that VHH6 promotes and increased and sustained pSTAT3 signal. y-axis: pSTAT3 fluorescence; x-axis: time (min).

IL-6 labelled with NT647 was used to track over-time internalization of the IL-6-gp80 complex. VHH6 significantly increased the rate of IL-6-NT647 accumulation in vesicles in a time-dependent manner, (d) A representative time-course experiment showing IL-6NT647 signal at 30, 180 and 360 min. (Scale bar = 20 $\mu$m). (e) Accumulation of IL-6-NT647 co-localized with LAMP-1 (t=360 min; Scale bar = 20 $\mu$m). (f) Statistical analysis of IL-6-NT647 vesicle intensity at 15, 30, 90, 180 and 360 min. y-axis: IL-6-NT647 vesicle intensity; x-axis: treatments grouped per time point.

Figure 12 shows transcriptomic analysis of HUVEC cells treated with VHH6-IL-6-gp80 and confirms selective up-regulation of pro-inflammatory genes. Threee different batches of HUVEC cells were analyzed at 30, 180 and 360 min. VHH6-IL-6-gp80 (sample) were compared to IL-6-gp80 (control). For genes which showed downregulation or upregulation, the fold changes from each batch of HUVEC cells at three different time points were plotted and clustered according to cellular function. (a) cytokines, chemokines and chemokine receptors; (b) transcription factors and cytokine regulators; (c) kinases. y-axis: fold change; x-axis: time (min). Statistical significance: *=$p \leq 0.05$; **=$p \leq 0.01$ and ***=$p \leq 0$ 001.

Figure 13 shows differential hydrogen bonding in the two copies of the crystallographic asymmetric unit. There are two copies of the VHH6—IL-6—gp80 complex in the crystallographic asymmetric unit. In one copy, IL-6 residue Ser22 forms a hydrogen bond with VHH6 residue Tyr32 (left), and in the second copy, Ser22 forms a hydrogen bond with VHH6 residue Tyr27 (right).

Figure 14 shows a model of the crystal structure of VHH6—IL-6—gp80 superimposed with gp130. Superimposition of IL-6 and gp80 from the signaling complex, IL-6—gp80—gp130 (PDB code 1P9M), showed low r.m.s.d. values of 1.4$\pm$0.2 Å and 1.35$\pm$0.05 Å respectively, indicating that VHH6 holds IL-6 and gp80 together in a form which is able to bind gp130.

Figure 15 shows that stabilization of the complex IL-6-gp80 by VHH6 promoted higher and sustained STAT3 phosphorylation signal in HUVEC cells comparable to FusionIL-6 fusion protein. HUVEC cells were treated with FusionIL-6, IL-6+gp80 and VHH6+IL-6+gp80. STAT3 phosphorylation signal was quantified at different time points using the 'spot total intensity per object' parameter. Statistical analysis of pSTAT3 signal from three replicates was performed for each time point (statistical significance: *=$p \leq 0.05$; **=$p \leq 0.01$ and ***=$p \leq 0.001$). Unlike IL-6-gp80, in the presence of VHH6 or when FusionIL-6 was added to the culture, pSTAT3 was increased at earlier (30 min) and later time points (180 min and 360 min). y-axis: pSTAT3 fluorescence expressed as spot total intensity per object; x-axis: time (min).

Figure 16 shows that transcriptomic analysis of HUVEC cells treated with VHH6-IL-6-gp80 confirmed selective up-regulation of pro-inflammatory genes. Threee different batches of HUVEC cells were analyzed at 30, 180 and 360 min. VHH6-IL-6-gp80 (sample) were compared to IL-6-gp80 (control). Data were analysed using the RT2 Profiler PCR array web based data analysis template v3.5 (http://pcrdataanalysis.sabiosciences.com/pcr/arrayanalysis) and changes in gene expression changes were calculated using the $\Delta\Delta C_t$ method with normalization of the raw data to housekeeping genes. Data from samples were compared to controls (84 genes from 3 samples and 3 control) and a heat map was generated using Genedata's Analyst software. Gene regulation for each sample is expressed as fold change when compared to control.

Figure 17 shows the correlation between $k_{on}$, $k_{off}$ and $K_D$ values. In particular, the bold diagonal line represents a $K_D$ of 10 nM. Anything to the right of the line has a higher (i.e. weaker) equilibirum dissociation constant than 10 nM. Anything to the left of the line has a lower (i.e. stronger/improved) dissociation constant than 10 nM. The diagonal line demonstrates how a high off rate (a high $k_{off}$ value, e..g $10^0$ $s^{-1}$) and high on rate (a high $k_{on}$ value e.g. $10^8$ $M^{-1}s^{-1}$), or a low off rate (a low $k_{off}$ value e.g. $10^{-6}$ $s^{-1}$) and a low on rate (a low $k_{on}$ value e.g. $10^2$ $M^{-1}s^{-1}$) can both result in a dissociation constant of 10 nM.

Figure 18 shows the alignment of Llama VHH6 sequence with humanized grafts of VHH6 variable heavy chain using IGHV3-74 human germline as the acceptor framework. CDRs are shown in bold/underlined. Donor residues are shown in bold/italic and are highlighted: F4, T24, F37, E44, R45, E46, G47, A49, Q72, V79 and A98. Modifications to CDRs are shown in bold/underlined and are highlighted: C33S, C104S.

## Description of the sequence listing

SEQ ID NOs: 1-8 show the CDR and heavy chain variable region amino acid and nucleotide sequences of the VHH6 antibody.
SEQ ID NO: 9 shows the IL-6 sequence from Figure 9 (full IL-6 sequence).
SEQ ID NO: 10 shows the gp80 sequence from Figure 9 (modified gp80 sequence as used in the Examples).
SEQ ID NOs: 11-146 show sequences featured in the Examples.
SEQ ID Nos: 147-153 show the CDRs and heavy chain variable region amino acid sequences of the humanized VHH6 antibodies.
SEQ ID NO: 154 shows the sequence of the human IGHV3-74 IGHJH4 acceptor framework.

## Detailed description of the invention

It is to be understood that different applications of the disclosed products and methods may be tailored to the specific needs in the art. It is also to be understood that the terminology used herein is for the purpose of describing particular embodiments of the invention only, and is not intended to be limiting.

In addition as used in this specification and the appended claims, the singular forms "a", "an", and "the" include plural referents unless the content clearly dictates otherwise. Thus, for example, reference to "an amino acid sequence" includes two or more such sequences, and the like.

*Binding protein specific for a junctional epitope created by a transient protein:protein interaction*

The present invention relates to the concept of a binding protein specific for a junctional epitope created by a transient protein:protein interaction, which binding protein stabilises the protein:protein interaction.

The concept of "transient protein:protein interactions" (or PPIs) is well known in the art. See, for example, Nooren and Thornton (2003) The EMBO Journal, 22, 3486-3492 and Perkins et al (2010) Structure. 18, 1233-1243. PPIs can be distinguished based on the lifetime of the complex. In contrast to a permanent interaction which is usually very stable and thus exists in its complexed form, a transient interaction associates and dissociates *in vivo.* A complex qualifies as transient if it readily undergoes changes in oligomeric state.

PPIs may be classified based on the equilibrium dissociation constant ($K_D$) which takes account of the rate of the complex dissociation ($k_{off}$) and the rate of the complex association ($k_{on}$), where:

$$K_D = \frac{k_{off}}{k_{on}}$$

$K_D$ is expressed in M, $k_{off}$ is expressed in $s^{-1}$ and $k_{on}$ is expressed in $M^{-1}s^{-1}$.

Methods of investigating protein:protein interactions include yeast two hybrid screens (e.g. repeated yeast two hybrid screens, where transient interactions are often detected in only one screen, whereas permanent interactions are detected in multiple screens) and tandem affinity purification in conjunction with mass spectrometry. Such techniques are discussed in Perkins et al (2010) Structure. 18, 1233-1243. Another technique used to characterise protein:protein interactions is NMR (see Vonigradova and Qin (2012), Top Curr Chem, 326, 35-45).

In the context of the present invention, it is preferable that surface plasmon resonance (SPR; Biacore) is used in characterising the binding kinetics of the PPI. SPR is described in the Examples, and the protocols/equipment as used in the Examples may be employed in the present invention. In the invention, the SPR experiments are typically carried out at 25 °C.

The transient PPI preferably has a weak affinity (equilibrium dissociation constant; $K_D$). The weak $K_D$ is typically characterised by a high (fast) dissociation rate ($k_{off}$). The PPI, in the context of the present invention, may be characterised by both a low association rate ($k_{on}$) and a high dissociation rate ($k_{off}$). In other words, the two protein components of the PPI preferably have low complex formation in the absence of the binding protein, and any complexes formed dissociate quickly. This high dissociation rate and low association rate makes the PPI suited to the methods described below. In some scenarios, the protein:protein interaction may also have a higher association rate, but a fast dissociation rate still means the complex dissociates rapidly, thus still making the protein:protein interaction suited to the methods described below. In other situations, it is also possible that the complex has a low dissociation rate but a low association rate means complex formation happens infrequently.

In the absence of the binding protein, the protein components of the transient PPI may have undetectable binding to one another or may have insufficient interaction to result in the downstream function of the complex. For example, the two protein components of the PPI may have undetectable binding using techniques such as gel filtration (size exclusion chromatography), ELISA or surface plasmon resonance. These techniques are discussed further below and are utilised in the Examples.

With regards to lack of downstream function, this would be determined by the nature of the PPI. The present Examples utilise the IL-6/gp80 interaction, which binds to gp130 (see above) and leads to a series of phosphorylation events, ultimately culminating in the phosphorylation of STAT3. gp130 binding or STAT3 phosphorylation could therefore be used as a measure of interaction of the IL-6 and gp80. For example, whilst the IL-6/gp interaction already has a fast off rate, the binding interface of the proteins could be engineered (residues mutated) to increase the off rate and/or decrease the on rate and/or increase the dissociation constant of the interaction (a higher dissociation constant means weaker binding). Binding might then become undetectable using the above mentioned techniques or might reduce or eliminate downstream signalling.

The protein components of the transient PPI may also have insufficient interaction in the absence of the binding protein to see an end result in a patient using the methods described below.

In the absence of the binding protein, the protein components of the transient PPI may have binding to one another when assessed by surface plasmon resonance at 25 °C which follows a similar pattern to that shown for IL-6 and gp80 in the absence of VHH6 in Figure 10. Here, the sensogram curve shows a rapid dissociation phase for the gp80 with the addition of VHH6 inhibiting the dissociation curve. Other methods which can be used to detect protein:protein interactions are described below.

The two protein components of the PPI may have an equilibrium dissociation constant ($K_D$) of greater than 1 nM, or preferably greater than 10 nM in the absence of the binding protein. More preferably, this could mean a $K_D$ of greater than 1 $\mu$M or most preferably even greater than 1 mM. These binding kinetics are typically determined by surface plasmon resonance at 25 °C. As mentioned above, the equilibrium dissociation constant may be weak enough that binding of the two components is undetectable in the absence of the binding protein.

Figure 17 illustrates the relationship between $k_{on}$, $k_{off}$ and $K_D$ values. The bold diagonal line represents a $K_D$ of 10 nM. Anything to the right of the line has a higher (i.e. weaker) dissociation constant than 10 nM. Anything to the left of the line has a lower (i.e. stronger/improved) dissociation constant than 10 nM. The diagonal line demonstrates how a high off rate (a high $k_{off}$ value, e.g $10^0$ $s^{-1}$) and high on rate (a high $k_{on}$ value e.g. $10^8$ $M^{-1}s^{-1}$), or a low off rate (a low $k_{off}$ value e.g. $10^{-6}$ $s^{-1}$) and a low on rate (a low $k_{on}$ value e.g. $10^2$ $M^{-1}s^{-1}$) can both result in a dissociation constant of 10 nM.

The transient PPI may have any appropriate $k_{on}$ and $k_{off}$ value that results in a $K_D$ of greater than 1 nM, preferably greater than 10 nM (i.e. $k_{on}$, $k_{off}$ values which result in the $K_D$ falling to the right of the 1 nM or 10 nM lines in Figure 17 respectively). It is though typical that the weak equilibrium dissociation constant is characterised by a high off rate.

The two protein components of the transient PPI preferably have an association rate ($k_{on}$) in the absence of the binding protein of less than $10^2$ $M^{-1}s^{-1}$ or even less than 10 $M^{-1}s^{-1}$. However, as discussed above, the $k_{on}$ may be higher where the PPI has a fast dissociation rate. For example, with a $K_D$ of greater than 10 nM the PPI could potentially have an association rate of less than $10^8$ $M^{-1}$ $s^{-1}$, optionally less than $10^6$ $M^{-1}$ $s^{-1}$, less than $10^4$ $M^{-1}$ $s^{-1}$ or less than $10^3$ $M^{-1}$ $s^{-1}$.

The two protein components of the transient PPI typically have a dissociation rate ($k_{off}$) in the absence of the binding protein of greater than 0.0001 $s^{-1}$ ($10^{-4}$ $s^{-1}$), preferably greater than 0.001 $s^{-1}$ ($10^{-3}$ $s^{-1}$), more preferably greater than 0.01 $s^{-1}$ ($10^{-2}$ $s^{-1}$) and most preferably greater than 0.025 $s^{-1}$. These dissociation rates may be combined with a slow association rate, such as those mentioned above, but a higher association rate with a fast dissociation rate would still make the PPI useful for the methods described below. Similarly, as discussed above, a lower dissociation rate (e.g. $10^{-7}$ $s^{-1}$, $10^{-6}$ $s^{-1}$ or $10^{-5}$ $s^{-1}$) could also be combined with a low association rate and still result in an overall weak affinity.

For example, for an equilibrium dissociation constant of greater than10 nM:
The PPI may have a dissociation rate of greater than $10^{-7}$ $s^{-1}$, typically a dissociation rate of greater than $10^{-4}$ $s^{-1}$, preferably a dissociation rate of greater than $10^{-3}$ $s^{-1}$, more preferably a dissociation rate of greater than $10^{-2}$ $s^{-1}$ and even more preferably a dissociation rate of greater than $10^{-1}$ $s^{-1}$.

In the context of the present invention, the binding protein specific for the junctional epitope created by the transient PPI stabilises the PPI. In terms of $k_{on}$ for the interaction of the two proteins of the PPI, this is typically greater than $10^2$ $M^{-1}$ $s^{-1}$, optionally greater than $10^3$ $M^{-1}$ $s^{-1}$ in the presence of the binding protein. The $k_{on}$ could also be greater than $10^4$ $M^{-1}$ $s^{-1}$ or $10^5$ $M^{-1}$ s. The $k_{off}$ for the interaction of the two proteins of the PPI is typically less than 0.01 $s^{-1}$, optionally less than 0.001 $s^{-1}$ or preferably less than 0.0005 $s^{-1}$ in the presence of the binding protein. The dissociation constant ($K_D$) for the interaction of the two proteins of the PPI is typically less than 10 nM, preferably less than 1 nM or more preferably less than 100 pM in the presence of the binding protein. Once again, these binding kinetics are typically determined by surface plasmon resonance at 25 °C .

Use of a binding protein and PPI in the methods described below is largely dependent on how much the binding protein stabilises the interaction, rather than necessarily on specific values for the $k_{off}$, $k_{on}$ and $K_D$. The $K_D$ for the protein:protein interaction may reduced (i.e. improved) at least 10 fold, at least 50 fold or even at least 100 fold in the presence of the binding protein. Even a 10 fold stabilisation of the interaction would greatly increase the therapeutic utility of a complex and thus allow for its use in one of the methods described below. Preferably, in the context of the present invention the transient PPI is characterised by a $k_{off}$ in the absence of the binding protein of greater than 0.01 $s^{-1}$, greater than 0.025 $s^{-1}$ or even greater than 0.04 $s^{-1}$. In the presence of the binding protein, the $k_{off}$ is preferably reduced to less than 0.01 $s^{-1}$, optionally less than 0.001 $s^{-1}$ or preferably less than 0.0005 $s^{-1}$. Stabilisation of the PPI by the binding protein may reduce the $k_{off}$ at least 50 fold, 100 fold or 200 fold. A decrease in $k_{off}$ of at least 10 fold could be sufficient for stabilisation of the PPI to find use in the methods described below.

Preferably, both the $k_{off}$ and the $K_D$ are reduced (improved) at least 10 fold, more preferably at least 50 fold and most preferably at least 100 fold in the presence of the binding protein.

In the context of the present invention, the transient PPI is preferably not between two domains of an antibody (i.e. between the $V_H$ and $V_L$ domains of an antibody).

The transient PPI may be, for example, the IL-6/gp 80 interaction. Other examples of suitable transient PPIs potentially include antibody: antigen interactions.

As mentioned above, a protein:protein interaction could also be engineered so that the kinetics of the interaction make it appropriate for stabilisation via a binding protein described herein and for use in the methods described below. For example, residues at the binding interface for a high affinity/low dissociation rate interaction could be mutated to reduce the affinity and/or increase the dissociation rate of the complex. The interaction may then be undetectable in the absence of the binding protein using techniques such as ELISA, gel filtration (size exclusion chromatography) or surface plasmon resonance.

In the context of the present invention, the binding protein is specific (or selective) for a junctional epitope created by the transient PPI. The binding protein specifically binds at or over the junction (interface) created when the two protein components of a transient PPI form a complex. This means that the binding protein is not allosteric, i.e. the binding protein does not recognise a site created when the two protein components of the PPI come together that is located away from the protein:protein interface.

"Selectively binds" or "selectively recognises" a transient PPI means the binding protein binds with preferential or high affinity to the PPI complex for which it is selective but does not substantially bind, or binds with very low affinity, to other proteins (e.g. the individual components of the PPI). Typically, as discussed further below, the binding protein has no detectable binding to each individual component of the protein: protein interaction.

By specific (or selective), it will be understood that the binding protein binds to the PPI of interest with no significant cross-reactivity to any other molecule, which may include the individual protein components of the PPI. Cross-reactivity may be assessed by any suitable method described herein. Cross-reactivity of a binding protein (such as an antibody) with a molecule other than the PPI may be considered significant if the binding protein binds to the other molecule at least about 10%, at least 20%, at least 30% , at least 40% or at least 50% as strongly as it binds to the PPI complex of interest. A binding protein that is specific (or selective) for the PPI may bind to another molecule at less than about 50%, less than about 40%, less than about 30%, less than about 20% or less than 10% the strength that it binds to the PPI complex. The binding protein may bind to the other molecule at less than about 20%, less than about 15%, less than about 10% or less than about 5%, less than about 2% or less than about 1% the strength that it binds to the PPI. Binding of less than 10%, less than 5% or less than 1% is preferable for binding to the individual protein components of the PPI when compared with binding to the PPI complex.

The rates at which a binding protein binds to a PPI (or other molecule) is referred to herein as the "on" rate" $k_{on\text{-}bp}$ and the rate at which the binding protein dissociates is referred to herein as the "off" rate or $k_{off\text{-}bp}$. As used herein, the symbol "$K_{D\text{-}bp}$" denotes the binding dissociation constant of a binding protein for a PPI complex (or other molecule). $K_{D\text{-}bp}$ is defined as $k_{off\text{-}bp}/k_{on\text{-}bp}$.

The $K_{D\text{-}bp}$ value of the binding protein for binding to a PPI complex of interest may be at least about 10 times, 100 times, 200 times, 300 times or 500 times lower (better) than the $K_{D\text{-}bp}$ value for binding to the individual protein components of the PPI. Such low binding would not interfere with use of the binding protein in the methods described below.

The binding affinity may be given in any appropriate units, such as $\mu$M, nM or pM. The smaller the $K_{D\text{-}bp}$ value, the larger the binding affinity of the binding protein to the PPI complex.

The decrease in the $K_{D\text{-}bp}$ value of the binding protein for binding to the PPI complex compared to the $K_{D\text{-}bp}$ value of the binding protein binding to the individual components may result from an increase in the on rate ($k_{on\text{-}bp}$) and/or a decrease in the off rate ($k_{off\text{-}bp}$).

The on rate ($k_{on\text{-}bp}$) of the binding protein binding to the PPI complex is generally increased compared to the on rate of the binding protein binding to the individual components of the PPI. The off rate ($k_{off\text{-}bp}$) of the binding protein binding to the PPI complex is generally decreased compared to the off rate of the binding protein binding to the individual components of the PPI. Most typically, the on rate is increased and the off-rate is decreased.

The $k_{on\text{-}bp}$, $k_{off\text{-}bp}$, and $K_{D\text{-}bp}$ values may be determined using any appropriate technique, but are typically determined using surface plasmon resonance at 25 °C.

The $K_{D\text{-}bp}$ value of the binding protein binding to a PPI complex may be less than 1 nM, less than 500 pM, less than 100 pM or less than 10 pM

Most preferably, the binding protein shows undetectable binding to the individual protein components of the protein:protein interaction. Methods of identifying whether a binding protein binds a complex formed by a protein:protein interaction, but not to each individual component of the protein:protein interaction are described in the Examples. Methods include ELISA, where the individual protein components and a complex of the PPI may be immobilised overnight on plates before addition of the binding protein. Other techniques which can be used to determine whether a binding protein binds a complex formed by a protein:protein interaction, but not to each individual component of the protein:protein, are size exclusion chromatography (HPLC), MicroScale Thermophoresis or Octet (Bio-Layer Interferometry). As discussed above, surface plasmon resonance may also be used. Once again, these techniques would be readily known to the skilled persona and are described in the Examples. In the context if the present invention, the binding protein may show undetectable binding to each individual component of the PPI using any one, preferably multiple, more preferably all, of the above techniques.

The binding protein binds an epitope which spans across the interface between the two protein components of the PPI complex. Between 20% and 80% of the epitope is typically present on one of the proteins of the protein: protein interaction, with the remainder of the epitope present on the other protein. Preferably, between 30% and 70% of the epitope is present on one of the proteins of the protein:protein interaction, with the remainder of the epitope present on the other protein. More preferably, between 40% and 60% of the epitope is present on one of the proteins of the protein:protein interaction, with the remainder of the epitope present on the other protein. In the present context, "epitope" refers to the amino acid residues recognised by the binding protein. In other words, between 20% and 80% of the amino acids recognised by the binding protein are typically present on one of the proteins of the protein:protein interaction, with the remainder of the amino acids recognised by the binding protein present on the other protein. Preferably, between 30% and 70% of the amino acids recognised by the binding protein are present on one of the proteins of the protein:protein interaction, with the remainder of the amino acids recognised by the binding protein present on the other protein. More preferably, between 40% and 60% of the amino acids recognised by the binding protein are present on one of the proteins of the protein:protein interaction, with the remainder of the amino acids recognised by the binding protein present on the other protein. Methods of determining a binding epitope are described in the Examples below and include crystallography and hydrogen-deuterium exchange mass spectrometry. For example, an epitope residue may be classed as a residue within 4 Å of an antibody paratope residue.

*Junctional antibody*

The binding protein in the context of the present invention is an antibody or an antibody fragment (a junctional antibody). An antibody refers to a glycoprotein comprising at least two heavy (H) chains and two light (L) chains interconnected by disulfide bonds, or an antigen-binding portion thereof. Each heavy chain is comprised of a heavy chain variable region (abbreviated herein as HCVR or $V_H$) and a heavy chain constant region. Each light chain is comprised of a light chain variable region (abbreviated herein as LCVR or $V_L$) and a light chain constant region. The variable regions of the heavy and light chains contain a binding domain that interacts with an antigen. The $V_H$ and $V_L$ regions can be further subdivided into regions of hypervariability, termed complementarity determining regions (CDR), interspersed with regions that are more conserved, termed framework regions (FR).

The constant regions of the antibodies may mediate the binding of the immunoglobulin to host tissues or factors, including various cells of the immune system (e.g., effector cells) and the first component (Clq) of the classical complement system.

An antibody will typically be a monoclonal antibody.

Antibodies generated against PPI complexes may be obtained, where immunisation of an animal is necessary, by administering the polypeptides to an animal (see below), e.g. a non-human animal, using well-known and routine protocols, see for example Handbook of Experimental Immunology, D. M. Weir (ed.), Vol 4, Blackwell Scientific Publishers, Oxford, England, 1986). Many warm-blooded animals, such as rabbits, mice, rats, sheep, cows, camels, llamas or pigs may be immunized. However, mice, rabbits, pigs and rats are typically suitable, as are camels or llamas for the generation of VHH antibodies.

Monoclonal antibodies may be prepared by the hybridoma technique (Kohler & Milstein, 1975, Nature, 256:495-497), the trioma technique, the human B-cell hybridoma technique (Kozbor et al., 1983, Immunology Today, 4:72) and the EBV-hybridoma technique (Cole et al., Monoclonal Antibodies and Cancer Therapy, pp77-96, Alan R Liss, Inc., 1985).

Antibodies may also be generated using single lymphocyte antibody methods by cloning and expressing immunoglobulin variable region cDNAs generated from single lymphocytes selected for the production of specific antibodies by for example the methods described by Babcook, J. et al., 1996, Proc. Natl. Acad. Sci. USA 93(15): 7843-78481;

WO92/02551; WO2004/051268 and WO2004/106377.

Antibodies can also be generated using various phage display methods known in the art and include those disclosed by Brinkman et al. (in J. Immunol. Methods, 1995, 182: 41-50), Ames et al. (J. Immunol. Methods, 1995, 184:177-186), Kettleborough et al. (Eur. J. Immunol. 1994, 24:952-958), Persic et al. (Gene, 1997 187 9-18), Burton et al. (Advances in Immunology, 1994, 57:191-280) and WO 90/02809; WO 91/10737; WO 92/01047; WO 92/18619; WO 93/11236; WO 95/15982; WO 95/20401; and US 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108.

Fully human antibodies are those antibodies in which the variable regions and the constant regions (where present) of both the heavy and the light chains are all of human origin, or substantially identical to sequences of human origin, but not necessarily from the same antibody. Examples of fully human antibodies may include antibodies produced, for example by the phage display methods described above and antibodies produced by mice in which the murine immunoglobulin variable and optionally the constant region genes have been replaced by their human counterparts e.g. as described in general terms in EP 0546073, US 5,545,806, US 5,569,825, US 5,625,126, US 5,633,425, US 5,661,016, US 5,770,429, EP 0438474 and EP 0463151.

Antibody molecules in the context of the present invention may comprise a complete antibody molecule having full length heavy and light chains or a fragment or antigen-binding portion thereof. The term "antigen-binding portion" of an antibody refers to one or more fragments of an antibody that retain the ability to selectively bind to an antigen. It has been shown that the antigen-binding function of an antibody can be performed by fragments of a full-length antibody. Antibodies and fragments and antigen binding portions thereof include Fab, modified Fab, Fab', modified Fab', $F(ab')_2$, Fv, single domain antibodies (e.g. VH or VL or VHH), scFv, bi, tri or tetra-valent antibodies, Bis-scFv, diabodies, triabodies, tetrabodies and epitope-binding fragments of any of the above (see for example Holliger and Hudson, 2005, Nature Biotech. 23(9): 1126-1136; Adair and Lawson, 2005, Drug Design Reviews - Online 2(3), 209-217). The methods for creating and manufacturing these antibody fragments are well known in the art (see for example Verma et al., 1998, Journal of Immunological Methods, 216, 165-181). Other antibody fragments include the Fab and Fab' fragments described in International patent applications WO 2005/003169, WO 2005/003170 and WO 2005/003171 and Fab-dAb fragments described in International patent application WO2009/040562. These antibody fragments may be obtained using conventional techniques known to those of skill in the art, and the fragments may be screened for utility in the same manner as intact antibodies.

In the context of the present invention, the antibody is preferably a Fab, scFV or VHH antibody. These types of antibody have short serum half lives and rapid renal clearance, so are useful in the therapeutic methods described below. Other suitable antibody formats include fynomers, human nanobodies and shark IgNARs.

The constant region domains of the antibody molecule, if present, may be selected having regard to the proposed function of the antibody molecule, and in particular the effector functions which may be required. For example, the constant region domains may be human IgA, IgD, IgE, IgG or IgM domains. In particular, human IgG constant region domains may be used, especially of the IgG1 and IgG3 isotypes when the antibody molecule is intended for therapeutic uses and antibody effector functions are required. Alternatively, IgG2 and IgG4 isotypes may be used when the antibody molecule is intended for therapeutic purposes and antibody effector functions are not required.

An antibody may be prepared, expressed, created or isolated by recombinant means, such as (a) antibodies isolated from an animal (e.g., a mouse) that is transgenic or transchromosomal for the immunoglobulin genes of interest or a hybridoma prepared therefrom, (b) antibodies isolated from a host cell transformed to express the antibody of interest, e.g., from a transfectoma, (c) antibodies isolated from a recombinant, combinatorial antibody library, and (d) antibodies prepared, expressed, created or isolated by any other means that involve splicing of immunoglobulin gene sequences to other DNA sequences.

An antibody may be a human antibody or a humanised antibody. The term "human antibody", as used herein, is intended to include antibodies having variable regions in which both the framework and CDR regions are derived from human germline immunoglobulin sequences. Furthermore, if the antibody contains a constant region, the constant region also is derived from human germline immunoglobulin sequences. The human antibodies may include amino acid residues not encoded by human germline immunoglobulin sequences (e.g., mutations introduced by random or site-specific mutagenesis *in vitro* or by somatic mutation *in vivo*). However, the term "human antibody", as used herein, is not intended to include antibodies in which CDR sequences derived from the germline of another mammalian species, such as a mouse, have been grafted onto human framework sequences.

Such a human antibody may be a human monoclonal antibody. Such a human monoclonal antibody may be produced by a hybridoma that includes a B cell obtained from a transgenic nonhuman animal, e.g., a transgenic mouse, having a genome comprising a human heavy chain transgene and a light chain transgene fused to an immortalized cell.

Human antibodies may be prepared by *in vitro* immunisation of human lymphocytes followed by transformation of the lymphocytes with Epstein-Barr virus.

The term "human antibody derivatives" refers to any modified form of the human antibody, e.g., a conjugate of the antibody and another agent or antibody.

The term "humanized antibody" is intended to refer to CDR-grafted antibody molecules in which CDR sequences derived from the germline of another mammalian species, such as a mouse, have been grafted onto human framework sequences. Additional framework region modifications may be made within the human framework sequences.

As used herein, the term 'CDR-grafted antibody molecule' refers to an antibody molecule wherein the heavy and/or light chain contains one or more CDRs (including, if desired, one or more modified CDRs) from a donor antibody (e.g. a murine or rat monoclonal antibody) grafted into a heavy and/or light chain variable region framework of an acceptor antibody (e.g. a human antibody). For a review, see Vaughan et al, Nature Biotechnology, 16, 535-539, 1998. In some cases rather than the entire CDR being transferred, only one or more of the specificity determining residues from any one of the CDRs described herein above are transferred to the human antibody framework (see for example, Kashmiri et al., 2005, Methods, 36, 25-34). In some circumstances only the specificity determining residues from one or more of the CDRs described herein above are transferred to the human antibody framework. In other circumstances only the specificity determining residues from each of the CDRs described herein above are transferred to the human antibody framework.

When the CDRs or specificity determining residues are grafted, any appropriate acceptor variable region framework sequence may be used having regard to the class/type of the donor antibody from which the CDRs are derived, including mouse, primate and human framework regions. Suitably, the CDR-grafted antibody has a variable domain comprising human acceptor framework regions as well as one or more of the CDRs or specificity determining residues described above. Thus, provided in one aspect of the disclosure is a neutralising CDR-grafted antibody wherein the variable domain comprises human acceptor framework regions and non-human donor CDRs.

Examples of human frameworks which can be used are KOL, NEWM, REI, EU, TUR, TEI, LAY and POM (Kabat *et al.*, *supra*). For example, KOL and NEWM can be used for the heavy chain, REI can be used for the light chain and EU, LAY and POM can be used for both the heavy chain and the light chain. Alternatively, human germline sequences may be used; these are available for example at: http://www.vbase2.org/ (see Retter et al, Nucl. Acids Res. (2005) 33 (supplement 1), D671-D674).

In a CDR-grafted antibody, the acceptor heavy and light chains do not necessarily need to be derived from the same antibody and may, if desired, comprise composite chains having framework regions derived from different chains.

Also, in a CDR-grafted antibody, the framework regions need not have exactly the same sequence as those of the acceptor antibody. For instance, unusual residues may be changed to more frequently occurring residues for that acceptor chain class or type. Alternatively, selected residues in the acceptor framework regions may be changed so that they correspond to the residue found at the same position in the donor antibody (see Reichmann et al., 1998, Nature, 332, 323-324). Such changes should be kept to the minimum necessary to recover the affinity of the donor antibody. A protocol for selecting residues in the acceptor framework regions which may need to be changed is set forth in WO 91/09967.

It will also be understood by one skilled in the art that antibodies may undergo a variety of posttranslational modifications. The type and extent of these modifications often depends on the host cell line used to express the antibody as well as the culture conditions. Such modifications may include variations in glycosylation, methionine oxidation, diketopiperazine formation, aspartate isomerization and asparagine deamidation. A frequent modification is the loss of a carboxy-terminal basic residue (such as lysine or arginine) due to the action of carboxypeptidases (as described in Harris, RJ. Journal of Chromatography 705:129-134, 1995).

The antibody heavy chain may comprise a CH1 domain and the antibody light chain comprises a CL domain, either kappa or lambda.

Biological molecules, such as antibodies or fragments, contain acidic and/or basic functional groups, thereby giving the molecule a net positive or negative charge. The amount of overall "observed" charge will depend on the absolute amino acid sequence of the entity, the local environment of the charged groups in the 3D structure and the environmental conditions of the molecule. The isoelectric point (pI) is the pH at which a particular molecule or surface carries no net electrical charge. In some cases the antibody or fragment according to the present disclosure has an isoelectric point (pI) of at least 7. In some cases the antibody or fragment has an isoelectric point of at least 8, such as 8.5, 8.6, 8.7, 8.8 or 9. In some cases the pI of the antibody is 8. Programs such as ** ExPASY http://www.expasy.ch/tools/pi_tool.html_(see Walker, The Proteomics Protocols Handbook, Humana Press (2005), 571-607) may be used to predict the isoelectric point of the antibody or fragment.

The binding protein may not be a bispecific antibody, where one arm of the bispecific antibody contacts one protein component of the PPI and the second arm of the bispecific antibody contacts the second protein component of the PPI.

Typically, a single heavy chain variable region of the antibody or fragment thereof has interactions with both proteins of the transient PPI, a single light chain variable region has interactions with both proteins of the transient PPI or both single heavy and light chain variable regions have interactions with both proteins of the transient PPI. In other words, a single heavy chain variable region of the antibody or fragment thereof contacts both proteins of the transient PPI, a single light chain variable region contacts both proteins of the transient PPI or both single heavy and light chain variable regions contact both proteins of the transient PPI. This can be determined by X-ray crystallography, where contact may be defined as residues within 4Å of an antibody paratope residue. Mutational analysis and analysing changes in binding kinetics may additionally be used.

Preferably, one or more individual CDRs of the antibody interact with both proteins of the PPI. For example, one of the CDRs of the heavy chain, possibly two CDRs of the heavy chain, or even all three CDRs of the heavy chain may interact with both proteins of the PPI. Likewise, one of the CDRs of the light chain, possibly two CDRs of the light chain, or even all three CDRs of the light chain may interact with both proteins of the PPI. In other words, one or more individual CDRs of the antibody contact both proteins of the PPI. For example, one of the CDRs of the heavy chain, possibly two CDRs of the heavy chain, or even all three CDRs of the heavy chain may contact both proteins of the PPI. Likewise, one of the CDRs of the light chain, possibly two CDRs of the light chain, or even all three CDRs of the light chain may contact both proteins of the PPI. Once again, this can be determined by X-ray crystallography (where contact may be defined as residues within 4Å of the antibody paratope residue), optionally in combination with mutational analysis and measuring changes in binding kinetics.

Where the PPI is the IL-6/gp80 interaction, the antibody or fragment of the disclosure may comprise at least one heavy chain CDR sequence selected from SEQ ID NOs: 2-4. These are the CDRs of the VHH6 antibody described in the Examples. The antibody may comprise a HCDR3 of SEQ ID NO: 4. The antibody may additionally comprise a HCDR1 of SEQ ID NO: 2 and/or a HCDR2 of SEQ ID NO: 3.

The antibody of the disclosure may also comprise the CDR sequences present in SEQ ID NO: 1 (the $V_H$ of VHH6) as determined by the Kabat or Chothia methods. These methods are well known in the art and would be readily understood by the skilled person.

The antibody of the disclosure may also comprise a $V_H$ of SEQ ID NO: 1

Alternatively the antibody of the disclosure is a humanized VHH6 antibody. Specifically, said antibody may comprise at least at least one heavy chain CDR sequence selected from SEQ ID NOs: 147 and 148. The antibody may comprise a HCDR3 of SEQ ID NO: 152 or 153. The antibody may additionally comprise a HCDR1 of SEQ ID NO: 149 or 150 and/or a HCDR2 of SEQ ID NO: 151. More specifically said antibody comprises HCDR1 of SEQ ID NO: 149, HCDR2 of SEQ ID NO: 151 and HCDR3 of SEQ ID NO: 152. Alternatively such antibody comprises HCDR1 of SEQ ID NO: 150, HCDR2 of SEQ ID NO: 151 and HCDR3 of SEQ ID NO: 153.The antibody of the disclosure may alternatively be or may comprise a variant of one of the specific sequences recited above. For example, a variant may be a substitution, deletion or addition variant of any of the above amino acid sequences.

A variant antibody may comprise 1, 2, 3, 4, 5, up to 10, up to 20 or more amino acid substitutions and/or deletions from the specific sequences discussed above. "Deletion" variants may comprise the deletion of individual amino acids, deletion of small groups of amino acids such as 2, 3, 4 or 5 amino acids, or deletion of larger amino acid regions, such as the deletion of specific amino acid domains or other features. "Substitution" variants typically involve the replacement of one or more amino acids with the same number of amino acids and making conservative amino acid substitutions. For example, an amino acid may be substituted with an alternative amino acid having similar properties, for example, another basic amino acid, another acidic amino acid, another neutral amino acid, another charged amino acid, another hydrophilic amino acid, another hydrophobic amino acid, another polar amino acid, another aromatic amino acid or another aliphatic amino acid. Some properties of the 20 main amino acids which can be used to select suitable substituents are as follows:

| | | | |
|---|---|---|---|
| Ala | aliphatic, hydrophobic, neutral | Met | hydrophobic, neutral |
| Cys | polar, hydrophobic, neutral | Asn | polar, hydrophilic, neutral |
| Asp | polar, hydrophilic, charged (-) | Pro | hydrophobic, neutral |
| Glu | polar, hydrophilic, charged (-) | Gln | polar, hydrophilic, neutral |
| Phe | aromatic, hydrophobic, neutral | Arg | polar, hydrophilic, charged (+) |
| Gly | aliphatic, neutral | Ser | polar, hydrophilic, neutral |
| His | aromatic, polar, hydrophilic, charged (+) | Thr | polar, hydrophilic, neutral |
| Ile | aliphatic, hydrophobic, neutral | Val | aliphatic, hydrophobic, neutral |
| Lys | polar, hydrophilic, charged(+) | Trp | aromatic, hydrophobic, neutral |
| Leu | aliphatic, hydrophobic, neutral | Tyr | aromatic, polar, hydrophobic |

"Derivatives" or "variants" generally include those in which instead of the naturally occurring amino acid the amino acid which appears in the sequence is a structural analog thereof. Amino acids used in the sequences may also be derivatized or modified, e.g. labelled, providing the function of the antibody is not significantly adversely affected.

Derivatives and variants as described above may be prepared during synthesis of the antibody or by post-production modification, or when the antibody is in recombinant form using the known techniques of site- directed mutagenesis, random mutagenesis, or enzymatic cleavage and/or ligation of nucleic acids.

A variant antibody of the disclosure may have a $V_H$ sequence at least 90% (preferably 95%) identical to SEQ ID NO: 1. A variant may have at least 90% or 95% identity to SEQ ID NO: 1, but retaining the exact CDR sequences of SEQ ID NOs: 2, 3 and 4.

A variant antibody of the disclosure may have a $V_H$ sequence at least 90% (preferably 95%) identical to SEQ ID NO: 147 or 148. A variant may have at least 90% or 95% identity to SEQ ID NO: 147 or 148, but retaining the corresponding exact CDR sequences of SEQ ID NOs: 149-153.

This level of amino acid identity may be seen across the full length of the relevant SEQ ID NO sequence or over a part of the sequence, such as across about 20, 30, 50, 75, 100, 150, 200 or more amino acids, depending on the size of the full length polypeptide. Typically, identity is measured over the full length of the relevant sequence.

In connection with amino acid sequences, "sequence identity" refers to sequences which have the stated value when assessed using ClustalW (Thompson *et al.*, 1994, supra) with the following parameters:

Pairwise alignment parameters -Method: accurate, Matrix: PAM, Gap open penalty: 10.00, Gap extension penalty: 0.10;

Multiple alignment parameters -Matrix: PAM, Gap open penalty: 10.00, % identity for delay: 30, Penalize end gaps: on, Gap separation distance: 0, Negative matrix: no, Gap extension penalty: 0.20, Residue-specific gap penalties: on, Hydrophilic gap penalties: on, Hydrophilic residues: GPSNDQEKR. Sequence identity at a particular residue is intended to include identical residues which have simply been derivatized.

Antibodies having specific sequences and variants which maintain the function or activity of these chains are therefore provided by the present disclosure.

A polynucleotide sequence of SEQ ID NO: 5 encodes the $V_H$ of SEQ ID NO: 1.

The present disclosure also provides an isolated DNA sequence encoding the heavy and/or light chain(s) of any antibody molecule of the present disclosure. Suitably, the DNA sequence encodes the heavy or the light chain of an antibody molecule of the present disclosure.

A suitable polynucleotide sequence may alternatively be a variant of one of these specific polynucleotide sequences. For example, a variant may be a substitution, deletion or addition variant of any of the above nucleic acid sequences. A variant polynucleotide may comprise 1, 2, 3, 4, 5, up to 10, up to 20, up to 30, up to 40, up to 50, up to 75 or more nucleic acid substitutions and/or deletions from the sequences given in the sequence listing. Generally, a variant has 1- 20, 1-50, 1-75 or 1-100 substitutions and/or deletions.

Suitable variants may be at least about 70% homologous to a polynucleotide of any one of nucleic acid sequences disclosed herein, typically at least about 80 or 90% and more suitably at least about 95%, 97% or 99% homologous thereto. Variants may retain at least about 90%, 91%, 92%, 93%, 94%, 95%, 96%, 97%, 98% or 99% identity. Variants typically retain about 60% - about 99% identity, about 80% - about 99% identity, about 90% - about 99% identity or about 95% - about 99% identity. Homology and identity at these levels is generally present at least with respect to the coding regions of the polynucleotides. Methods of measuring homology are well known in the art and it will be understood by those of skill in the art that in the present context, homology is calculated on the basis of nucleic acid identity. Such homology may exist over a region of at least about 15, at least about 30, for instance at least about 40, 60, 100, 200 or more contiguous nucleotides (depending on the length). Such homology may exist over the entire length of the unmodified polynucleotide sequence.

Methods of measuring polynucleotide homology or identity are known in the art. For example the UWGCG Package provides the BESTFIT program which can be used to calculate homology (e.g. used on its default settings) (Devereux et al (1984) Nucleic Acids Research 12, p387-395).

The PILEUP and BLAST algorithms can also be used to calculate homology or line up sequences (typically on their default settings), for example as described in Altschul S.F. (1993) J Mol Evol 36:290-300; Altschul, S, F et al (1990) J Mol Biol 215:403-10.

Software for performing BLAST analysis is publicly available through the National Centre for Biotechnology Information (http://www.ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al,* supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89:10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90:5873-5787. One measure of similarity provided by the BLAST algorithm is the smallest sum probability (P(N)), which provides an indication of the probability by which a match between two nucleotide or amino acid sequences would occur by chance. For example, a sequence is considered similar to another sequence if the smallest sum probability in comparison of the first sequence to the second sequence is less than about 1, typically less than about 0.1, suitably less than about 0.01, and most suitably less than about 0.001. For example, the smallest sum probability may be in the range of about 1 - about 0.001, often about 0.01 - about 0.001.

The homologue may differ from a sequence in the relevant polynucleotide by less than about 3, 5, 10, 15, 20 or more mutations (each of which may be a substitution, deletion or insertion). For example, the homologue may differ by 3-50 mutations, often 3-20 mutations. These mutations may be measured over a region of at least 30, for instance at least about 40, 60 or 100 or more contiguous nucleotides of the homologue.

A variant sequence may vary from the specific sequences given in the sequence listing by virtue of the redundancy in the genetic code. The DNA code has 4 primary nucleic acid residues (A, T, C and G) and uses these to "spell" three letter codons which represent the amino acids the proteins encoded in an organism's genes. The linear sequence of codons along the DNA molecule is translated into the linear sequence of amino acids in the protein(s) encoded by those genes. The code is highly degenerate, with 61 codons coding for the 20 natural amino acids and 3 codons representing "stop" signals. Thus, most amino acids are coded for by more than one codon - in fact several are coded for by four or more different codons. A variant polynucleotide may therefore encode the same polypeptide sequence as another polynucleotide, but may have a different nucleic acid sequence due to the use of different codons to encode the same amino acids.

The DNA sequence may comprise synthetic DNA, for instance produced by chemical processing, cDNA, genomic DNA or any combination thereof.

DNA sequences which encode an antibody molecule can be obtained by methods well known to those skilled in the art. For example, DNA sequences coding for part or all of the antibody heavy and light chains may be synthesised as desired from the determined DNA sequences or on the basis of the corresponding amino acid sequences.

General methods by which the vectors may be constructed, transfection methods and culture methods are well known to those skilled in the art. In this respect, reference is made to "Current Protocols in Molecular Biology", 1999, F. M. Ausubel (ed), Wiley Interscience, New York and the Maniatis Manual produced by Cold Spring Harbor Publishing.

Also provided by the present disclosure is a host cell comprising one or more cloning or expression vectors comprising one or more DNA sequences encoding an antibody. Any suitable host cell/vector system may be used for expression of the DNA sequences encoding the antibody molecule. Bacterial, for example *E. coli,* and other microbial systems may be used or eukaryotic, for example mammalian, host cell expression systems may also be used. Suitable mammalian host cells include CHO, myeloma or hybridoma cells.

The present disclosure also provides a process for the production of an antibody molecule according to the present disclosure comprising culturing a host cell containing a vector of the present disclosure under conditions suitable for leading to expression of protein from DNA encoding the antibody molecule of the present disclosure, and isolating (collecting) the antibody molecule.

Antibodies may bind to the same epitope, or compete for binding with the VHH6 antibody of the disclosure. Residues in the VHH6 epitope are identified in the Examples and Figures. In particular, the VHH6 epitope comprises residues D221, S228, R231 and H256 of gp80, and residues S22, E23, E80 and Q183 of IL-6. An antibody may bind an epitope which comprises at least one of these residues from gp80 and at least one of the residues from IL-6, preferably at least two or at least three residues from both IL-6 and gp80. Most preferably, an antibody of the disclosure binds an epitope with all of the above residues. Antibody epitopes may be determined by x-ray crystallography analysis. Antibodies of the present disclosure may therefore be assessed through x-ray crystallogray analysis of the antibody bound to the IL-6/gp80 complex. Epitopes may, in particular, be identified in this way by determining residues on IL-6/gp80 within 4Å of an antibody paratope residue.

One can easily determine whether an antibody binds to the same epitope as, or competes for binding with, a reference antibody by using other routine methods known in the art. For example, to determine if a test antibody binds to the same epitope as a reference antibody of the disclosure, the reference antibody is allowed to bind to a protein or peptide under saturating conditions. Next, the ability of a test antibody to bind to the protein or peptide is assessed. If the test antibody is able to bind to the protein or peptide following saturation binding with the reference antibody, it can be concluded that the test antibody binds to a different epitope than the reference antibody. On the other hand, if the test antibody is not able to bind to protein or peptide following saturation binding with the reference antibody, then the test antibody may bind to the same epitope as the epitope bound by the reference antibody of the disclosure.

To determine if an antibody competes for binding with a reference antibody, the above-described binding methodology is performed in two orientations. In a first orientation, the reference antibody is allowed to bind to a protein/peptide under saturating conditions followed by assessment of binding of the test antibody to the protein/peptide molecule. In a second orientation, the test antibody is allowed to bind to the protein/peptide under saturating conditions followed by assessment of binding of the reference antibody to the protein/peptide. If, in both orientations, only the first (saturating) antibody is capable of binding to the protein/peptide, then it is concluded that the test antibody and the reference antibody compete for binding to the protein/peptide. As will be appreciated by the skilled person, an antibody that competes for binding with a reference antibody may not necessarily bind to the identical epitope as the reference antibody, but may sterically block binding of the reference antibody by binding an overlapping or adjacent epitope.

Two antibodies bind to the same or overlapping epitope if each competitively inhibits (blocks) binding of the other to the antigen. That is, a 1-, 5-, 10-, 20- or 100-fold excess of one antibody inhibits binding of the other by at least 50%, 75%, 90% or even 99% as measured in a competitive binding assay (see, e.g., Junghans et al., Cancer Res, 1990:50:1495-1502). Alternatively, two antibodies have the same epitope if essentially all amino acid mutations in the antigen that reduce or eliminate binding of one antibody reduce or eliminate binding of the other. Two antibodies have overlapping epitopes if some amino acid mutations that reduce or eliminate binding of one antibody reduce or eliminate binding of the other.

Additional routine experimentation (e.g., peptide mutation and binding analyses) can then be carried out to confirm whether the observed lack of binding of the test antibody is in fact due to binding to the same epitope as the reference antibody or if steric blocking (or another phenomenon) is responsible for the lack of observed binding. Experiments of this sort can be performed using ELISA, RIA, surface plasmon resonance, flow cytometry or any other quantitative or qualitative antibody-binding assay available in the art.

To screen for antibodies that bind to a particular epitope, a routine cross-blocking assay such as that described in Antibodies, Harlow and Lane (Cold Spring Harbor Press, Cold Spring Harb., NY) can be performed. Other methods include alanine scanning mutants, peptide blots (Reineke (2004) Methods Mol Biol 248:443-63), or peptide cleavage analysis. In addition, methods such as epitope excision, epitope extraction and chemical modification of antigens can be employed (Tomer (2000) Protein Science 9: 487-496). Such methods are well known in the art.

Binding to the same epitope or competitive binding could also be determined using X-ray crystallography as mentioned above.

A binding protein of the disclosure does not have to be an antibody. A binding protein could be any type of protein which can be selected for binding to the transient protein:protein interaction. For example, the binding protein may comprise ankyrin repeat domains.

*Use of a binding protein specific for a junctional epitope created by a transient protein:protein interaction in delivering cargo to a target cell*

The disclosure also provides use of a binding protein as described above in delivering cargo to a target cell. The cargo can be a diagnostic or therapeutic compound. The target cell can be any appropriate cell type, but is typically a cancer cell.

Examples of cancer cells include cells of a haematologic tumour, such as cells selected from the group consisting of acute myeloic leukemia cells, chronic myeloic leukemia cells, acute lymphatic leukemia cells, chronic lymphatic leukemia cells, lymphoma cells, myeloproliferative syndrome cells, myelodysplasia cells, myeloma cells, cells of a non-haematologic tumour, such asa cell selected from the group consisting of renal cell carcinoma cells, bladder cancer cells, lung cancer cells, mesothelioma cells, prostate cancer cells, brain cancer cells, bone cancer cells, sarcoma cells, soft tissue cancer cells, ovarian cancer cells, cervix cancer cells, breast cancer cells, endometrial cancer cells, uterine cancer cells, germ cell tumour cells, anal cancer cells, rectal carcinoma cells, colon carcinoma cells, small intestine carcinoma cells, gastric carcinoma cells, gastrointestinal stroma tumour cells, liver carcinoma cells, pancreas carcinoma cells, bile duct carcinoma cells, gall bladder carcinoma cells, head and neck cancer cells, hypopharyngeal cancer cells, laryngeal cancer cells, cells of a cancer of the esophagus, skin cancer cells, preferably melanoma cells, cells of a childhood cancer, cells of an endocrine tumour, cells of a carcinoid tumour, thymoma cells, thyroid cancer cells, cells of an islet cell tumour, cells of an adrenal cell tumour, cells of a neuroendocrine tumour and cells of a cancer of unknown primary (cancer of unknown primary origin).

As discussed above, the present disclosure provides a more specific means for targeting cargo to a cell. This method involves recognition of two different target molecules on the target cell and then delivery of the cargo via the binding protein disclosed herein. The target molecules may for example be antigens or receptors, such as tumour antigens on a tumour cell. The term "antigen" generally encompasses molecules capable of being recognised by an antibody. The term "antigen" may encompass receptors. However, receptors are also specifically discussed herein as the targeting portion of the targeting molecule may be a ligand for a receptor (rather than an antibody).

The combination of both antigens (or receptors) may only be found on the target (e.g. tumour cells) or expressed on cells that are not target cells (i.e. non-cancerous cells) to a negligible extent. Both tumour antigens may be specific for cancerous cells, and for a certain type of cancer.

Antigen pairings are provided for example in WO 2013/104804, such as:

(i) one of said antigens is EpCAM and the other one is EGFR, HER2/neu, CD 10, VEGF-R or MDR;
(ii) one of said antigens is MCSP and the other one is melanoferrin or EpCAM;
(iii) one of said antigens is CA125 and the other one CD227
(iv) one of said antigens is CD56 and the other one is CD 140b or GD3 ganglioside;
(v) one of said antigens is EGFR and the other one is HER2;
(vi) one of said antigens is PSMA and the other one is HER2;
(vii) one of said antigens is Sialyl Lewis and the other one is EGFR;
(viii) one of said antigens is CD44 and the other one is ESA, CD24, CD133, MDR or CD1 17;
(ix) one of said antigens is CD34 and the other one is CD19, CD79a, CD2, CD7, HLA-DR, CD13, CD1 17, CD33 or CD15;
(x) one of said antigens is CD33 and the other one is CD 19, CD79a, CD2, CD7, HLA-DR, CD13, CD117 or CD15;
(xi) one of said antigens is MUC1 and the other one is CD10, CEA or CD57;
(xii) one of said antigens is CD38 and the other one is CD 138;
(xiii) one of said antigens is CD 24 and the other one is CD29 or CD49f;
(xiv) one of said antigens is carbonic anhydrase IX and the other one is aquaporin-2;
(xv) one of said antigens is HLA-A2 and the other one is EpCAM;
(xvi) one of said antigens is HLA-A2 and the other one is CD45;
(xvii) one of said antigens is HLA-A2 and the other one is EGFR;
(xviii) one of said antigens is HLA-A2 and the other one is Her2;
(xix) one of said antigens is HLA-A2 and the other one is CEA;
(xx) one of said antigens is EpCAM and the other one is CEA;
(xxi) one of said antigens is CD45 and the other one is CD 138;
(xxii) one of said antigens is EGFR and the other one is CEA;
(xxiii) one of said antigens is Her2 and the other one is CEA; or
(xxiv) one of said antigens is CD19 and the other one is a clonotypic antibody on the surface of a B cell.

Other antigens expressed on different cell types are well known in the art and could also be identified using routine experimentation. Examples of markers for the malignant state of a cell include: E-cadherin for epithelial cells and ductal-type breast carcinoma cells; Ca-125 for Epitheloid malignancies and ovary cancer cells, adenocarcinoma cells and breast cancer cells; Her-2/neu for breast cancer cells; gross cystic disease fluid protein (BRST-2 protein) for breast cancer cells; BCA-225 (breast carcinoma associated glycoprotein) for lung and breast cancer cells; CA 19-9 (carbohydrate antigen 19-9) for pancreas, bile duct and intestinal tract cancer cells; CEA for colorectal cancer cells; CD117 (c-kit) for gist (gastrointestinal stromal tumour) cells ( and myeloid and mast cells); CD30 for Reed-Sternberg cells (and i-1 activated T-cells and B-cells); Epithelial antigen (BER-EP4), Epithelial membrane antigen, and Epithelial Related Antigen (MOC-31) for epithelial cancer cells; Epidermal growth factor receptor (HER1) for cells of various cancers; Platelet derived growth factor receptor (PDGFR) alpha for cells of various cancers; Melanoma associated marker/Mart 1/Melan-A for melanoma cells; CD133 for cancer stem cell populations and others; TAG 72 (tumour associated gp 72) for adenocarcinoma cells.

Further examples for markers for a malignant state of a cell/cells include: EpCAM, CD 19, HER-2, HER-3, HER-4, PSMA, MUC-1 (mucin), MUC2, MUC3, MUC4, MUC5AC, MUC5B, MUC7, Lewis-Y, CD20, CD33, CD44v6, Wue-1, Plasma Cell Antigen, (membrane-bound) IgE, Melanoma Chondroitin Sulfate Proteoglycan (MCSP), STEAP, mesothelin, Prostate Stem Cell Antigen (PSCA), sTn (sialylated Tn antigen), FAP (fibroblast activation antigen), EGFRvIII, Iga, Ig, MT-MMPs, Cora antigen, EphA2, L6 and CO-29, CCR5, pHCG, ganglioside GD3, 9-0-Acetyl-GD3, GM2, Globo H, fucosyl GM1, Poly SA, GD2, Carboanhydrase IX (MN/CA IX), Sonic Hedgehog (Shh), CCR8, TNF-alpha precursor, A33 Antigen, Ly-6, desmoglein 4, E-cadherin neoepitope, Fetal Acetylcholine Receptor, CD25, Muellerian inhibitor Substance (MIS) Receptor type II, endosialin, SAS, CD63, TF-antigen, CD7, CD22, Iga(CD79a), Ig (CD79b), G250, gplOO, F19-antigen and EphA2.

Examples for antigens that are specific for a certain cell type/cell lineage or for a few cell types/cell lineages (cell type markers/cell lineage markers) include: CD45 for hematopoietic cells; CD34 for endothelial cells, stem cells, and stromal cells; CD33 for myeloid cells; CD 138 for plasma cells and a subset of epithelial cells; CD 15 for epithelial, myeloid, and Reed-Sternberg cells; CD la for cortical thymocytes and Langerhans cells; CD2 for thymic cells, T-cells, and Natural Killer (NK) cells; CD3 for T-cells; CD4 for helper T-cells; CD5 for T-cells, a subset of B-cells, and thymic carcinoma cells; CD8 for cytotoxic T-cells; CD20 for B-cells; CD23 for activated B-cells; CD31 for endothelial cells; CD43 for T-cells, myeloid cells, a subset of B-cells, histiocytes, and plasma cells; CD56 for NK cells; CD57 for neuroendocrine cells, and NK cells; CD68 for macrophages; CD79a for B-cells and plasma cells; CD 146 for the endothelial cell lineage; surfactant proteins for lung cells; synaptophysin, CD56 or CD57 for neuroendocrine cells; nicotinic acetylcholine receptor or muscle-specific kinase (MUSK) for muscle cells; voltage-gated calcium channel (P/Q-type) or voltage-gated potassium channel (VGKC) or N-methyl-D-aspartate receptor (NMD A) for muscle cells and neurons; TSH (thyroid stimulating hormone) receptor for thyroid gland; amphiphysin for muscle cells; HepPar-1 for hepatocytes; ganglioside GQ1B, GD3 or GM1 for neuronal cells; and glycophorin-A for cells of the erythropoietic cell lineage.

Specifically, the present disclosure provides a method of delivering cargo to a target cell, comprising:

(1) administering two targeting molecules, wherein:

- the first targeting molecule comprises a first targeting portion which binds specifically to a first target molecule on the target cell and wherein the targeting portion is linked to the first protein component of a transient protein:protein interaction; and
- the second targeting molecule comprises a second targeting portion which binds specifically to a second target molecule on the target cell and wherein the targeting portion is linked to the second protein component of the transient protein:protein interaction;

(2) administering a binding protein specific for a junctional epitope created by the transient protein:protein interaction, which binding protein is linked to the cargo and wherein the binding protein is as defined above.

This method is exemplified in Figure 1.

The first and second targeting portions are typically antibodies or antibody fragments (antibody fragments are described above), which specifically bind the first and second target molecules on the target cell. The targeting portions may also be ligands where the target molecules are receptors. Specific binding is discussed above. The targeting portions may linked to the protein components of the PPI by an appropriate means, such as a linker sequence. Methods of fusing proteins whilst retaining functionality are well known in the art.

The second step of the method involves administration of the binding protein, which, as discussed above, is typically an antibody or antibody fragment. The cargo may be attached to the binding protein by any appropriate means. The cargo may be a diagnostic or therapeutic agent. For example, the therapeutic compound may be a radiolabel (radioactive compound) such as $^{90}Y$, $^{177}Lu$, $^{131}I$, $^{32}P$, $^{10}B$ or $^{213}B$. The therapeutic compound may also be a toxin, such as *B. anthracis* edema factor, *B. anthracis* lethal factor, *C. perfringens* iota toxin, C. *botulinum* C2 toxin, C. difficile ADP-ribosyltransferase, C. *diphtheriae* diphteria toxin fragment A, *Burgholderia sp.* shiga toxin (subunit A), *Clostridium perfringens* str. 13 toxin pfoA perfringolysin O, Ricin A chain, plant RIP bouganin, Human RNASE3 ribonuclease (RNase A family, 3) and anthrax lethal factor endopeptidase.

The therapeutic agent could also be any appropriate drug or chemotherapeutic agent. Such agents are well known in the art.

The diagnostic agent could also be a radioactive compound, such as $^{99m}Tc$, $^{111}In$, $^{82}Rb$ or $^{201}Tl$. The diagnostic agent could also be a fluorescent compound, preferably GFP, a GFP variant, or a fluorescent small-molecule compound such as FITC (fluorescein isothiocyanate), PE (phycoerythrin), an alexa fluor dye (such as AlexaFluor488 or related dyes) or a cyanine dye (such as Cy3 (Indocarbocyanine) or Cy5 (Indodicarbocyanine) or related dyes). The diagnostic agent may be a molecule capable of mediating bioluminescence, preferably a luciferase molecule, more preferably *Gaussia* luciferase.

Depending on the type of cargo, the method described above can have a number of different purposes. For example, where the cargo is a therapeutic agent, the method may be a method of treatment of the human or animal body, e.g. a method of treatment of cancer. When the cargo is a diagnostic agent, the method could be an *in vivo* method of diagnosis.

Preferably, when used *in vivo,* the binding protein is a Fab, scFV or VHH. These formats result in rapid clearance of the cargo as such fragments rapidly pass through the renal filter. The small size of such fragments also means that rapid tissue penetration occurs.

The method could also be used *in vitro* when delivering cargo which is either a therapeutic or diagnostic agent.

The method described above provides a number of advantages over known techniques. Use of the binding protein provides greater specificity than when using an un-engineered bispecific antibody and avoids the need to engineer the bispecific antibody to effectively target only cells expressing both target molecules, rather than one. The method described herein is also independent of the target molecule. Furthermore, the method allows for fine tuning of the different steps, e.g. depending on the affinity of the targeting molecules for their target molecules, as well as the affinity of the binding protein for the transient PPI.

The dosage regimes will vary depending on the exact system to be used, and could readily be determined by the skilled person. The system should allow for accumulation of the targeting molecules at the desired sites with systemic clearance at non-target sites followed by secondary administration of the binding protein linked to the cargo. The binding protein would accumulate rapidly at the target site due to its interaction with the targeting molecules. Typically, the binding protein linked to the cargo would be administered 2-3 days after administration of the targeting molecules. However, spacing could be anything from hours (e.g. 4, 5, 6 hours) up to 3, 4 or 5 days.

Where the transient PPI is the IL-6/gp80 interaction, it would be appropriate to use a mutated system where interactions with gp130 are attenuated. This could readily be achieved by mutation of residues at the site 2 interface on either 11-6 or gp80. This would minimise any endogenous signalling.

A method for testing whether a binding protein can cross-link two antibody fusions (and thereby their antigens) is described in the Examples. A cancer cell killing assay would also be appropriate for testing a system where a chemotherapeutic agent is to be delivered. Such assays are well known in the art.

*Switching on a biological effect using a binding protein specific for a junctional epitope created by a transient protein:protein interaction*

The disclosure also provides use of a binding protein as defined above in switching on a biological effect. The biological effect may be switched on solely via administration of the binding protein to a biological system. In other words, the binding protein on its own may act as a molecular switch to switch on the biological effect associated with the transient PPI. Here, when one or two out of the first protein component of the transient PPI, second protein component of the transient PPI and the binding protein are present, the biological effect is not observed. However, when all three of the first and second protein components of the transient PPI and the binding protein are present, and the first and second protein components are co-localised, the PPI is stabilised and the biological effect is induced.

Such a system could have both *in vitro* and *in vivo* uses. For example, as discussed further below, stabilisation of the IL-6/gp80 interaction can be used to increase STAT3 phosphorylation (and associated downstream effects). *In vivo,* such a system has utility for example in liver regeneration after injury (see below).

The present disclosure also provides a second molecular switching method, where the binding protein is used to switch on a biological effect other than of the transient protein:protein interaction.

In particular, the disclosure provides a method of switching on a biological effect, comprising:

(1) administering two targeting molecules, wherein:

- the first targeting molecule comprises a first targeting portion which binds specifically to a first target molecule on the target cell and wherein the targeting portion is linked to the first protein component of a transient protein:protein interaction; and
- the second targeting molecule comprises a second targeting portion which binds specifically to a second target molecule on the target cell and wherein the targeting portion is linked to the second protein component of the transient protein:protein interaction; and

(2) administering a binding protein specific for a junctional epitope created by the transient protein:protein interaction, wherein the binding protein is as defined above and wherein administration of the binding protein switches on the biological effect.

This "molecular switch" method is illustrated in Figure 2.

The biological effect is not limited and could be any effect which is achieved via cross-linking of two target molecules. In an "off' position, with only one or two of the target molecules present (and accordingly only one of the two targeting molecules binding to the target molecule), the biological effect is not observed. However, in an "on" position, where the two target molecules are present and co-located, and the two targeting molecules are bound, addition of the binding protein causes cross linking and activation of the biological effect.

As discussed above, the targeting portions are typically antibodies (or antibody fragments), or ligands, which may be linked to the first and second components of the PPI be any known means, such as using a linker sequence. The target molecules may be antigens or receptors.

The above method may be used as a molecular switch *in vitro* or *in vivo.* For example, switching on a biological effect *in vivo* may be used in the treatment and/or prevention of a disease or condition.

As discussed above, when the transient PPI is the IL-6/gp80 interaction, it would be appropriate to use a mutated system where interactions with gp130 are attenuated.

Example 2 provides a general example of how bispecific targeting which only gives function when two molecular markers are present can be tested.

The present disclosure further provides use of a binding protein as defined above for altering a biological effect. In particular, said biological effect is a signalling response or a biological response. Amplifying the signalling by stabilizing a transient interaction using VHH6 enhances the signalling is demonstrated in particular in Figure 11. Figure 16 also demonstrates that said binding proteins modify the biological effect.

The present disclosure provides a method of altering of a biological effect, comprising:

(1) administering two targeting molecules, wherein:

- the first targeting molecule comprises a first targeting portion which binds specifically to a first target molecule on the target cell and wherein the targeting portion is linked to the first protein component of a transient protein:protein interaction; and
- the second targeting molecule comprises a second targeting portion which binds specifically to a second target molecule on the target cell and wherein the targeting portion is linked to the second protein component of the transient protein:protein interaction; and

(2) administering a binding protein specific for a junctional epitope created by the transient protein:protein interaction, wherein the binding protein is as defined above and wherein administration of the binding protein alters the biological effect.

More specifically the method provided above is used to amplify, decrease or modify a biological response. More particularly said method is used to switch on/off a biological effect.

*Use of a binding protein specific for a junctional epitope created by a transient protein:protein interaction in cross-linking target cells*

The disclosure also provides use of a binding protein as defined above in cross-linking target cells. In the particular, the disclosure provides a method of cross-linking two target cells using a binding protein, wherein first target cell expresses a first target molecule and the second target cell expresses a second target molecule, and wherein the method comprises:

(1) administering two targeting molecules, wherein:

- the first targeting molecule comprises a first targeting portion which binds specifically to the first target molecule on the first target cell and wherein the targeting portion is linked to the first protein component of a transient protein:protein interaction; and
- the second targeting molecule comprises a second targeting portion which binds specifically to the second target molecule on the second target cell and wherein the targeting portion is linked to the second protein component of the transient protein:protein interaction; and

(2) administering a binding protein specific for a junctional epitope created by the transient protein:protein interaction as defined above.

This method is illustrated in Figure 3.

The disclosure also provides method of cross-linking two target cells, wherein the first target cell expresses a first and second target molecule and the second target cell expresses a third target molecule, and wherein the method comprises:

(1) administering two targeting molecules, wherein:

- the first targeting molecule comprises a first targeting portion which binds specifically to the first target molecule on the first target cell and wherein the targeting portion is linked to the first protein component of a transient protein:protein interaction; and
- the second targeting molecule comprises a second targeting portion which binds specifically to the second target molecule on the first target cell and wherein the targeting portion is linked to the second protein component of the transient protein:protein interaction; and

(2) administering a third targeting molecule comprising a third targeting portion which binds specifically to the third target molecule on the second target cell, wherein the targeting portion is linked to a binding protein specific for a junctional epitope created by the transient protein:protein interaction and wherein the binding protein is as defined above.

This method is illustrated in Figure 4.

Furthermore, the disclosure provides a method of cross-linking two target cells, wherein the first target cell expresses a first and second target molecule and wherein the second target cell is engineered to present a binding protein on its surface, and wherein the method comprises administering two targeting molecules, where

- the first targeting molecule comprises a first targeting portion which binds specifically to the first target molecule on the first target cell and wherein the targeting portion is linked to the first protein component of a transient protein:protein interaction; and
- the second targeting molecule comprises a second targeting portion which binds specifically to the second target molecule on the first target cell and wherein the targeting portion is linked to the second protein component of the transient protein:protein interaction;

wherein the binding protein is as defined above.

This method is illustrated in Figure 5.

In all of these methods, the first, second and third (where present) targeting portions may be antibodies or fragments thereof, or ligands for the target molecules. The first, second and third (where present) target molecules may be antigens or receptors.

The first or second target cell is not particularly limited, but could be a cancer cell, which may be cross linked to an effector cell, such as a T cell. Target molecules on cancer cells and T cells would be well known to the person skilled in the art. Exemplary cancer antigens are discussed above. For example, the target molecule on a T cell could be CD3, particularly CD3ε. The target cell could also be an NK (natural killer) cell, where the target molecule may be NKp46. Once again, the method could be an *in vivo* method, whereby the method is a therapeutic or prophylactic method. The method could also be an *in vitro* method.

The method can be fine-tuned via the different binding kinetics of the binding protein and of the targeting molecules.

*Medical uses*

Binding proteins of the present disclosure may have medical applications when administered without targeting molecules. For example, the binding proteins could be used to stabilise the transient PPI *in vivo* and therefore induce a therapeutic or prophylactic effect (see above). The disclosure thus provides a method of treatment of the human or animal body comprising administration of a binding protein as described above. The binding protein could also be used to stabilise a transient PPI, before administration of the complex comprising the PPI with binding protein.

As discussed in the Examples of the present application, VHH6 was shown to significantly increase signalling through IL-6/gp80/gp130, culminating in phosphorylation of STAT3. Phosphorylated STAT molecules are translocated to the nucleus, where transcription of genes involved in the response to IL-6 is activated. VHH6 was indeed shown to affect transcription of genes in the IL-6 pathway. VHH6 thus seems to modulate disease states and therefore offers potential in further understanding the role of IL-6 e.g. in inflammation.

VHH6 (or indeed other binding proteins specific for a junctional epitope created by the IL-6/gp80 interaction) could also be used therapeutically where IL-6 has a role as growth factor, such as in tissue regeneration (particularly after injury in liver regeneration). For example, the binding protein could be used as a treatment to promote liver regeneration and restore liver function. Liver injury may be caused by a toxic substance (e.g. drug induced hepatopathy or alcoholic hepatitis), mechanical trauma, malignancy, an autoimmune disease or by a pathogen (e.g. hepatitis virus). The binding protein of the disclosure is administered in a therapeutically effective amount, which could be determined by the skilled person. The binding protein of the disclosure is typically administered in a pharmaceutical composition as discussed below.

Such a binding protein could also be used *in vitro* in the activation of hepatocyte-like progenitor cells and in expansion of hematopoietic progenitor cells.

Galun (2013) Methods in Molecular Biology, 982, 59-77 considers the regenerative role of IL-6. Wuestefeld et al (2003) The Journal of Biological Chemistry, 278, 11281-11288 considers the role of the IL-6/gp80 pathway in liver regeneration, Best et al (2010) Experimental and Molecular Pathology, 88, 7-14 looks at hepatocyte-like progenitor cells in rat liver injury and Fischer (1997) Nature Biotechnology, 15, 142-145 considers hematopoietic progenitor expansion. The use of covalently linked IL-6/gp80 has also been described for tissue regeneration (particularly liver) in WO 99/62534.

*Use of a binding protein specific for a junctional epitope created by a transient protein:protein interaction in tissue regeneration.*

Importantly, the present disclosure allows to target biological effects to the sites of the two targets described above to avoid systemic effects that might occur if one of these targets was administered systemically. Systemic administration of IL-6 could cause broad inflammation which would be undesirable (Gabay et al. Arthritis Res Ther 8, suppl. 2:S3 (2006)). Using the binding proteins disclosed herein, effects could be restricted to the site of liver injury where gp80 and IL-6 are released. The binding proteins disclosed herein could be used to enhance optic nerve regeneration by promoting IL-6 signalling (Fischer, Eye 31, 173-178 (2017)), in airway tissue regeneration (Tadokoro et al. PNAS 111(35), E3641-E3649 (2014)) or more broadly in other areas of regenerative medicine.

In particular, the present disclosure provides a method of amplifying or increasing the IL-6/gp80 signalling, comprising administering a binding protein o described herein. Said method might be practiced as an *in vitro* method or an *in vivo* method.

In particular, the present disclosure provides a method of tissue regeneration, comprising administering a binding protein described herein. In a particular embodiment of the disclosure said method is an *in vitro* method. In yet another embodiment of the disclosure said method is an *in vivo* method.

In particular, the present disclosure provides a method of tissue regeneration, comprising administering a binding protein described herein. Said protein might be administered in a pharmaceutical composition as disclosed further herein.

The present disclosure provides further the use of the binding protein of the disclosure for tissue regeneration.

In particular embodiment of the disclosure said protein is a protein specific for a junctional epitope created by a transient protein:protein interaction, wherein said protein:protein interaction is the IL-6/gp80 interaction. More specifically said protein amplifies or increases the IL-6/gp80 signalling by stabilizing a transient interaction.

More particular said tissue regeneration is liver regeneration. In another embodiment of the disclosure said tissue regeneration is nerve regeneration. In yet another embodiment of the disclosure said tissue regeneration is airways regeneration.

More specifically said binding protein is the VHH6 antibody. More particular said binding protein is a humanized VHH6 antibody. More specifically said humanized VHH6 antibody is the humanized VHH6 antibody as disclosed herein.

*Pharmaceutical composition*

A binding protein described herein may be provided in a pharmaceutical composition. The pharmaceutical composition will normally be sterile and will typically include a pharmaceutically acceptable carrier and/or diluent. A pharmaceutical composition may additionally comprise a pharmaceutically acceptable adjuvant and/or carrier.

As used herein, "*pharmaceutically acceptable carrier*" includes any and all solvents, dispersion media, coatings, antibacterial and antifungal agents, isotonic and absorption delaying agents, and the like that are physiologically compatible. The carrier may be suitable for parenteral, e.g. intravenous, intramuscular, intradermal, intraocular, intraperitoneal, subcutaneous, spinal or other parenteral routes of administration, for example by injection or infusion. Alternatively, the carrier may be suitable for non-parenteral administration, such as a topical, epidermal or mucosal route of administration. The carrier may be suitable for oral administration. Depending on the route of administration, the modulator may be coated in a material to protect the compound from the action of acids and other natural conditions that may inactivate the compound.

The pharmaceutical compositions may include one or more pharmaceutically acceptable salts. A "*pharmaceutically acceptable salt*" refers to a salt that retains the desired biological activity of the parent compound and does not impart any undesired toxicological effects. Examples of such salts include acid addition salts and base addition salts.

Pharmaceutically acceptable carriers comprise aqueous carriers or diluents. Examples of suitable aqueous carriers that may be employed in the pharmaceutical compositions include water, buffered water and saline. Examples of other carriers include ethanol, polyols (such as glycerol, propylene glycol, polyethylene glycol, and the like), and suitable mixtures thereof, vegetable oils, such as olive oil, and injectable organic esters, such as ethyl oleate. In many cases, it will be desirable to include isotonic agents, for example, sugars, polyalcohols such as mannitol, sorbitol, or sodium chloride in the composition.

Therapeutic compositions typically must be sterile and stable under the conditions of manufacture and storage. The composition can be formulated as a solution, microemulsion, liposome, or other ordered structure suitable to high drug concentration.

Pharmaceutical compositions may comprise additional active ingredients.

Also within the scope of the present disclosure are kits comprising the binding proteins of the disclosure and instructions for use.

The binding proteins disclosed herein or formulations or compositions thereof may be administered for prophylactic and/or therapeutic treatments.

In therapeutic applications, compounds are administered to a subject already suffering from a disorder or condition as described above, in an amount sufficient to cure, alleviate or partially arrest the condition or one or more of its symptoms. Such therapeutic treatment may result in a decrease in severity of disease symptoms, or an increase in frequency or duration of symptom-free periods. An amount adequate to accomplish this is defined as a "*therapeutically effective amount*".

In prophylactic applications, formulations are administered to a subject at risk of a disorder or condition as described above, in an amount sufficient to prevent or reduce the subsequent effects of the condition or one or more of its symptoms. An amount adequate to accomplish this is defined as a "*prophylactically effective amount*". Effective amounts for each purpose will depend on the severity of the disease or injury as well as the weight and general state of the subject.

A subject for administration may be a human or non-human animal. The term "*non-human animal*" includes all vertebrates, *e.g.*, mammals and non-mammals, such as non-human primates, sheep, dogs, cats, horses, cows, chickens, amphibians, reptiles, etc. Administration to humans is typical.

A pharmaceutical composition may be administered via one or more routes of administration using one or more of a variety of methods known in the art. As will be appreciated by the skilled artisan, the route and/or mode of administration will vary depending upon the desired results. Examples of routes of administration for compounds or pharmaceutical compositions include intravenous, intramuscular, intradermal, intraocular, intraperitoneal, subcutaneous, spinal or other parenteral routes of administration, for example by injection or infusion. The phrase "*parenteral administration*" as used herein means modes of administration other than enteral and topical administration, usually by injection. Alternatively, compound or pharmaceutical composition can be administered via a non-parenteral route, such as a topical, epidermal or mucosal route of administration. The pharmaceutical composition may be for oral administration.

A suitable dosage of pharmaceutical composition may be determined by a skilled medical practitioner. Actual dosage levels of the active ingredients in the pharmaceutical compositions may be varied so as to obtain an amount of the active ingredient that is effective to achieve the desired therapeutic response for a particular patient, composition, and mode of administration, without being toxic to the patient. The selected dosage level will depend upon a variety of pharmacokinetic factors including the activity of the particular compositions employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A suitable dose may be, for example, in the range of from about 0.01μg/kg to about 1000mg/kg body weight, typically from about 0.1μg/kg to about 100mg/kg body weight, of the patient to be treated. For example, a suitable dosage may be from about 1μg/kg to about 10mg/kg body weight per day or from about 10 μg/kg to about 5 mg/kg body weight per day.

Dosage regimens may be adjusted to provide the optimum desired response (e.g., a therapeutic response). For example, a single dose may be administered, several divided doses may be administered over time or the dose may be proportionally reduced or increased as indicated by the exigencies of the therapeutic situation. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Administration may be in single or multiple doses. Multiple doses may be administered via the same or different routes and to the same or different locations. Alternatively, doses can be via a sustained release formulation, in which case less frequent administration is required. Dosage and frequency may vary depending on the half-life of the antagonist in the patient and the duration of treatment desired.

As mentioned above, pharmaceutical composition may be co-administered with one or other more other therapeutic agents.

Combined administration of two or more agents may be achieved in a number of different ways. Both may be administered together in a single composition, or they may be administered in separate compositions as part of a combined therapy. For example, the one may be administered before, after or concurrently with the other.

*Method of producing an antibody specific for a junctional epitope created by a transient protein:protein interaction*

The present invention provides a method of producing an antibody specific for a junctional epitope created by a transient protein:protein interaction. The method involves immunising an animal with a fusion protein of the stabilised PPI complex. Suitable animals are described above, but are may usefully be camels in order to generate VHH antibodies.

With regards to the fusion protein, for example, one of the protein components of the PPI may be fused directly onto the terminus (e.g. the C-terminus) of the other protein as described in the Examples for generation of VHH6. Methods of fusing proteins together are well known in the art.

The method then involves selecting for an antibody which interacts with the complex of the PPI but not with each individual component of the PPI in the absence of the other. Methods of determining whether an antibody interacts with the PPI complex, but not with each individual component, are described above and include ELISA, HLPC, MicroScale Thermophoresis, Octet (Biol-Layer Interferometry) and surface plasmon resonance (typically at 25 °C). In the present invention, the binding protein may show undetectable binding to each individual component of the PPI using any one, preferably multiple, more preferably all, of the above techniques. As discussed above, the binding protein may in some instances show a very low level of binding to the individual proteins of the PPI, but not enough to limit the usefulness of the binding protein.

The method then involves determining that the antibody stabilises the protein:protein interaction. Methods and representative values for stabilisation of the PPI are discussed above.

The method may for example also use additional techniques, such as X-ray crystallography to confirm the junctional nature of the binding protein. Junctional epitopes are described above.

*Use of a binding protein specific for a junctional epitope created by a transient protein:protein interaction in screening for a substance which modulates signalling through a protein:protein interaction*

The disclosure further provides use of a binding protein of the disclosure in screening for substances which modulate signalling through a transient PPI. The substance may for example be a small molecule, peptide or even an antibody. The substance is typically an inhibitor i.e. a substance which reduces signalling through the transient PPI. In other words, the binding protein may be used in structure-aided drug discovery. Such analysis may be computational.

The following Examples illustrate the invention. Using the interaction between IL-6 and gp80 as a well-known example of a PPI, the present inventors have produced and characterized VHH6, a prototype locking (junctional) antibody. VHH6 specifically recognizes an epitope that spans the junction between IL-6 and gp80, locking them together in a stable functional unit. Such an antibody finds use in a number of different applications, including specific targeting of antigens on cancer cells.

## Examples

### Example 1: Production and testing of VHH6

### Materials and Methods - Antibody Production

Protein production and purification: With the exception of Human HyperIL-6 (Fischer, M. et al. I. Nature biotechnology 15, 142-145 (1997)), all proteins (FusionIL-6, gp80V4, gp80-hscFc, IL-6 and IL-6S21) were designed in house. FusionIL-6 is a modified version of HyperIL-6 where all non-natural linker residues have been removed, IL6 (V30-M212) was fused directly onto the C-terminus of gp80 at the end of domain 3 (S320). In addition, two unpaired cysteines were mutated to alanine (C211A, C277A). To increase success in crystallization, an engineered from of gp80 was used (gp80V4). In this construct the N-terminal 14aa of gp80 domain 1 (L20-G33) were fused to the N-terminus of gp80 domain 2 using a short linker. The cysteine (C25) in the domain 1 fragment forms a disulphide bond with cysteine 193 in domain 2 stabilising the protein. In addition gp80V4 also has unpaired cysteines C211 and C277 mutated to alanine.

All those proteins were produced in house. For constructs, vectors and cell systems used for protein production see Table 1 below:

**Table 1: Constructs used in protein production**

| Construct | Expression Host | Promoter | Affinity Tag | Expressed Protein |
|---|---|---|---|---|
| **pNAFL-8His-fusionIL-6 (gp80D123)** | CHO | CMV | 8His | gp80(L20-S320)(C211A, C277A)-IL-6(V30-M212) |
| **pNAFL-gP80D123-hscFc** | CHO | CMV | Single chain human IgG Fc fragment | gp80(M1-P322)(C211A, C277A)-hscFc |
| **pNAFL-8His-gp80delta1,2,3 V4** | CHO | CMV | 8His | gp80(L20-G33-AGAG linker-D111-P322 (C211A,C277A) |
| **pTrx-6His-hIL-6** | *E.coli* | T7 | Thioredoxin-6His | Trx-6His-IL-6 (A28-M212) |
| **pTrx-6His-IL-6 (S21)** | *E.coli* | T7 | Thioredoxin-6His | Trx-6His-IL-6(S49-M212) |
| **pIMMs-6His-VHH6** | Expi-HEK | CMV | 6His | VHH6-6His |

Numbering for gp80 is based on Uniprot reference P08887.

Protein used for crystallography and HDX-MS were produced in Expi-HEK or CHO cells (Gibco, Thermo Fisher Scientific) in media containing Kifunensine (GlycoSyn). IL-6 was produced in E.coli. For *in vitro* experiments IL-6, gp80 were purchased from R&D Systems. For SPR experiments gp130 was purchased from R&D Systems.

Ni purification: Clarified supernatant was passed through a 0.22 μm filter and loaded onto a 1 ml/5 ml Ni Excel column (GE Healthcare) pre-equilibrated in PBS (pH 7.4) containing 20 mM imidazole. Bound protein was eluted with PBS (pH 7.4) containing 250 mM imidazole. Fractions containing protein of interest as judged by 4-12% Bis/Tris NuPage (Life Technologies, Gibco, Thermo Fisher Scientific) gel electrophoresis were dialyzed to remove imidazole and treated with TEV protease at a ratio of 1 mg per 100 mg protein. After O/N incubation at 4 °C the sample was passed over a Ni Excel column for a second time removing non-cleaved protein and His tagged TEV protease resulting in pure cleaved protein in the column flow through.

Fc-taggedprotein purification: Clarified supernatant was passed through a 0.22 μm filter and loaded onto a 1ml/5ml Mab Selectsure column (GE Healthcare) pre-equilibrated in PBS (pH 7.4). If the protein was mouse Fc-tagged, 0.5 M NaCl was added to the supernatant to facilitate binding. Protein was then eluted with a phosphate/citrate pH gradient using 0.15 M $Na_2HPO_4$ at pH 9 and 0.1 mM Sodium Citrate at pH 2. Proteins were eluted at around pH 3.5 and immediately neutralized using 2 M Tris at pH 8.

TEV-HuFc tagged proteins were eluted by adding TEV protease directly to the Mab Selectsure beads at 100 μg TEV per ml of beads and incubating in PBS at 4 °C overnight. Beads were then removed by centrifugation to leave cleaved untagged protein.

Gel Filtration: Proteins were concentrated using Centricon Tubes (Merck Millipore) and loaded onto an S75 26/60 gel filtration column (GE Healthcare) pre-equilibrated in PBS. Peak fractions were pooled and concentrated Centricon Tubes (Merck Millipore).

Antibody discovery campaign: Two camels were immunized subcutaneously over a period of three months with HyperIL-621, escalating the dose from 3 μg up to maximum 1 mg in half log increments, at the Central Veterinary Research Laboratory (CVRL) in Dubai, according to a humane protocol approved by the Scientific Directorate of the CVRL. Experimental procedures were performed in accordance with relevant guidelines/regulations and approved by the Scientific Directorate of the CVRL. Initial immunization was carried out with complete Freund's adjuvants, while the subsequent boosts used incomplete Freund's adjuvants. 3 mL serum samples were collected at 8, 10, 12, and 18-week post-initial immunization to monitor the immune response; at 19 week, 200 mL of heparinized blood were collected and PBMCs isolated and frozen. Single domain VHHs were generated from frozen PBMCs from immunized animals by cloning variable region genes from isolated B cells using the antibody discovery platform described in Tickle, S. et al.. JALA 14, 303-307 (2009) and Lightwood, D. et al. Journal of molecular biology 425, 577-593 (2013).. VHHs were subsequently cloned in pIMMs vector with TEV-6His tag downstream the VHH fragment for mammalian expression (Hancock, L.M. A study of camelid antibodies reveal structural features of a cytokine. PhD Thesis University of London (2011)).

**Materials and Methods** - **Preliminary screening assays**

ELISA - IL-6, gp80 and FusionIL-6 used in ELISA to coat plates were diluted to a final concentration of 2 mg/mL in carbonate buffer (0.16% $Na_2CO_3$ and 0.3% $NaHCO_3$ in $H_2O$). Coated plates were incubated at 4°C overnight. T he following day, the plates were washed twice in PBST, blocked in PBS/5%BSA for 2 h at RT, and again washed twice in PBST. For preliminary screening, either supernatant from HEK cells or purified VHHs was added. Anti-llama-HRP or anti-His-HRP antibody was added for 1h at RT. Afterwards the plates were washed twice with PBST and developed with TMB substrate. The reaction was stopped by adding NaF. Plates were read at 650 nm with a reference wavelength of 450 nm (Biotek PowerWave HT Microplate Spectrophotometer). In other experiments, IL-6 was coated overnight on the plate; VHH6 was added alone or with gp80-hscFc. Alternatively, gp80-hscFc was coated overnight on the plate, VHH6 was added alone or with IL-6. Anti-llama-HRP antibodies (Bethyl Laboratories), anti-human Fc-HRP (Jackson Lab, USA) or anti-His-HRP (Qiagen) antibodies were used for revealing the plate.

HPLC - Solutions VHH6, gp80 and IL-6 alone or in combination were incubated overnight at 4 °C. The following day, size exclusion chromatography was carried out on a HiLoad 16/60, Superdex G3000 column (GE Healthcare) equilibrated with 50 mM NaCl, 25 mM Tris, 5% (v/v) glycerol using an Agilent 1100 Series column (GE Healthcare).

MST - MicroScale Thermophoresis (MST) relates to the movement of particles subjected to a temperature gradient. W hen a temperature gradient is applied, changes of the hydration shell of proteins reflect in protein structure/conformation changes and this can be used to determine receptor:ligand binding affinities (Jerabek-Willemsen, M., Wienken et al. Assay and drug development technologies 9, 342-353 (2011). Both IL-6 and gp80 were labelled with NT647 using the Monolith labelling Red NHS kit (NanoTemper Technologies GmbH, Germany). NT647 dye is optimized for MST and carries a reactive NHS-ester group that modifies primary amines in lysine. Labelling and purification was carried out according to the protocol provided by the supplier. Standard treated capillaries NT. 115 Series (NanoTemper Technologies GmbH, Germany) were used throughout the experiments. The red channel of Monolith NT. 115 Blue/Red Monolith NT. 115 (NanoTemper Technologies GmbH, Germany) was used. Both IL-6 and gp80 were labelled with NT647 dye with a ratio protein to dye above 0.5 to check for binding of IL-6 or gp80 only; a titration experiment using up to 20 mM VHH6 was used. To determine the binding between VHH6 and IL-6-gp80, 10 nM IL-6NT647 and 20 nM gp80 was used, and VHH6 was titrated in, at a concentration started with at least ten-fold the concentration of labelled molecules.

Octet - Octet is based on Bio-Layer Interferometry (BLI) for measuring e.g. protein:protein interaction and is a label-free technique. BLI analyzes the interference pattern of white light reflected from two surfaces: a layer of immobilized protein on the biosensor tip, and an internal reference layer. Bio-Layer Interferometry (BLI) was used as a label-free technology for protein: protein binding (Abdiche et al Journal of immunological methods 382, 101-116 (2012)). The binding between VHH6 immobilized on the biosensor tip surface using Biosensor NiNTA tips (ForteBio, Pall Corporation) and FusionIL-6, IL-6, gp80 and IL-6-gp80 in complex was measured. Interference patterns for the binding to or dissociating from the biosensor were measured in real time to generate a response profile on the Octet® System Octet 384 Red (ForteBio, Pall Corporation).

SPR measurements - All SPR experiments were carried out at 25°C in HBS-P+ buffer (10 mM HEPES pH 7.4, 0.15 M NaCl, 0.05% (v/v) surfactant P20, (GE Healthcare) as the running buffer. VHH6, IL-6, gp80 and gp130 proteins were immobilized on CM5 sensor chips (GE Healthcare) by standard amine coupling method, as recommended by the manufacturer.

Qualitative SPR analysis was performed using a BIAcore 3000 (GE Healthcare); gp80 and VHH6 were injected at 50 nM concentration at a flow rate of 30 μl/min. gp80 was injected for 180 s and the dissociation was monitored for 360 s. Alternatively, gp80 was injected for 180 s and subsequently VHH6 was co-injected and the dissociation was monitored for 360 s.

Quantitative SPR analysis was carried out using a BIAcore T200 (GE Healthcare). The binding measurements were performed at a flow rate of 100 μl/min. The protein of interest was injected for 180 s and the dissociation was monitored for 360 s. Binding of IL-6, gp80 and VHH6 were individually tested in a concentration series (0-250 nM, as twofold serial dilution). When proteins were tested in combinations, i.e. IL-6 and gp80, IL-6 and VHH6, gp80 and VHH6, gp80 and IL-6 and VHH6, one of the protein was titrated in a concentration series (0-250 nM), while the other was kept constant at an excess concentration of 2 μM to ensure complex formation. The sensor surface was regenerated with 4 mM MgCl2. Background subtraction binding curves were analyzed using the T200 evaluation software (version 1.0) following standard procedures. Due to the complexity of the interactions studied, the data analysis was limited to the most informative, in this case $k_d$ parameter, which was determined by fitting a single exponential decay model to dissociation data within 360 s range.

In order to evaluate the effect of VHH6 on the binding of the IL-6-gp80 complex to gpl30, the kinetic data were fitted by applying a 1:1 binding model. The affinities for the interactions that reached equilibrium during the injection time of the experiment were determined from a steady state affinity fit, and are reported in the Supplementary Tables. The binding curves were re-plotted in Prism (GraphPad Software Inc.) to render the figures with a better resolution.

Mutant versions of VHH6 containing single alanine substitutions at positions 27, 32, 74, 101 and 113, and double alanine substitutions at positions 27/32, 74/101 and 113/101 were generated and supernatants produced in transient transfected cells (see paragraph Generation of VHH6 mutants) were tested by SPR. SPR analysis was performed using a BIAcore 3000 (GE Healthcare). His-tagged VHH6 and VHH6 mutants (1:5 diluted supernatants) were captured on a NTA chip (30 μl at 10 μl/min) following charging of the surface by injection of 10 μl 0.5 M $NiCl_2$. An equimolar mixture of IL-6 and gp80 (50 nM each) was injected for 300 s at a flow rate of 10 μl/min followed by a dissociation phase of 300 s. At the end of each cycle the chip surface was regenerated by injection of 350 mM EDTA (2 × 10 μl).

Cell culture - Pooled HUVEC cells were purchased from Gibco (Gibco, Thermo Fisher Scientific) and grown in 200PRF media supplemented with Low Serum Supplement Media (Gibco, Thermo Fisher Scientific). Different batches of HUVEC cells were tested in our experiments in order to have a good population representation. Cells were not used beyond the 5-6$^{th}$ passage.

Phospho-STAT3 staining - Cells were plated the day before at $1\times10^4$ cells/well in 96well BD Falcon Becton Black/ Clear tissue culture treated plates (Becton Dickinson). The following day, the cells were treated with FusionIL-6 (3 ng/mL) and IL-6+gp80 alone (respectively 10 ng/mL and 20ng/mL) or in combination with an excess of VHH6 and fixed with PFA 4% at chosen time points (30 min, 180 min and 360 min). After 20 min incubation at RT, the cells were washed twice with PBS and ice-cold methanol was added and stored at -20°C. Before staining, the cells were washed twice in PBS, incubated for 20 min at 4°C with PBS/10%FCS. Afterwards, anti-Phospho-STAT3 (Tyr705) (clone D3A7, Cell Signalling) was added at a dilution suggested by the manufacturer (1:100 in PBS/10%FCS) and incubated for 1 h at 4°C. After washing twice in PBS, secondary antibody goat anti-rabbit-AlexaFluor568 (Gibco, Thermo Fisher Scientific) was added and incubated for 1h at 4°C. Then cells were washed twice in PBS, and nuclei stained for 10 min with a solution of DAPI in PBS/10%FCS (final concentration 2 mg/mL). Finally, cells were washed twice with PBS and 200 μL of fresh PBS were added to each well and stored in the dark at 4 °C.

Internalization of IL-6—gp80 complex - Cells were plated the day before at 1×10$^4$ cells/well in 96 well BD BIOCOAT Cell Environment Collagen Cellware Black/Clear plates (Becton Dickinson). The following day the cells were treated with IL-6-NT647 alone (100 ng/mL), IL-6-NT647+gp80 (respectively 100 ng/mL and 200 ng/mL) with and without the addition of an excess of VHH6 and fixed with PFA 4% at chosen time points. After 20 min incubation at RT, the cells were washed twice with PBS and ice-cold methanol was added and stored at -20°C. Before staining, the cells were washed twice in PBS, incubated for 20 min at 4 °C with PBS/10%FCS. Afterwards, mouse anti-LAMP1 antibody (clone H4A3, Abcam) was added at a dilution suggested by the manufacturer (1:100 in PBS/10%FCS) and incubated for 1h at 4 °C. After washing twice in PBS, secondary antibody goat anti-mouse-AlexaFluor488 (Gibco, Thermo Fisher Scientific) was added and incubated for 1h at 4 °C. Then cells were washed twice in PBS, and nuclei stained for 10 min with a solution of DAPI in PBS/10%FCS (final concentration 2 mg/mL). Finally, cells were washed twice with PBS and 200 μL of fresh PBS were added to each well and stored in the dark at 4 °C.

Image Acquisition and Analysis - Images were acquired using Cellomics Arrayscan (Thermoscientific). For each well, 16-fields were captured containing approximately 100 cells per field at a resolution of 1104×1104 pixels. DAPI, Alexa488, Alexa568 and Alexa647 were detected using appropriate excitation and emission filter sets. Exposure times were kept constant between repeated experiments.

Cells were analyzed using the Cellomics Spot Detector Algorithm. Nuclei were detected on the basis of DAPI staining and STAT3 intensity measured within this region. The 'spot total intensity per object' parameter was used for further analysis. For analysis of internalized IL-6-NT647, the mask for analysis was extended from the nuclei and NT-647 intensity measured from spots detected in this region.

Statistical analysis - The raw data from the Arrayscan analysis were processed using the 'spot total intensity per object' parameter, and this parameter from three replicates was statistically analyzed using a linear mixed model for repeated measurements using the software package SAS 9.2 (SAS Institute, Cary NC).

qPCR array - Briefly, HUVEC cells were seeded at a concentration of 5×10$^5$ cells/well in 6-well plates (Falcon, BD Biosciences) the day before. The following morning, the cells were treated with gp80+IL-6 and VHH6+gp80-+L-6 at the same concentration used for the pSTAT3 staining for 30, 180 and 360 min. At different time points, cells were washed twice with PBS, re-suspended in lysis buffer (Qiagen) and immediately frozen in dry ice and stored at -80 °C.

Total RNA was extracted from HUVEC cell lysates using the RNeasy Plus Mini Kit (Qiagen) and quantitated using a Nanodrop (Thermo Fisher). Using the RT$^2$ First Strand cDNA Kit (SABiociences, Qiagen) 400 ng of mRNA was converted to cDNA according to the manufacturer's protocol. The 'Human IL-6/STAT3 Signalling Pathway Plus' (PAHS-160YE-4) array designed to quantitatively measure mRNA levels of 84 key genes involved IL-6 signaling, 384 well format, were run on an ABI 7900HT machine. Data were analysed using the RT2 Profiler PCR array web based data analysis template v3.5 (http://pcrdataanalysis.sabiosciences.com/pcr/arrayanalysis.php) and changes in gene expression changes were calculated using the $\Delta\Delta C_t$ method with normalization of the raw data to housekeeping genes. Each RT$^2$ Profiler PCR Array plates contained 84 genes related to IL-6 pathway plus 5 different housekeeping genes. Three biological independent replicas of the same experiments were analyzed together. Only statistical significant results ($p \leq 0.05$) were showed alongside with p-value.

**Materials and Methods** - **Structure determination**

X-ray crystallography - The IL-6, gp80 and VHH6 complex was prepared using the engineered gp80V4 construct (described in protein production and purification). The additional domain 1 residues for gp80 were necessary as they make a range of intramolecular interactions to domain 2, as observed in the isolated gp80 X-ray structure (PDB entry 1N26). The majority of the domain 1 residues are not seen in the X-ray structure due to molecular disorder although the cysteine disulphide bond (Cys25-Cys193) is still retained and is clearly visible. The purified VHH6, gp80 and IL-6 were mixed in a molar ratio of 2:1:1 and incubated overnight at 4 °C to allow the complex formation. The complex was purified by size exclusion chromatography (SEC) over a HiLoad 16/60, Superdex 200 column (GE Healthcare) equilibrated with 50 mM NaCl, 25 mM Tris, 5% (v/v) glycerol. Fractions containing the complex were pooled and concentrated to 9 mg/ml for crystallography. Conditions suitable for crystal growth were identified by the sitting drop vapor diffusion method using commercially available crystallization screens (Qiagen). To generate diffraction quality crystals, hanging drop vapor diffusion method was used. 1 μl of protein solution was mixed with 1 μl of reservoir solution containing 0.1 M MES, pH 6.5, 14% PEG20K. Crystals reached full size in 14 d at 19 °C. Crystals were harvested and flash frozen in liquid nitrogen without additional cryo-protectant. Diffraction data to 2.7 A was collected from a single crystal on the Proximal beamline at Soleil Synchrotron, France, and processed using XDS (Kabsch, W. Xds. Acta crystallographica. Section D, Biological crystallography 66, 125-132 (2010)).. The structure of the complex was solved by molecular replacement with Phaser (McCoy, A.J. et al. Journal of applied crystallography 40, 658-674 (2007)), using coordinates of an in-house VHH structure, and IL-6 and gp80 from PDB entry 1P9M (Boulanger M.J. et al. Science 300, 2101-2104 (2003)), as search models. The initial structure was refined with iterative cycles of simulated annealing, energy minimization and manual rebuilding using CNS (Brunger, A.T. et al. Crystallography & NMR system: A new software suite for macromolecular structure determination. Acta crystallographica. Section D, Biological crystallography 54, 905-921 (1998) and Brunger, A.T. Version 1.2 of the Crystallography and NMR system. Nature protocols 2, 2728-2733 (2007)) and COOT (Emsley, P. & Cowtan, K. Coot: model-building tools for molecular graphics. Acta crystallographica. Section D, Biological crystallography 60, 2126-2132 (2004)). Model geometry was validated using Molprobity (Chen, V.B. et al. MolProbity: all-atom structure validation for macromolecular crystallography. Acta crystallographica. Section D, Biological crystallography 66, 12-21 (2010)). Surface areas of the protein interfaces were calculated using PISA (Krissinel, E. & Henrick, K. Inference of macromolecular assemblies from crystalline state. Journal of molecular biology 372, 774-797 (2007)). Molecular visualizations were generated with Pymol (DeLano W., The PyMOL Molecular Graphics System., 2002, version 1.7, DeLano Scientific LLC, San Carlos, CA. www.pymol.org). Data collection and refinement statistics are summarized below:

**Table 2: Data collection and refinement statistics**

| | **Junctional Epitope Antibody in complex with IL-6 and gp80** |
|---|---|
| Data collection | |
| **Space group** | C 1 2 1 |
| **Cell dimensions** | |
| ***a**, b, c* **(Å)** | 249.03, 67.80, 78.16 |
| $\alpha$, $\beta$, $\gamma$ (°) | 90.00, 104.53, 90.00 |
| **Resolution (Å)** | 47.84-2.70(2.86-2.69) * |
| Rmeas **(%)** | 7.2(50.1) |
| $CC_{1/2}$ **(%)** | 99.9(91.5) |
| $I / \sigma I$ | 20.88(3.97) |
| **Completeness (%)** | 99.4(96.9) |
| **Redundancy** | 7.5(7.5) |
| Refinement | |
| **Resolution (Å)** | 20.00-2.70 |
| **No. reflections** | 34,870 |
| $R_{work}$ / $R_{free}$ | 0.2313 / 0.2872 |
| **No. atoms** | |
| **Protein** | 7590 |
| **Water** | 111 |
| ***B*-factors** | |
| **Protein** | 56.33 |
| **Water** | 43.19 |
| **R.m.s. deviations** | |
| **Bond lengths (Å)** | 0.007 |
| **Bond angles (°)** | 1.289 |
| *Values in parentheses are for highest-resolution shell. | |

Generation of VHH6 mutants - To investigate the contribution to binding of IL-6 and gp80 by VHH6 residues identified in the crystal structure as being likely contact residues, we generated a panel of mutants using oligonucleotide-directed mutagenesis. Following small scale transient expression in ExpiHEK cells, supernatants were analyzed by SPR. The *k*d generated using supernatants from the single and double mutants were compared to the supernatant from VHH6.

HDX-MS - 40 mM gp80V4-IL-6 or 40 mM gp80V4-IL-6-VHH6 complex, both purified by SEC, were diluted 20x either into 10 mM phosphate in $H_2O$ (pH 7.0), or into 10 mM phosphate in $D_2O$ (pD 7.0) and left to react for 0.5, 2, 10, 30 or 120 min. After reaction, these were diluted 1:1 with a cold quench solution (1°C, 3.4 M Gdn.HCl, 500 mM TCEP, 200 mM phosphate) to give a final pH of 2.5. This mixture was immediately injected into the nanoAcquity HDX module (Waters Corp.) for peptic digest. All liquid handling steps were performed by a LEAP-PAL robot (CPC). The protein samples were washed onto the Enzymate pepsin column (25 °C) with 0.1% HCOOH in $H_2O$ at a flow-rate of 90 mL $\times$ $min^{-1}$, and resulting peptides trapped on a VanGuard C18 trapping column over 3 min. These peptides were washed off the trap-column by reversing the flow and separated over a BEH C18 column (10 x 1.0 cm, 1.7 $\mu$m), using the following gradient: 0 min, 5% B; 7 min, 40% B; 8 min, 95% B; 10 min, 95% B, 11 min, 5% B (B: 0.2% HCOOH in $H_2O$, A: 0.1%

HCOOH in MeCN). Data acquisition was performed on a Synapt G2S (Waters Corp.) run in Tofonly mode over an m/z range of 50-2000 Th, using an MSe method (low collision energy, 10V; high collision energy: ramp from 15V to 35V). Glu-1-Fibrinopeptide B peptide was used for internal lock mass correction.

HDX-MS Data Processing - Data were collected in triplicate, with blanks run between each data-point. MSe results from the $H_2O$-diluted sample were analyzed using PLGS v3.0.1, searching for peptides against a database of the IL-6 and gp80V4 sequences only, with precursor intensity threshold of 500 counts and 3 matched product ions required for assignment. Ion accounting files for the 6 control samples were combined into a peptide list imported into Dynamx v2.0 software, which was further curated to disallow peptides with low energy intensity less than 100, peptides longer than 25 amino acids, and peptides eluting with a retention time longer than 9 minutes. Dynamx v2.0 was used to quantify the isotopic envelopes resulting from deuterium uptake for each peptide at each time-point; these data were then interrogated manually to ensure correct assignment of m/z peaks. Students t-tests were performed on the triplicate data-points for each time-point, and the significance of differential HDX between gp80V4—L-6 and gp80V4—IL-6—VHH6 being defined as $p<0.01$.

## Results

VHH6 recognizes specifically IL-6—gp80 complex

Immunization of camels with HyperIL-6, a fusion protein of gp80 joined to IL-6 with a flexible peptide linker and subsequent antibody discovery led to the generation of 41 unique VHH sequences. All 41 VHHs recognized HyperIL-6 and FusionIL-6 in an initial ELISA screen. FusionIL-6 was an in-house designed fusion protein between IL-6 and gp80. These 41 VHHs were separated according to their ability to bind to either IL-6 (30 VHHs) or gp80 (7 VHHs). Four VHHs recognized only FusionIL-6, but neither IL-6 nor gp80 alone. Of these, VHH6 was taken forward for further characterization, due to its superior yield in the transient expression system. Data generated using ELISA (Fig. 7a), MicroScale Thermophoresis (MST, Fig. 7b) and Bio-Layer Interferometry (Octet, Fig. 7c) and analytical size exclusion chromatography (Fig. 7d) showed that VHH6 is able to recognize and bind only to the IL-6-gp80 complex.

VHH6 increases the stability of the IL6—gp80 complex

Four independent biophysical techniques strongly indicated that VHH6 specifically recognizes the complex between IL-6 and gp80. This suggested that VHH6 either recognized a conformational shift in one of the proteins or an epitope at the interface of IL-6-gp80 complex. To understand the nature of the epitope, the crystal structure of the VHH6-IL-6-gp80 complex was determined (see table 2 above). The complex crystallized in space group C 121 and was refined to 2.7 Å. The crystal structure confirmed that VHH6 binds to an epitope that spans across the interface of IL-6 and gp80. More specifically, it binds to the junction of IL-6 and domain I of gp80 with sites II and III on IL-6 remaining accessible for binding by gp130 (Fig. 8a). VHH6 binds to an epitope which is split spatially across IL-6 and gp80 almost evenly (45% of the epitope surface area lying on IL-6 and 55% on gp80).

All three of the complementarity-determining regions (CDRs) are involved in binding (Fig. 8b). Binding to IL-6 is mediated though multiple hydrogen bonds from the side chains of Tyr27, Tyr32, Ser101 and the backbone NH group of Ile102 (Fig. 8c). There is also a single salt bridge from Lys113 and a likely aliphatic interaction between Tyr27 and Glu23 of IL-6. Binding to gp80 is mediated through multiple hydrogen bonds from the backbone CO groups of Ser30, Thr31, Asn54 and the side chain of Asn74 (Fig. 8d).

To assess the contributions of the side chains to binding, single alanine substitutions at positions 27, 32, 74, 101 and 113, and double alanine substitutions at positions 27/32, 74/101 and 113/101 were generated. Either His-tagged VHH6, or mutant forms, were captured from supernatant on a nickel charged NTA chip and an equimolar mixture of IL-6 and gp80 injected over 300 s injection period. In a three-component system, it is not possible to calculate meaningful association rate constants. However, the dissociation rate constants, being concentration independent, were determined. For single residue mutants, the dissociation rates were very similar to VHH6 except for the Y27A mutant which showed a 1.9-fold increase. The dissociation rates for the double mutants all increased, with 3.8- and 4.3-fold increases observed for double mutants Asn74Ala/Ser101Ala and Ser101Ala/Lys113Ala respectively (Tables 3 and 4). These data confirmed the role in binding of the contacts identified in the crystal structure.

**Table 3: Single mutants of VHH6**

| | **koff (1/s)** |
|---|---|
| wtVHH6 | 3.6E-04 |

(continued)

| | koff (1/s) |
|---|---|
| N74A | 1.5E-04 |
| K113A | 2.6E-04 |
| Y32A | 3.0E-04 |
| S101A | 3.9E-04 |
| Y27A | 7.0E-04 |

**Table 4: Double mutants of VHH6**

| | koff (1/s) |
|---|---|
| wtVHH6 | 3.6E-04 |
| Y27A Y32A | 4.3E-04 |
| N74A S101A | 13.5E-04 |
| K113A S101A | 14.3E-04 |
| K113S S101A | 16.4E-04 |

To understand how VHH6 affects the structural dynamics of the IL-6-gp80 interaction, we used HDX-MS (Konermann, L., Pan, J. & Liu, Y.H. Hydrogen exchange mass spectrometry for studying protein structure and dynamics. Chemical Society reviews 40, 1224-1234 (2011)) (see Figure 9a-b). A lower amount of deuterium exchange is indicative of decreased structural dynamics and/or protection of that region by a binding event, whereas higher exchange indicates an increase in dynamics. Data are obtained on the peptide level, whereby changes observed can be attributed to one or more of the residues contained therein. Lower deuterium uptake was seen in the presence of VHH6 for the sequences SSERIDKQ (SEQ ID NO: 11) and KDGCFQSCFNEE (SEQ ID NO: 12) (residues 21-28 and 70-81) in IL-6 and LSVTWQD (SEQ ID NO: 13) and MVKDLQHHAVIHD (SEQ ID NO: 14) (residues 215-221 and 250-262) in gp80, each of which contains residues located within the VHH6 interface, reinforcing that the epitope spans both proteins. No data were obtained for the residues 179-184 (RALRQM; SEQ ID NO: 15) at the C-terminus of IL6, also expected to interact directly with the VHH. Peptides in the regions MTTHLILRSFKEFLQSSL (SEQ ID NO: 16) (residues 161-178, IL-6) and PEGDSSFY (SEQ ID NO: 17) and RAQEEFGQGEWSE (SEQ ID NO: 18) (residues 162-169 and 274-286, gp80) also showed a decrease in HDX in the presence of the VHH (Figure 9a, right); these are located at the IL6-gp80 interface, suggesting a tightening of the interaction here resulting in reduced structural dynamics. Finally, residues 126-144, LQKKAKNLAITTPDPTTN (SEQ ID NO: 19), in IL6 are shown to be less dynamic in the presence of the VHH6 (Figure 9a, left); indeed, these are resolved in the crystal structure of the trimer whereas are too flexible to be modelled in the structure of IL6 alone (1ALU), indicating once more an increase in structural rigidity here upon VHH binding. There is also an indication of an allosteric conformational/ dynamical change occurring on the distal side of IL6 (RKETCNKSNM, residues 40-49; SEQ ID NO: 20). For a more complete list of overlapping peptides defined by comparing HDX patterns see Tables 5 and 6.

**Table 5: Peptides in IL-6 identified and quantified by HDX-MS. Black asterisk (*) indicates deuterium uptake increasing the presence of VHH6; # indicates deuterium uptake decreasing in the presence of VHH6.**

| Start Residue | End Residue | Sequence | 2 timepoints with p<0.01 | 4 timepoints with p<0.01 | SEQ ID NO |
|---|---|---|---|---|---|
| **0** | 11 | APVPPGEDSKDV | | | 21 |
| **3** | 23 | PPGEDSKDVAAP HRQPLTSSE | | | 22 |
| **21** | 28 | SSERIDKQ | * | | 11 |
| **21** | 31 | SSERIDKQIRY | * | | 23 |
| **22** | 31 | SERIDKQIRY | * | | 24 |
| **24** | 33 | RIDKQIRYIL | * | * | 25 |
| **25** | 36 | IDKQIRYILDGI | * | * | 26 |
| **29** | 39 | IRYILDGISAL | | | 27 |
| **38** | 49 | ALRKETCNKSN M | # | | 28 |

(continued)

| Start Residue | End Residue | Sequence | 2 timepoints with p<0.01 | 4 timepoints with p<0.01 | SEQ ID NO |
|---|---|---|---|---|---|
| **39** | 49 | LRKETCNKSNM | # | | 29 |
| **40** | 49 | RKETCNKSNM | # | | 20 |
| **40** | 51 | RKETCNKSNMC E | | | 30 |
| **41** | 61 | KETCNKSNMCES SKEALAENN | # | | 31 |
| **42** | 56 | ETCNKSNMCESS KEA | | | 32 |
| **52** | 75 | SSKEALAENNLN LPKMAEKDGCF Q | | | 33 |
| **53** | 63 | SKEALAENNLN | | | 34 |
| **53** | 75 | SKEALAENNLNL PKMAEKDGCFQ | | | 35 |
| **55** | 65 | EALAENNLNLP | | | 36 |
| **58** | 67 | AENNLNLPKM | | | 37 |
| **58** | 74 | AENNLNLPKMA EKDGCF | * | * | 38 |
| **59** | 80 | ENNLNLPKMAE KDGCFQSGFNE | * | | 39 |
| **60** | 67 | NNLNLPKM | | | 40 |
| **60** | 70 | NNLNLPKMAEK | | | 41 |
| **60** | 82 | NNLNLPKMAEK DGCFQSGFNEET | * | | 42 |
| **68** | 74 | AEKDGCF | * | * | 43 |
| **70** | 81 | KDGCFQSGFNEE | * | | 44 |
| **75** | 84 | QSGFNEETCL | | | 45 |
| **85** | 92 | VKIITGLL | | | 46 |
| **85** | 93 | VKIITGLLE | | | 47 |
| **87** | 93 | IITGLLE | | | 48 |
| **98** | 108 | LEYLQNRFESS | | | 49 |
| **99** | 109 | EYLQNRFESSE | | | 50 |
| **99** | 110 | EYLQNRFESSEE | | | 51 |
| **99** | 112 | EYLQNRFESSEE QA | | | 52 |
| **99** | 120 | EYLQNRFESSEE QARAVQMSTK | | | 53 |
| **100** | 110 | YLQNRFESSEE | | | 54 |
| **100** | 112 | YLQNRFESSEEQ A | | | 55 |
| **104** | 114 | RFESSEEQARA | | | 56 |
| **106** | 112 | ESSEEQA | | | 57 |
| **106** | 116 | ESSEEQARAVQ | | | 58 |
| **109** | 116 | EEQARAVQ | | | 59 |
| **115** | 122 | VQMSTKVL | | | 60 |
| **115** | 122 | VQMSTKVL | | | 61 |
| **120** | 126 | KVLIQFL | | | 62 |
| **123** | 135 | IQFLQKKAKNLD | | | 63 |
| **126** | 133 | LQKKAKNL | * | | 64 |
| **126** | 135 | LQKKAKNLDA | * | | 65 |
| **126** | 144 | LQKKAKNLDAIT TPDPTTN | * | | 66 |

(continued)

| Start Residue | End Residue | Sequence | 2 timepoints with p<0.01 | 4 timepoints with p<0.01 | SEQ ID NO |
|---|---|---|---|---|---|
| **126** | 145 | LQKKAKNLDAIT TPDPTTNA | | | 67 |
| **126** | 147 | LQKKAKNLDAIT TPDPTTNASL | * | | 68 |
| **129** | 143 | KAKNLDAITTPD PTT | | | 69 |
| **136** | 147 | ITTPDPTTNASL | * | * | 70 |
| **148** | 155 | LTKLQAQN | | | 71 |
| **148** | 155 | LTKLQAQN | | | 72 |
| **148** | 158 | LTKLQAQNQWL | | | 73 |
| **148** | 160 | LTKLQAQNQWLQD | | | 74 |
| **152** | 158 | QAQNQWL | | | 75 |
| **159** | 165 | QDMTTHL | * | | 76 |
| **159** | 167 | QDMTTHLIL | * | | 77 |
| **161** | 167 | MTTHLIL | * | | 78 |
| **166** | 172 | ILRSFKE | * | * | 79 |
| **166** | 173 | ILRSFKEF | * | * | 80 |
| **166** | 174 | ILRSFKEFL | * | | 81 |
| **168** | 174 | RSFKEFL | * | * | 82 |
| **168** | 178 | RSFKEFLQSSL | * | * | 83 |

**Table 6: Peptides in gp80 identified and quantified by HDX-MS. Black asterisk indicates deuterium uptake increasing the presence of VHH6.**

| Start Residue | End Residue | Sequence | 2 timepoints p<0.01 | 4 timepoints with p<0.01 | SEQ ID NO |
|---|---|---|---|---|---|
| **66** | 86 | HENLYFQGLAPRR CPAQEVAR | | | 84 |
| **73** | 82 | GLAPRRCPAQ | | | 85 |
| **73** | 83 | GLAPRRCPAQE | * | | 86 |
| **73** | 85 | GLAPRRCPAQEVA | * | | 87 |
| **78** | 96 | RCPAQEVARGAG AGDVPPE | | | 88 |
| **81** | 102 | AQEVARGAGAGD VPPEEPQLSC | * | | 89 |
| **82** | 95 | QEVARGAGAGDVPP | | | 90 |
| **83** | 94 | EVARGAGAGDVP | * | | 91 |
| **84** | 100 | VARGAGAGDVPPEEPQL | * | | 92 |
| **86** | 100 | RGAGAGDVPPEEP QL | * | | 93 |
| **92** | 110 | DVPPEEPQLSCFRK SPLSN | * | | 94 |
| **94** | 100 | PPEEPQL | | | 95 |
| **103** | 111 | FRKSPLSNV | | | 96 |
| **105** | 123 | KSPLSNVVCEWGP RSTPSL | | | 97 |
| **111** | 123 | VVCEWGPRSTPSL | | | 98 |
| **112** | 123 | VCEWGPRSTPSL | | | 99 |
| **115** | 129 | WGPRSTPSLTTKAVL | | | 100 |
| **124** | 130 | TTKAVLL | | | 101 |
| **130** | 141 | LVRKFQNSPAED | | | 102 |
| **131** | 141 | VRKFQNSPAED | | | 103 |
| **135** | 145 | QNSPAEDFQEP | | | 104 |

(continued)

| Start Residue | End Residue | Sequence | 2 timepoints p<0.01 | 4 timepoints with p<0.01 | SEQ ID NO |
|---|---|---|---|---|---|
| **139** | 158 | AEDFQEPCQYSQE SQKFSCQ | | | 105 |
| **141** | 147 | DFQEPCQ | | | 106 |
| **146** | 164 | CQYSQESQKFSCQLAVPEG | | | 107 |
| **147** | 164 | QYSQESQKFSCQLAVPEG | | | 108 |
| **148** | 158 | YSQESQKFSCQ | | | 109 |
| **152** | 158 | SQKFSCQ | | | 110 |
| **157** | 170 | CQLAVPEGDSSFYI | | | 111 |
| **159** | 168 | LAVPEGDSSF | * | | 112 |
| **162** | 168 | PEGDSSF | * | | 113 |
| **174** | 183 | CVASSVGSKF | | | 114 |
| **176** | 183 | ASSVGSKF | | | 115 |
| **195** | 205 | LQPDPPANITV | * | | 116 |
| **201** | 210 | ANITVTAVAR | | | 117 |
| **206** | 216 | TAVARNPRWLS | * | | 118 |
| **207** | 214 | AVARNPRW | | | 119 |
| **208** | 214 | VARNPRW | | | 120 |
| **208** | 217 | VARNPRWLSV | * | | 121 |
| **208** | 218 | VARNPRWLSVT | * | | 122 |
| **218** | 229 | TWQDPHSWNSSF | * | | 123 |
| **221** | 229 | DPHSWNSSF | | | 124 |
| **230** | 236 | YRLRFEL | | | 125 |
| **235** | 245 | ELRYRAERSKT | | | 126 |
| **237** | 245 | RYRAERSKT | | | 127 |
| **243** | 256 | SKTFTTWMVKDL QH | * | * | 128 |
| **250** | 262 | MVKDLQHHAVIHD | * | | 14 |
| **250** | 266 | MVKDLQHHAVIHDAWSG | * | ** | 129 |
| **250** | 273 | MVKDLQHHAVIH DAWSGLRHVVQL | * | | 130 |
| **265** | 273 | SGLRHVVQL | | | 131 |
| **267** | 273 | LRHVVQL | | | 132 |
| **268** | 275 | RHVVQLRA | | | 133 |
| **274** | 283 | RAQEEFGQGE | * | ** | 134 |
| **274** | 284 | RAQEEFGQGEW | * | * | 135 |
| **274** | 286 | RAQEEFGQGEWSE | * | ** | 18 |
| **277** | 295 | EEFGQGEWSEWSP EAMGTP | | | 136 |
| **280** | 296 | GQGEWSEWSPEA MGTPW | | | 137 |
| **284** | 291 | WSEWSPEA | * | | 138 |
| **284** | 298 | WSEWSPEAMGTP WTE | | | 139 |
| **284** | 303 | WSEWSPEAMGTP WTESRSPP | | | 140 |
| **285** | 291 | SEWSPEA | | | 141 |
| **285** | 298 | SEWSPEAMGTPW TE | | | 142 |
| **285** | 303 | SEWSPEAMGTPW TESRSPP | | | 143 |
| **287** | 298 | WSPEAMGTPWTE | | | 144 |
| **289** | 303 | PEAMGTPWTESRSPP | | | 145 |
| **292** | 298 | MGTPWTE | | | 146 |

To investigate the effect of VHH6 on the binding kinetics of the IL-6-gp80 complex, a qualitative SPR experiment was performed, immobilizing IL-6 on a CM5 chip and injecting gp80. As expected for a cytokine-receptor transient interaction, both fast association and dissociation phases were observed (Fig. 10a). Injection of VHH6 at the beginning of the dissociation phase of gp80, abrogated dissociation of gp80 from immobilized IL-6. VHH6 reduced the dissociation rate constant ($k_{off}$) for gp80 binding to IL-6 by 225-fold from 0.045 $s^{-1}$ to 0.0002 $s^{-1}$ (Fig. 10a). For brevity, Fig. 10a and b only report the SPR sensorgrams for IL-6 immobilization. Similar results were obtained when gp80 or VHH6 were immobilized (Tables 7-10). These data demonstrate that not only does VHH6 bind to the IL-6-gp80 complex, but that it significantly impedes the dissociation of IL-6 from gp80.

Table 7

| IL-6 immobilised | $k_{off}$ (1/s) | SE ($k_{off}$) | $K_D$ (M) | SE ($K_D$) |
|---|---|---|---|---|
| **gp80** | 0.046 | 5.40E-05 | 3.42E-08 | 7.20E-09 |
| **gp80+VHH6 (2 μM)** | 1.94E-04 | 1.90E-07 | NA | |

**Table 8**

| gp80 immobilised | $k_{off}$ (1/s) | SE ($k_{off}$) | $K_D$ (M) | SE ($K_D$) |
|---|---|---|---|---|
| **IL-6** | 0.048 | 4.70E-05 | 1.54E-08 | 1.50E-09 |
| **IL-6+VHH6 (2 μM)** | 2.10E-04 | 2.80E-07 | NA | |

**Table 9**

| VHH6 immobilised | $k_{off}$ (1/s) | SE ($k_{off}$) | $K_D$ (M) |
|---|---|---|---|
| **IL-6+gp80 (2 μM)** | 3.09E-04 | 4.40E-07 | NA |
| **gp80+IL-6 (2 μM)** | 3.75E-04 | 6.00E-07 | NA |

**Table 10**

| gp130 immobilised | $k_{on}$ (1/Ms) | $k_{off}$ (1/s) |
|---|---|---|
| **gp80 (IL-6 (2 μM))** | 3.6 10^5 | 0.037 |
| **gp80 (IL-6 (2 μM) VHH6 (2 μM))** | 4.7 10^5 | 0.029 |
| **IL-6 (gp80 (2 μM))** | 3.6 10^5 | 0.035 |
| **IL-6 (gp80 (2 μM) VHH6 (2 μM))** | 4.4 10^5 | 0.036 |

The model created by superimposing the crystal structure solved for VHH6-IL-6-gp80 on the published data for the complex IL-6—gp80—gp130 (Boulanger, M.J., Chow, D.C., Brevnova, E.E. & Garcia, K.C. Hexameric structure and assembly of the interleukin-6/IL-6 alpha-receptor/gp130 complex. Science 300, 2101-2104 (2003)) (PDB 1P9M) indicated that VHH6 would not block the binding of IL-6—gp80 to gp130 (Fig. 14). To confirm this, gp130 extracellular domain was immobilized on the chip and a mixture containing IL-6-gp80 or VHH6-IL-6-gp80 was tested. As predicted, VHH6 did not impair the binding of the IL-6-gp80 complex to gp130 (Fig. 10c). Furthermore, stabilization by VHH6, did not affect the kinetics of IL-6—gp80 complex binding to gp130. VHH6 modulates IL-6 signaling in a cell model for *trans*-signaling

The next step in the characterization of VHH6 was to explore the cellular outcomes following the stabilization of IL-6—gp80 complex. Recruitment of gp130 by IL-6-gp80 leads to a series of phosphorylation events ultimately culminating in the phosphorylation of Signal Transducer and Activator of Transcription 3 (pSTAT3) molecules. Phosphorylated STAT3 molecules are translocated to the nuclei (Heinrich, P.C. et al. Principles of interleukin (IL)-6-type cytokine signalling and its regulation. The Biochemical journal 374, 1-20 (2003)), where they activate transcription of genes involved in the response to IL-6. IL-6-induced STAT3 phosphorylation peaks at 30 min after stimulation, and whilst largely exhausted within 60-90 min, a second wave is observed after 4 h (Wang, Y., van Boxel-Dezaire, A.H., Cheon, H., Yang, J. & Stark, G.R. STAT3 activation in response to IL-6 is prolonged by the binding of IL-6 receptor to EGF receptor. Proceedings of the National Academy of Sciences of the United States of America 110, 16975-16980 (2013)). It was postulated that adding VHH6 to IL-6 and gp80 mixture would increase the magnitude of STAT3 phosphorylation, thus mimicking the effects of HyperIL-6 *in vitro.*

To have a controlled system, IL-6 and gp80 were added in a stoichiometric ratio 1:1 to Human Umbilical Vein Endothelial Cells (HUVEC), a well-established *in vitro* system for IL-6 trans-signaling (Romano, M. et al. Role of IL-6 and its soluble receptor in induction of chemokines and leukocyte recruitment. Immunity 6, 315-325 (1997)). HUVEC cells were treated with IL-6+gp80, VHH6+IL-6+gp80, and STAT3 phosphorylation was quantified at different time points using high-throughput fluorescence microscopy. FusionIL-6 was also included as internal control in all experiments (Fig. 15). As expected, VHH6+IL-6+gp80 showed a greater response than the mixture of IL-6 and gp80, with comparable intensity to FusionIL-6 at 30 min (Fig 15), and sustained effects at later time points (Fig 11). VHH6 addition to IL-6 and gp80 mixture clearly showed an increased activation of pSTAT3 at 30 min, with pSTAT3 signal predominantly detected in the nuclear compartment (Fig. 11a). Interestingly, treatment with VHH6 promoted a significant and sustained activation of pSTAT3 at later time points (Fig. 11b-c).

STAT3 phosphorylation is an indirect proof that the stabilization of the complex is instrumental in maximizing the effects of IL-6. Indeed, further proof would be to monitor the intracellular trafficking of IL-6 in the presence or absence of VHH6. It was investigated whether increased and sustained activation of STAT3 due to VHH6 was ascribable to an altered internalization of IL-6—gp80 using IL-6 labelled with NT647. Fig. 11d shows a representative time-course experiment with accumulation of IL-6-NT647 in vesicles at 30, 180 and 360 min. Also, accumulation of IL-6-NT647 co-localized with LAMP-1, indicating that IL-6NT647 has been taken up in vesicles (Fig. 11e). VHH6 promoted a significant increase in intracellular accumulation of IL-6-NT647 as early as 15 min and increasing over time when compared to IL-6-gp80 (Fig. 11f).

To further investigate the cellular effects of extended STAT3 phosphorylation and accumulation of IL-6 in intracellular vesicles, a transcriptomic profile of HUVEC cells was performed using a qPCR array specifically designed for the IL-6 pathway. The trans-signaling response to IL-6-gp80 treatment was compared in the absence (control group) and presence of VHH6 (treated samples). A heat-map generated from three replicates together clearly suggested that, despite the variability intrinsically related to primary cells, VHH6 treatment affected only a few genes related to cytokine/chemokines or transcription factors in IL-6 signaling (Fig 16) and is related to the length of treatment. Those genes were grouped into three categories: chemokines/cytokines, transcription factors and signaling molecules (Fig. 12a-c). Of the chemokine and cytokine genes, *CCL2, IL6* and *IL6ST* transcripts increased, whilst *TNF* decreased over time. Transcription factors genes for *CEBPD* and *JUNB* were upregulated at all times, whilst *NFKB1* followed an up-downup pattern. Signaling molecule *SOCS3* was up-regulated at all times; *STAT3* and *JAK2* showed a wave-like distribution, whilst *JAK3* and *TYK2* increased over time. These results pointed towards an increased inflammatory response in HUVEC cells due to VHH6 treatment.

## Discussion

VHH6 is a prototype locking antibody which recognizes an epitope newly formed at the junction where IL-6 and gp80 interact to form a transient complex before recruiting the signaling receptor gp130. The methods described above blend several approaches to prove the junctional epitope nature of VHH6 and its ability to stabilize the complex between IL-6 and gp80 by locking together the two proteins.

HDX-MS revealed how VHH6 alters the dynamic or "breathability" which characterizes the structure of the IL-6-gp80 complex. X-ray crystallography confirmed that VHH6 clamps the two molecules together without interfering with the recruitment of gp130 into the signaling complex. Once the VHH6-IL-6-gp80 ternary complex is formed, IL-6 and gp80 are no longer able to dissociate, as shown by SPR experiments.

The alteration of the kinetics between IL-6 and gp80 has allowed the structure of the IL-6-gp80 complex to be solved in the absence of gp130. VHH6 engages simultaneously two proteins using its CDRs. VHH6 differs from bispecific antibodies, specifically engineered to target two proteins simultaneously. In the literature there are several examples of junctional antibodies. Dixin *et al* (Diskin, R., Marcovecchio, P.M. & Bjorkman, P.J. Structure of a clade C HIV-1 gp120 bound to CD4 and CD4-induced antibody reveals anti-CD4 polyreactivity. Nature structural & molecular biology 17, 608-613 (2010)), described a bimolecular epitope scFv where one CDR of the VH domain contacts CD4 and one CDR of the VL domain contacts gp120. The crystal structure of a neutralizing antibody able to stabilize hemagglutinin stem was recently described by Impagliazzo *et al* (Impagliazzo , A., et al. A stable trimeric influenza hemagglutinin stem as a broadly protective immunogen. Science 349(6254),1301-1306 (2015)), while an example of an antibody (KZ52) that engages three discontinuous segments of Ebola virus GP1 and GP2 proteins was published by Lee *et al* (Lee, J.E., et al. Structure of the Ebola virus glycoprotein bound to an antibody from a human survivor. Nature. 454(7201), 177-82 (2008)).

However, VHH6 is the prototype of an antibody which stabilizes the transient PPI between IL-6 and gp80. This is a perfect example of transient PPIs, characterized by fast association and dissociation rates, which has an important consequence from an immunological point of view to control the downstream signaling of e.g. pro-inflammatory cytokines. The key feature is that, as VHH6 binds to the complex IL-6—gp80, it effectively acts as an agonistic antibody for IL-6, as proved by the in-depth characterization ranging from crystal structure to binding properties to biological activity.

VHH6 works similarly to an entropic trap, making collisions between IL-6 and gp80 efficacious while increasing the concentration at equilibrium of the IL-6-gp80 complex. The hypothesis is that VHH6, by overcoming the entropic cost required by the system to keep IL-6 and gp80 together, allows the stable complex formed to recruit gp130 and form the hexameric signaling complex.

The ability of VHH6 to alter the interaction between IL-6 and gp80 at the junction of the two molecules, without interfering with the recruitment of the signaling partner gp130, made it possible to reproduce *in vitro* some of the features of chronic inflammation using HUVEC cells.

In the HUVEC cell model for IL-6 trans-signaling, the addition to VHH6 to IL-6 and gp80 simply translates into a faster uptake and greater intracellular accumulation of the IL-6 in lysosomal vesicles. Not only did VHH6 significantly increase the pSTAT3 signal within 30 min of treatment, but the effect extended to 360 min at a significantly greater level than that achieved with the mixture of just IL-6 and gp80.

Sustained STAT3 phosphorylation is associated with auto-inflammatory disease , such as rheumatoid arthritis (RA) (Yu, H., Pardoll, D. & Jove, R. STATs in cancer inflammation and immunity: a leading role for STAT3. Nature reviews. Cancer 9, 798-809 (2009)). To confirm that stabilization of IL-6—gp80 leads *in vitro* to a pro-inflammatory response, the transcriptomic profile of HUVEC cells treated with VHH6-IL-6-gp80 was compared to that of cells treated with IL-6-gp80. The up-regulation of genes involved in the pro-inflammatory response was directly related to the length of treatment with VHH6-IL-6-gp80. Further supporting the findings that pSTAT3 was readily translocated into the nuclei, the up-regulated genes were linked to transcription factors and cytokine activity regulators (*NFKB1, CEBPD, JUNB, STAT3, SOCS3*), kinases of the JAK family, cytokine and cytokine receptors (*IL-6* and *IL-6ST,* alias gp130) and chemokines *(CCL2,* alias monocyte chemotactic protein 1 or MCP1). For the relevance to disease and disease biomarkers, the most interesting of these genes is the one coding for MCP1. MCP1 is upregulated in synovia (Koch, A.E. et al. Enhanced production of monocyte chemoattractant protein-1 in rheumatoid arthritis. The Journal of clinical investigation 90, 772-779 (1992)) and in serum samples (Hueber, W. et al. Proteomic analysis of secreted proteins in early rheumatoid arthritis: anti-citrulline autoreactivity is associated with up regulation of proinflammatory cytokines. Annals of the rheumatic diseases 66, 712-719 (2007)) of RA patients. It has been shown that pro-inflammatory stimulation of HUVEC cells leads to the production of IL-6 and CCL2, with both contributing to an increase in endothelial permeability (Stamatovic, S.M., Keep, R.F., Kunkel, S.L. & Andjelkovic, A.V. Potential role of MCP-1 in endothelial cell tight junction 'opening': signaling via Rho and Rho kinase. Journal of cell science 116, 4615-4628 (2003) and Desai, T.R., Leeper, N.J., Hynes, K.L. & Gewertz, B.L. Interleukin-6 causes endothelial barrier dysfunction via the protein kinase C pathway. The Journal of surgical research 104, 118-123 (2002)). Therefore, the manipulation of STAT3 signaling using a junctional epitope antibody appears to reflect the modulation that can occur in a disease state.

VHH6 offers great potential in future studies. Due to its ability to stabilize and modulate the IL-6 and gp80 interaction, VHH6 could find an application in understanding the role played by IL-6 cross-talk with other cytokines or to verify the hypothesis that the switch between IL-6 (pro-inflammatory cytokine) and anti-inflammatory cytokine (e.g. IL-10), which share STAT3 as a transcription factor, might be due to an extended pSTAT3 signal (Braun, D.A., Fribourg, M. & Sealfon, S.C. Cytokine response is determined by duration of receptor and signal transducers and activators of transcription 3 (STAT3) activation. The Journal of biological chemistry 288, 2986-2993 (2013)). The therapeutic applications of VHH6 or a fusion protein VHH6-gp80 in biological systems could be explored where IL-6 plays a role as a growth factor (Galun, E. & Rose-John, S. The regenerative activity of interleukin-6. Methods in molecular biology 982, 59-77 (2013)) e.g. after injury in liver regeneration (Wuestefeld, T. et al. Interleukin-6/glycoprotein 130-dependent pathways are protective during liver regeneration. The Journal of biological chemistry 278, 11281-11288 (2003)) or activation of hepatocyte-like progenitor cells (Best, D.H., Butz, G.M. & Coleman, W.B. Cytokine-dependent activation of small hepatocyte-like progenitor cells in retrorsine-induced rat liver injury. Experimental and molecular pathology 88, 7-14 (2010)) and in expansion of hematopoietic progenitor cells (Fischer, M. et al. I. A bioactive designer cytokine for human hematopoietic progenitor cell expansion. Nature biotechnology 15, 142-145 (1997)). Lastly and particularly pertinent for drug discovery purposes, VHH6, by locking IL-6 and gp80 together, could be instrumental for screening more specific inhibitors (peptides, small molecules or even antibodies) of the IL-6-gp80 complex thus leading to a selective inhibition of IL-6 trans-signaling pathway in pathologies.

In conclusion, the data generated in this proof-of-concept study demonstrates the functional consequences of manipulating the interface between two proteins by stabilizing their interaction with a complex-specific antibody. Using antibodies, instead of fusion protein constructs, offers the advantage of proteins with well-defined chemical-physical characteristics and the capability of preserving the correct conformation of the complex as a functional unit. Junctional epitope antibodies like VHH6, which lock together and stabilize a transient PPI, would be an excellent tool to explore novel biological hypothesis. Ultimately this may lead to a different way of approaching drug discovery and therapeutic interventions, when the target is not a single molecule but a multiprotein complex.

## Example 2 - Use of a junctional binding protein in cross-linking antibody fusions and their antigens

This Example illustrates how use of a junctional binding protein to cross-link two antibody fusions and thereby their associated antigens may be tested. This is based on assays described in WO 2015/181282 (see for example Examples 8/9/10). WO 2015/181282 provides an example of general bispecific targeting which only provides a function when both markers are targeted. WO 2016/009030 and WO 2016/009029 present more specific examples, where CD22/CD79 and CD45/CD79 are targeted.

*General method 1*

Human PBMC derived from platelet apheresis cones are banked as frozen aliquots. Prior to an assay being performed, cells are thawed, washed in DMEM (Life Technologies) and allowed to acclimatise to a 37 °C and 5% $CO_2$ environment.

*General method 2*

Fab'A-Component A (inactive-IL-6) and Fab'B-Component B (inactive GP80) are incubated together for 90 minutes (in a 37 °C/ 5% $CO_2$ environment) either in the presence of absence of the junctional antibody VHH6 before mixing with $2.5\times10^5$ PBMC in V-bottomed 96 well plates. PBMC plus the cross-linked (Fab'A-Component A and Fab'B-Component B and VHH6) or non-cross-linked (Fab'A-Component A and Fab'B-Component B) combinations are then incubated together for a further 90 minutes. After this time B cells are activated by the addition of 200 nM of goat $F(ab')_2$ anti-human IgM (Southern Biotechnology) for 8 minutes at 37 °C. The signalling reaction is then halted by adding an equal volume of Cytofix buffer (BD Biosciences). Plates are then left at room temperature for 15 minutes before centrifugation at 500g for 5 minutes. Excess supernatant is discarded from the cell pellet which is resuspended in flow buffer and washed once more. Cells are then resuspended in ice cold Perm Buffer III (BD Biosciences) for 30 minutes before being washed twice in flow buffer.

*General method 3*

Cells are activated as described in general method 2 and stained with a fluorescently labelled anti-CD20 antibody (BD Biosciences), anti-phospho Akt antibody that recognises a modified serine residue at position 473, an anti-phospho PLCg2 antibody that recognises a modified tyrosine residue at position 759 and an anti-IκB antibody that recognises total Iκ**B**. Plates are then resuspended and incubated for 1 hour at room temperature in the dark. After this time plates were washed a further two times and resuspended in 25 μl of flow buffer. Cellular expression of CD20, Akt and PLCg2 is measured using an Intellicyt HTFC™ flow cytometer

## Example 3 - *In silico* generation of humanized sequences of the VHH6 antibody

Llama VHH6 was humanized by grafting the CDRs from the Llama VHH-region onto a human germline antibody VH-region framework. A number of framework residues from the Llama VHH-region were also retained in the humanized sequence. These residues were selected using the protocol outlined by Adair et al. (Humanised antibodies. WO91/09967). Alignments of the Llama VHH (donor) V-region sequence with the human germline (acceptor) V-region sequence are shown in Figure 18, together with the designed humanized sequences.

The CDRs grafted from the donor to the acceptor sequence are as defined by Kabat *et al.* (Kabat et al., Sequences of proteins of immunological interest, 4th ed, pp. vii-804 (1987)), with the exception of CDR-H1 where the combined Chothia/Kabat definition is used (see Adair et al., 1991 Humanised antibodies. WO91/09967). Human V-region IGHV3-74 plus IGHJH4 J-region (http://www.imgt.org/) was chosen as the acceptor for the VHH CDRs. The heavy chain framework residues in graft gH1 are all from the human germline gene, with the exception of residues 4, 24, 37, 44, 45, 46, 47, 49, 71, 78 and 94 (Kabat numbering), where the donor residues Phenylalanine (F4), Threonine (T24), Phenylalanine (F37), Glutamic acid (E44), Arginine (R45), Glutamic acid (E46), Glycine (G47), Alanine (A49), Glutamine (Q71), Valine (V78) and Alanine (A94) were retained, respectively.

## Informal Sequence Listing

**SEQ ID NO: 1 (VHH6 heavy chain variable region)**

DVQFVESGGGSVHAGGSLRLNCATSGYIYSTYCMGWFRQAPGKEREGVAHIYTNSGRTYYA
DSVKGRFTISQDNAKNTVYLQMNSLKPEDTAIYYCAARPSIRCASFSATEYKDWGQGTQVT
VSS

**SEQ ID NO: 2 (VHH6 CDR1)**
GYIYSTYCMG

**SEQ ID NO: 3 (VHH6 CDR2)**
HIYTNSGRTYYADSVKG

**SEQ ID NO: 4 (VHH6 CDR3)**
RPSIRCASFSATEYKD

**SEQ ID NO: 5 (VHH3 heavy chain variable region)**

GATGTGCAGTTCGTGGAGTCTGGGGGAGGCTCGGTGCACGCTGGAGGGTCTCTAAGACTCA
ACTGTGCTACCTCTGGATACATTTACAGTACCTATTGCATGGGATGGTTCCGCCAGGCTCC
AGGGAAGGAGCGTGAGGGGGTCGCACATATTTATACGAATAGTGGACGCACATACTATGCC
GACTCCGTGAAGGGCCGATTCACCATCTCCCAAGACAACGCCAAGAACACGGTGTATCTGC
AAATGAACAGCCTGAAACCTGAGGACACGGCCATCTATTACTGTGCGGCCCGTCCCAGTAT
TAGGTGTGCTTCCTTCAGCGCCACCGAATATAAGGACTGGGGCCAGGGGACCCAGGTCACC
GTCTCCTCG

**SEQ ID NO: 6 (VHH6 CDR1)**
GGATACATTTACAGTACCTATTGCATGGGA

**SEQ ID NO: 7 (VHH6 CDR2)**
CATATTTATACGAATAGTGGACGCACATACTATGCCGACTCCGTGAAGGGC

**SEQ ID NO: 8 (VHH6 CDR3)**
CGTCCCAGTATTAGGTGTGCTTCCTTCAGCGCCACCGAATATAAGGAC

**SEQ ID NO: 9 (IL-6 from Figure 9)**

GSAPVPPGED SKDVAAPHRQ PLTSSERIDK QIRYILDGIS ALRKETCNKS
NMCESSKEAL AENNLNLPKM AEKDGCFQSG FNEETCLVKI ITGLLEFEVY

LEYLQNRFES SEEQARAVQM STKVLIQFLQ KKAKNLDAIT TPDPTTNASL
LTKLQAQNQW LQDMTTHLIL RSFKEFLQSS LRALRQM

**SEQ ID NO: 10 (gp80 from Figure 9)**

GLAP RRCPAQEVAR GAGAGDVPPE EPQLSCFRKS PLSNVVCEWG
PRSTPSLTTK AVLLVRKFQN SPAEDFQEPC QYSQESQKFS CQLAVPEGDS
SFYIVSMCVA SSVGSKFSKT QTFQGAGILQ PDPPANITVT AVARNPRWLS
VTWQDPHSWN SSFYRLRFEL RYRAERSKTF TTWMVKDLQH HAVIHDAWSG
LRHVVQLRAQ EEFGQGEWSE WSPEAMGTPW TESRSPP

**SEQ ID NOs: 11-147**
Sequences shown in the Examples

**SEQ ID NO: 147 (VHH6gH1 heavy chain variable region)**

EVQFVESGGGLVQPGGSLRLSCATSGYIYSTYCMGWFRQAPGKEREGVAHIYTNSGRTYYA
DSVKGRFTISQDNAKNTVYLQMNSLRAEDTAVYYCAARPSIRCASFSATEYKDWGQGTLVT
VSS

**SEQ ID NO: 148 (VHH6gH2 heavy chain variable region)**

EVQFVESGGGLVQPGGSLRLSCATSGYIYSTYSMGWFRQAPGKEREGVAHIYTNSGRTYYA
DSVKGRFTISQDNAKNTVYLQMNSLRAEDTAVYYCAARPSIRSASFSATEYKDWGQGTLVT
VSS

**SEQ ID NO: 149 (VHH6gH1 CDR1)**
GYIYSTYCMG

**SEQ ID NO: 150 (VHH6gH2 CDR1)**
GYIYSTYSMG

**SEQ ID NO: 151 (VHH6gH1 and VHH6gH2 CDR2)**
HIYTNSGRTYYADSVKG

**SEQ ID NO: 152 (VHH6gH1 CDR3)**
RPSIRCASFSATEYKD

**SEQ ID NO: 153 (VHH6gH2 CDR3)**
RPSIRSASFSATEYKD

**SEQ ID NO: 154 (Human IGHV3-74 IGHJH4 acceptor frawork)**

EVQLVESGGGLVQPGGSLRLSCAASGFTFSSYWMHWVRQAPGKGLVWVSRINSDGSSTSYA
DSVKGRFTISRDNAKNTLYLQMNSLRAEDTAVYYCARYFDYWGQGTLVTVSS

## Claims

1. A method of producing an antibody specific for a junctional epitope created by a transient protein:protein interaction, which antibody stabilises the protein:protein interaction, said method comprising:

   (i) immunising an animal with a fusion protein of the stabilised transient protein:protein interaction;
   (ii) selecting an antibody which interacts with the stabilised complex of the protein:protein interaction but not with each individual component of the protein:protein interaction in the absence of the other; and
   (iii) determining that the antibody stabilises the protein:protein interaction.

2. The method of claim 1, wherein said antibody is selected if it

   (a) has no detectable binding to each individual component of the protein:protein interaction in the absence of the other; and/or
   (b) has a dissociation constant ($K_D$) for binding to the protein:protein interaction that is at least 10 fold lower than the $K_D$ for binding to each individual protein component of the protein:protein interaction.

3. The method of claim 1 or 2, wherein the two components of the transient protein:protein interaction have a dissociation rate ($k_{off}$) in the absence of the antibody of greater than 0.01 $s^{-1}$

4. The method of any one of claims 1-3, wherein said antibody is selected if in the presence of the antibody the two components of the transient protein:protein interaction have a dissociation rate ($k_{off}$) of less than 0.01 $s^{-1}$.

5. The method of claims 3 or 4, wherein the $k_{off}$ is measured by surface plasmon resonance at 25 °C.

6. The method of claim 1, wherein said antibody specifically binds at or over the junction created when the two protein components of a transient protein:protein interaction form a complex, and does not recognise a site created when the two protein components of the protein:protein interaction come together and that is located away from the protein:protein interface.

7. The method of claim 1, wherein said antibody is selected if it binds an epitope which spans across the interface between the two protein components of the protein:protein interaction complex.

8. The method of claim 1, wherein said animal is a camel and said antibody is a VHH antibody.

9. The method of claim 1, wherein in said fusion protein one of the protein components of the protein:protein interaction is fused directly onto the terminus of the other protein.

10. The method of claim 1, wherein said antibody is selected if the dissociation rate ($k_{off}$) for the transient protein:protein interaction is reduced at least 10 fold.

11. The method of claim 1, wherein said antibody is selected if the dissociation constant ($K_D$) for the protein:protein interaction is reduced at least 10 fold.

12. The method of claim 1, wherein the heavy chain variable region ($V_H$) of the antibody or fragment thereof has interactions with both proteins of the protein:protein interaction, the light chain variable region ($V_L$) has interactions with both proteins of the protein:protein interaction, or both the $V_H$ and $V_L$ have interactions with both proteins of the protein:protein interaction

13. The method of claim 12, wherein one or more individual complementarity determining regions (CDRs) of the $V_H$ and/or $V_L$ of the antibody interact with both components of the protein:protein interaction.

14. The method of claim 1, wherein said antibody is a Fab, scFv or VHH

**Patentansprüche**

1. Verfahren zur Erzeugung eines für ein durch eine transiente Protein:Protein-Wechselwirkung geschaffenes Verbindungsstellenepitop spezifischen Antikörpers, wobei der Antikörper die Protein:Protein-Wechselwirkung stabilisiert, wobei das Verfahren Folgendes umfasst:

   (i) Immunisieren eines Tiers mit einem Fusionsprotein der stabilisierten transienten Protein:Protein-Wechselwirkung;
   (ii) Selektieren eines Antikörpers, der mit dem stabilisierten Komplex der Protein:Protein-Wechselwirkung, aber nicht mit einer der Einzelkomponenten der Protein:Protein-Wechselwirkung in Abwesenheit der jeweils anderen Einzelkomponente wechselwirkt; und
   (iii) Bestimmen, dass der Antikörper die Protein:Protein-Wechselwirkung stabilisiert.

2. Verfahren nach Anspruch 1, wobei der Antikörper selektiert wird, falls er

   (a) keine nachweisbare Bindung an eine der Einzelkomponenten der Protein:Protein-Wechselwirkung in Abwesenheit der jeweils anderen Einzelkomponente aufweist; und/oder
   (b) eine Dissoziationskonstante ($K_D$) für die Bindung an die Protein:Protein-Wechselwirkung aufweist, die wenigstens um das 10-Fache niedriger ist als die $K_D$ für die Bindung an jede einzelne Proteinkomponente der Protein:Protein-Wechselwirkung.

3. Verfahren nach Anspruch 1 oder 2, wobei die beiden Komponenten der transienten Protein:Protein-Wechselwirkung eine Dissoziationsgeschwindigkeitskonstante ($k_{off}$) größer 0,01 $s^{-1}$ in Abwesenheit des Antikörpers aufweisen.

4. Verfahren nach einem der Ansprüche 1-3, wobei der Antikörper selektiert wird, falls die beiden Komponenten der transienten Protein:Protein-Wechselwirkung in Gegenwart des Antikörpers eine Dissoziationsgeschwindigkeitskonstante ($k_{off}$) kleiner 0,01 $s^{-1}$ aufweisen.

5. Verfahren nach Anspruch 3 oder 4, wobei die $k_{off}$ mit Oberflächenplasmonenresonanz bei 25 °C gemessen wird.

6. Verfahren nach Anspruch 1, wobei der Antikörper an oder über der Verbindungsstelle, die beim Ausbilden eines Komplexes durch die beiden Proteinkomponenten einer transienten Protein:Protein-Wechselwirkung geschaffen wurde, spezifisch bindet und eine Stelle nicht erkennt, die beim Zusammenkommen der beiden Proteinkomponenten der transienten Protein:Protein-Wechselwirkung geschaffen wurde und die weg von der Protein:Protein-Grenzfläche liegt.

**7.** Verfahren nach Anspruch 1, wobei der Antikörper selektiert wird, falls er ein Epitop bindet, das sich über die Grenzfläche zwischen den beiden Proteinkomponenten des Protein:Protein-Wechselwirkungskomplexes erstreckt.

**8.** Verfahren nach Anspruch 1, wobei es sich bei dem Tier um ein Kamel und bei dem Antikörper um einen VHH-Antikörper handelt.

**9.** Verfahren nach Anspruch 1, wobei in dem Fusionsprotein eine der Proteinkomponenten der Protein:Protein-Wechselwirkung direkt an den Terminus des anderen Proteins fusioniert ist.

**10.** Verfahren nach Anspruch 1, wobei der Antikörper selektiert wird, falls die Dissoziationsgeschwindigkeitskonstante ($k_{off}$) für die transiente Protein:Protein-Wechselwirkung um wenigstens das 10-Fache verringert ist.

**11.** Verfahren nach Anspruch 1, wobei der Antikörper selektiert wird, falls die Dissoziationskonstante ($K_D$) für die Protein:Protein-Wechselwirkung um wenigstens das 10-Fache verringert ist.

**12.** Verfahren nach Anspruch 1, wobei die Schwere-Kettevariable-Region ($V_H$) des Antikörpers oder Fragments davon Wechselwirkungen mit beiden Proteinen der Protein:Protein-Wechselwirkung hat, die Leichte-Kette-variable-Region ($V_L$) Wechselwirkungen mit beiden Proteinen der Protein:Protein-Wechselwirkung hat oder sowohl die $V_H$ als auch die $V_L$ Wechselwirkungen mit beiden Proteinen der Protein:Protein-Wechselwirkung haben.

**13.** Verfahren nach Anspruch 12, wobei eine oder mehrere individuelle CDR (Complementarity Determining Regions) der $V_H$ und/oder $V_L$ des Antikörpers mit beiden Proteinen der Protein:Protein-Wechselwirkung wechselwirken.

**14.** Verfahren nach Anspruch 1, wobei es sich bei dem Antikörper um ein Fab, scFv oder VHH handelt.

## Revendications

**1.** Procédé de production d'un anticorps spécifique pour un épitope de jonction créé par une interaction protéine:protéine transitoire, ledit anticorps stabilisant l'interaction protéine:protéine, ledit procédé comprenant :

(i) l'immunisation d'un animal avec une protéine de fusion de l'interaction protéine-protéine transitoire stabilisée ;
(ii) la sélection d'un anticorps qui interagit avec le complexe stabilisé de l'interaction protéine-protéine mais pas avec chaque composant individuel de l'interaction protéine:protéine en l'absence de l'autre ; et
(iii) la détermination du fait que l'anticorps stabilise l'interaction protéine-protéine.

**2.** Procédé selon la revendication 1, dans lequel ledit anticorps est sélectionné s'il

(a) ne présente pas de liaison détectable à chaque composant individuel de l'interaction protéine-protéine en l'absence de l'autre ; et/ou
(b) présente une constante thermodynamique de dissociation ($K_D$) pour la liaison à l'interaction protéine-protéine qui est au moins 10 fois plus faible que le $K_D$ pour la liaison à chaque composant protéique individuel de l'interaction protéine-protéine.

**3.** Procédé selon la revendication 1 ou 2, dans lequel les deux composants de l'interaction protéine-protéine transitoire ont une constante cinétique de dissociation ($k_{off}$) en l'absence de l'anticorps supérieure à 0,01 $s^{-1}$.

**4.** Procédé selon l'une quelconque des revendications 1 à 3, dans lequel ledit anticorps est sélectionné si, en présence de l'anticorps, les deux composants de l'interaction protéine-protéine transitoire ont une constante cinétique de dissociation ($k_{off}$) inférieure à 0,01 $s^{-1}$.

**5.** Procédé selon les revendications 3 ou 4, dans lequel $k_{off}$ est mesuré par résonance des plasmons de surface à 25 °C.

**6.** Procédé selon la revendication 1, dans lequel ledit anticorps se lie spécifiquement à ou sur la jonction créée lorsque les deux composants protéiques d'une interaction protéine:protéine transitoire forment un complexe, et ne reconnaît pas un site créé lorsque les deux composants protéiques de l'interaction protéine-protéine sont assemblés et qui est situé à l'écart de l'interface protéine:protéine.

**7.** Procédé selon la revendication 1, dans lequel ledit anticorps est sélectionné s'il se lie à un épitope qui couvre l'interface entre les deux composants protéiques du complexe d'interaction protéine-protéine.

**8.** Procédé selon la revendication 1, dans lequel ledit animal est un chameau et ledit anticorps est un anticorps VHH.

**9.** Procédé selon la revendication 1, dans lequel, dans ladite protéine de fusion, l'un des composants protéiques de l'interaction protéine:protéine est fusionné directement sur la terminaison de l'autre protéine.

**10.** Procédé selon la revendication 1, dans lequel ledit anticorps est sélectionné si la constante cinétique de dissociation ($k_{off}$) pour l'interaction protéine:protéine transitoire est réduite d'au moins 10 fois.

**11.** Procédé selon la revendication 1, dans lequel ledit anticorps est sélectionné si la constante thermodynamique de dissociation ($K_D$) pour l'interaction protéine-protéine est réduite d'au moins 10 fois.

**12.** Procédé selon la revendication 1, dans lequel la région variable de chaîne lourde ($V_H$) de l'anticorps ou un fragment de celui-ci présente des interactions avec les deux protéines de l'interaction protéine: protéine, la région variable de chaîne légère ($V_L$) présente des interactions avec les deux protéines de l'interaction protéine:protéine, ou $V_H$ et $V_L$ présentent toutes deux des interactions avec les deux protéines de l'interaction protéine-protéine.

**13.** Procédé selon la revendication 12, dans lequel une ou plusieurs régions déterminant la complémentarité (CDR) individuelles des $V_H$ et/ou $V_L$ de l'anticorps interagissent avec les deux composants de l'interaction protéine-protéine.

**14.** Procédé selon la revendication 1, dans lequel ledit anticorps est un Fab, scFv ou VHH.

Figure 1

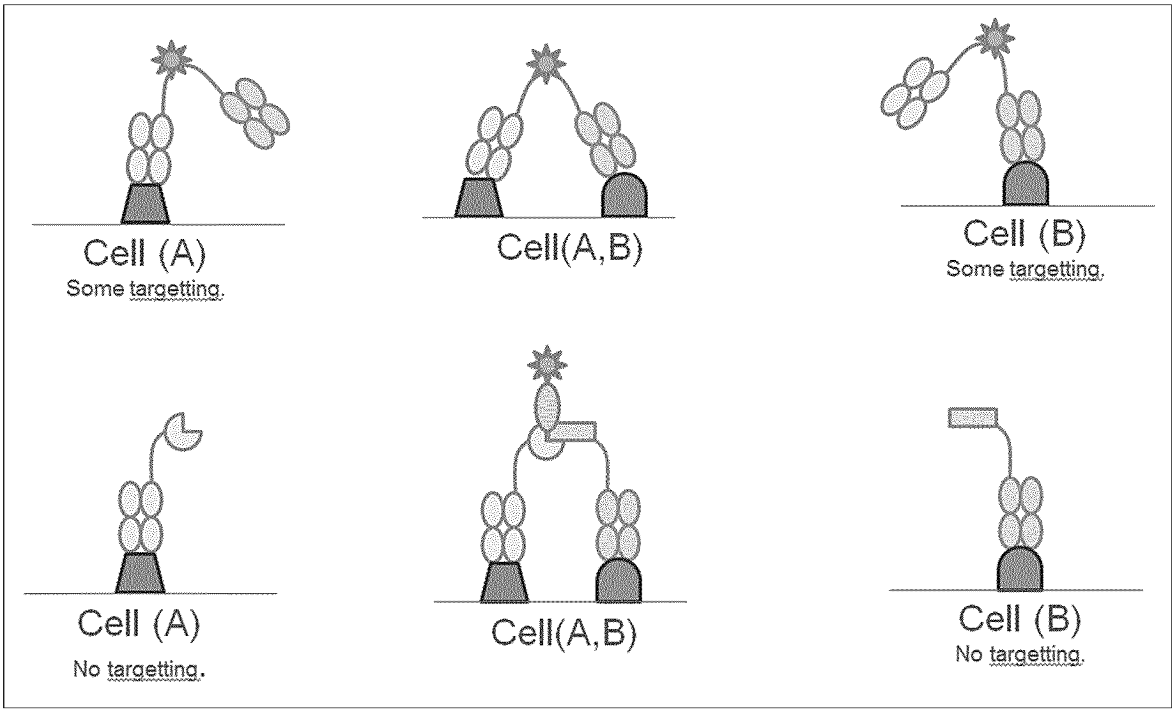


Figure 2

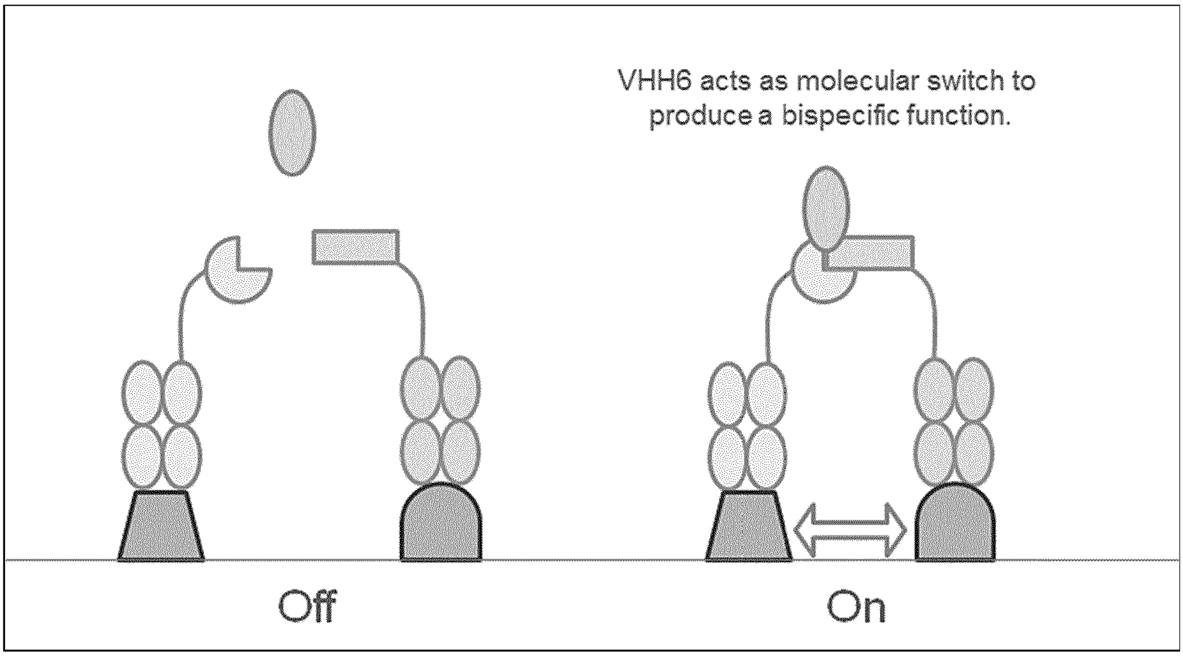

Figure 3

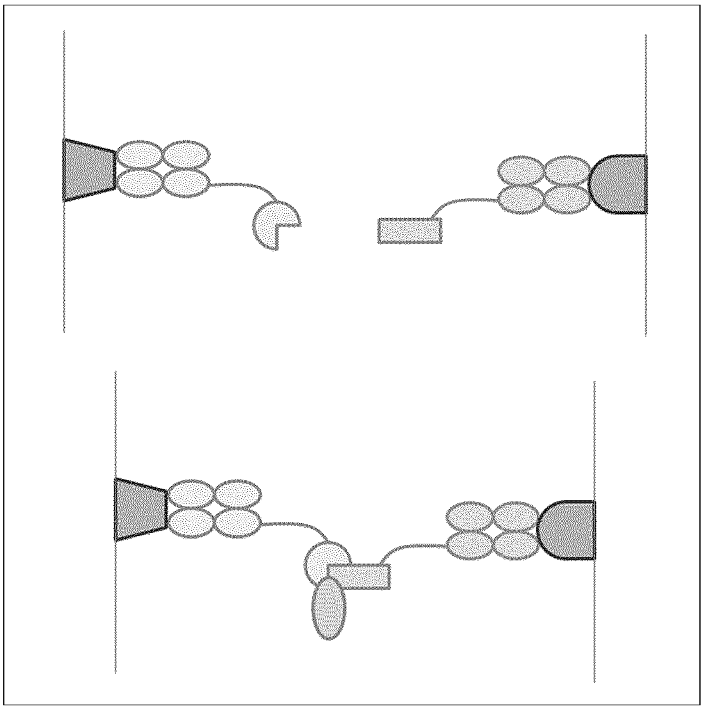

Figure 4

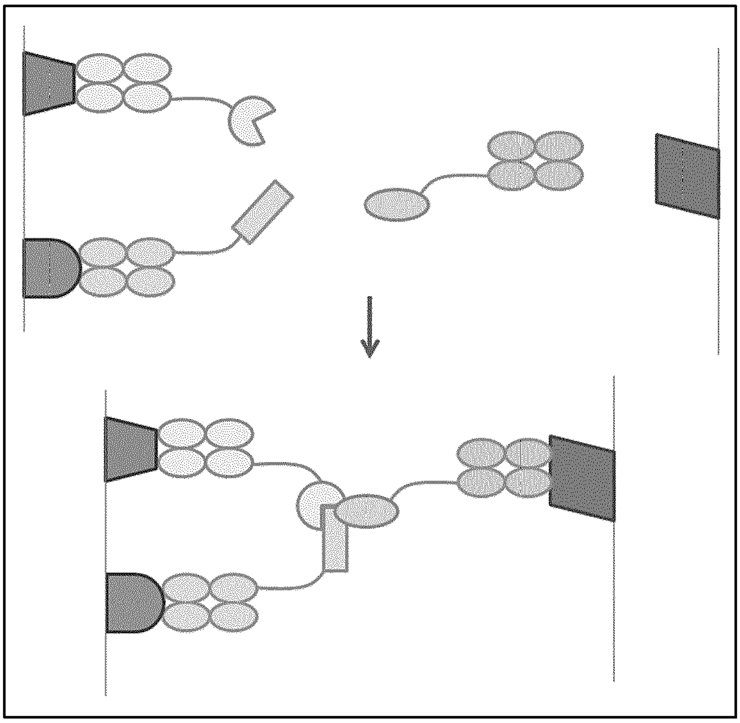

Figure 5

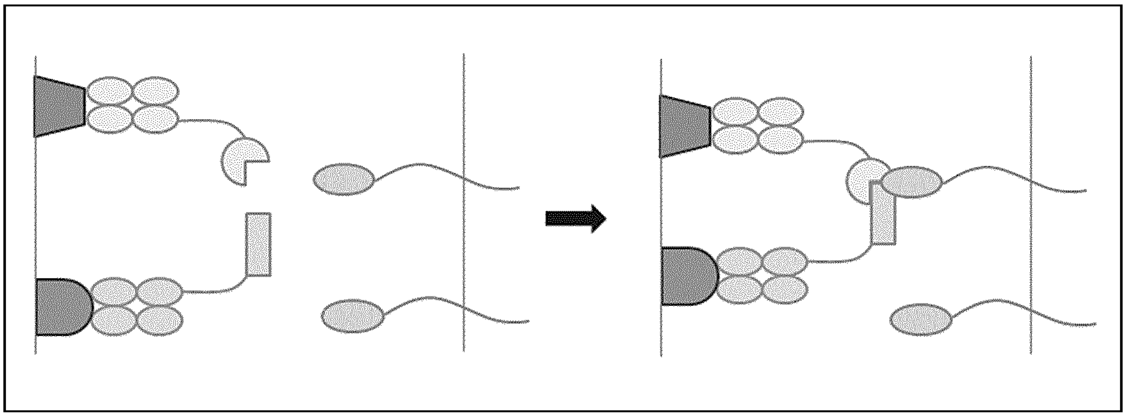

Figure 6

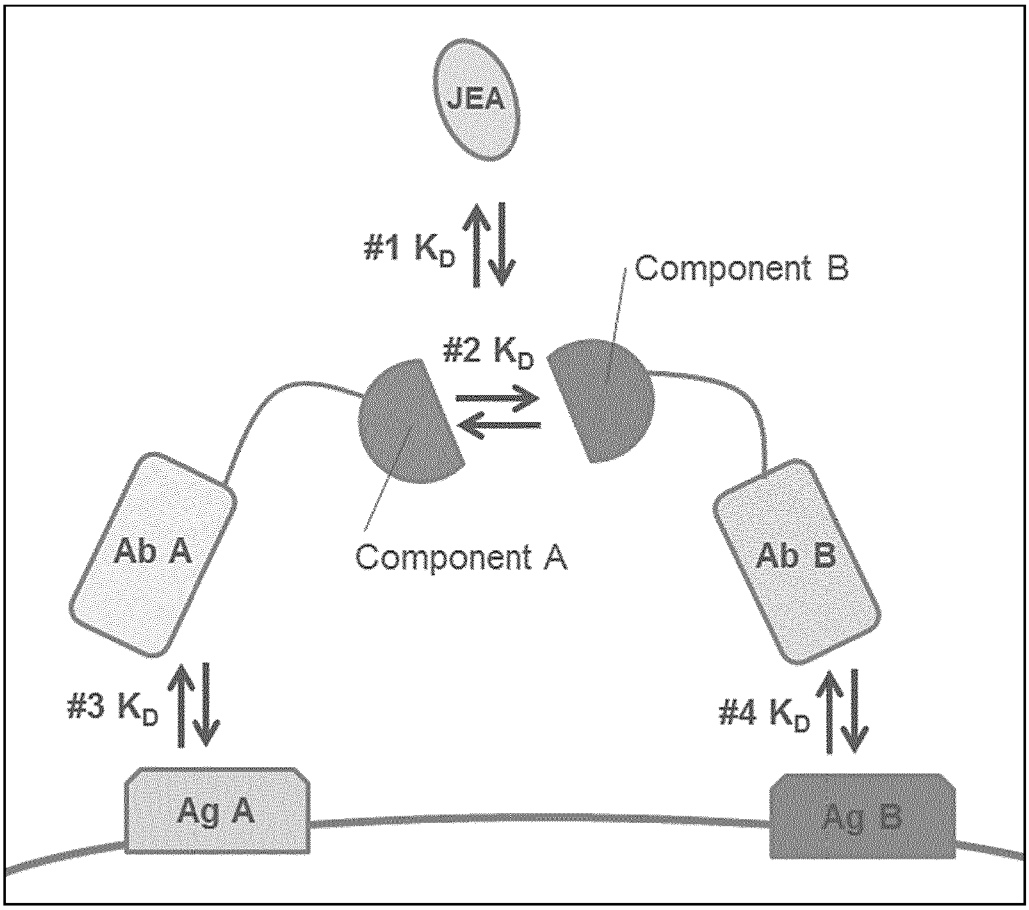

Figure 7

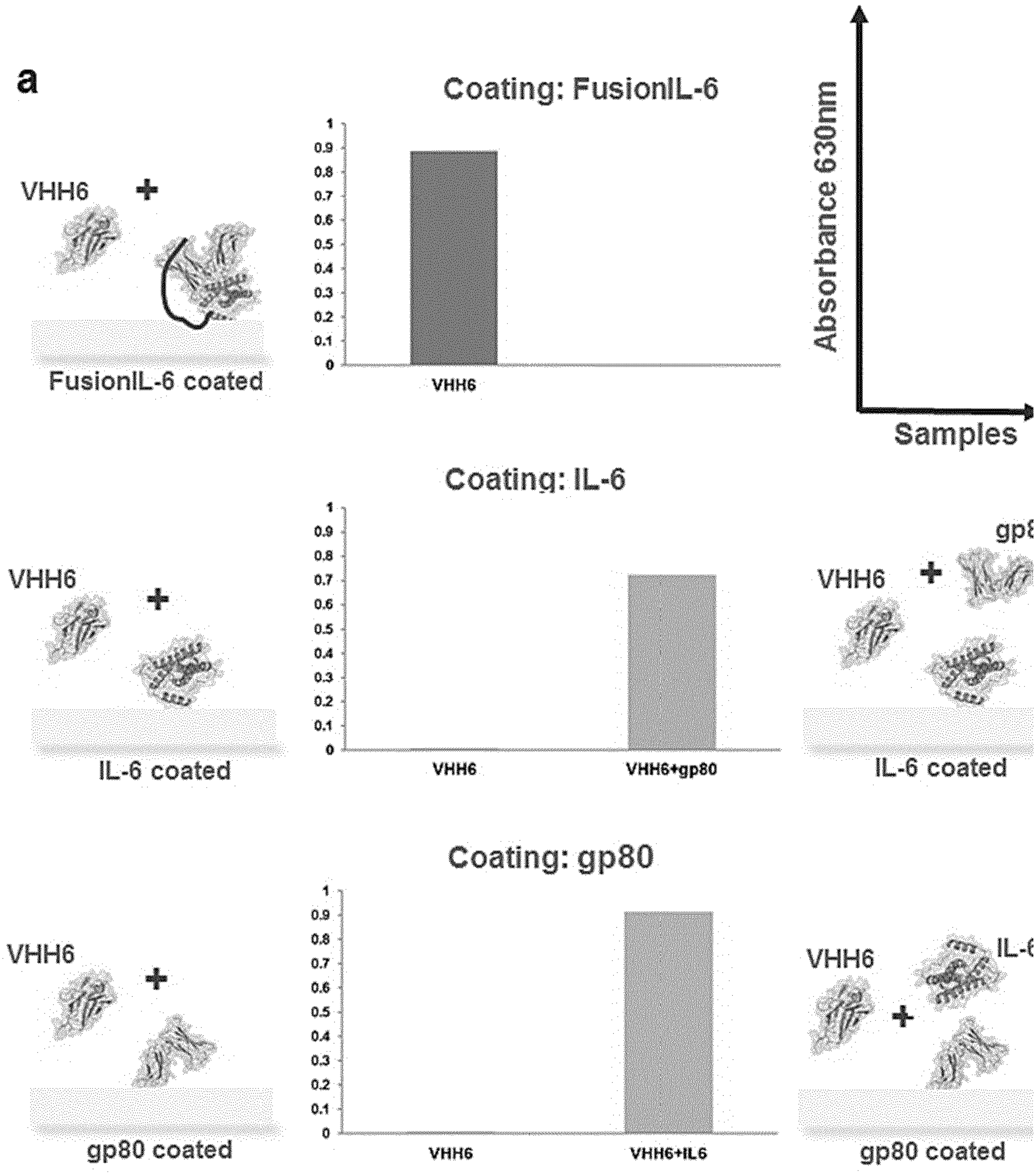
a
Coating: FusionIL-6
VHH6
FusionIL-6 coated
Absorbance 630nm
Samples
Coating: IL-6
VHH6
VHH6+gp80
IL-6 coated
Coating: gp80
VHH6+IL6
gp80 coated

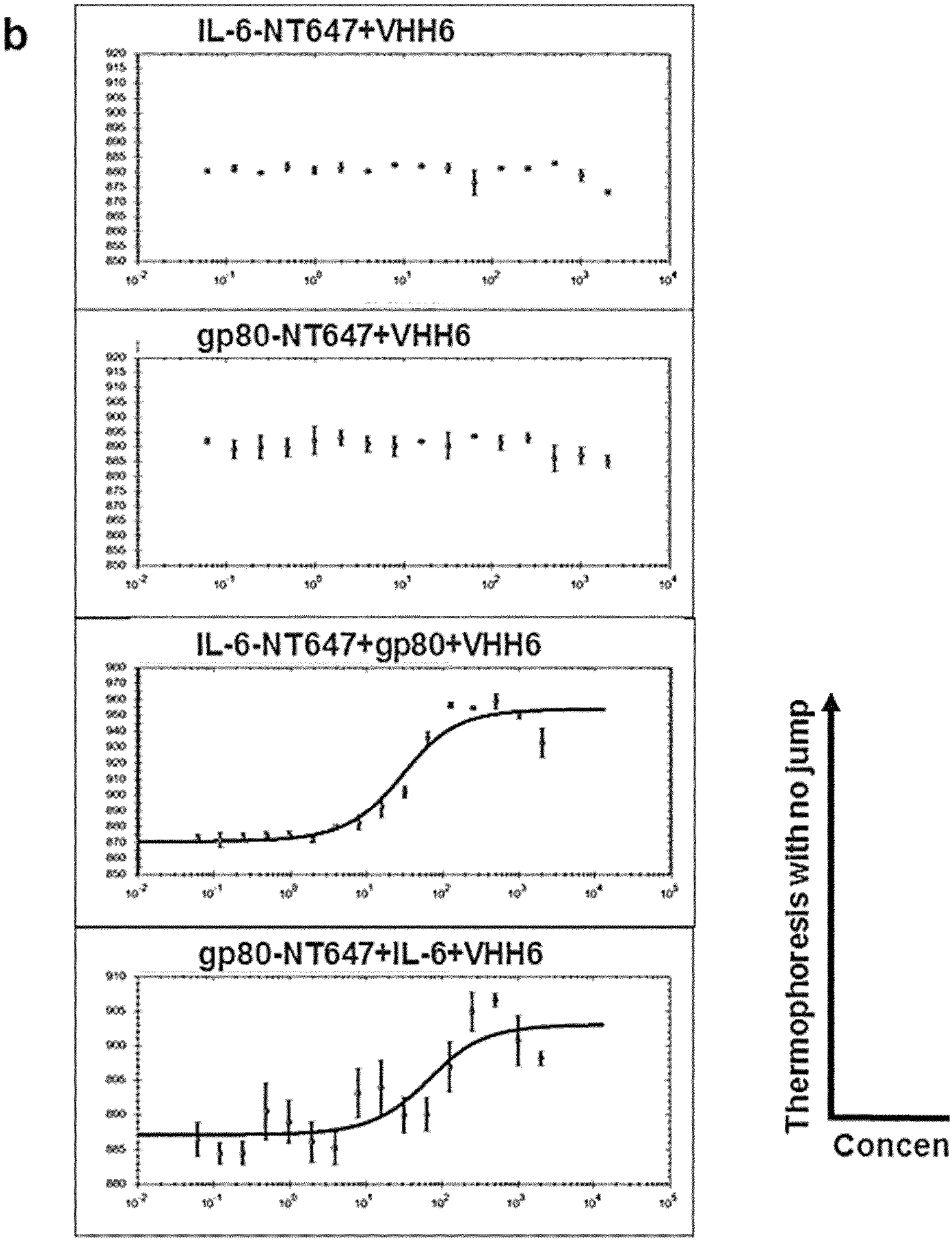
b
IL-6-NT647+VHH6
gp80-NT647+VHH6
IL-6-NT647+gp80+VHH6
gp80-NT647+IL-6+VHH6
Thermophoresis with no jump
Concen

C

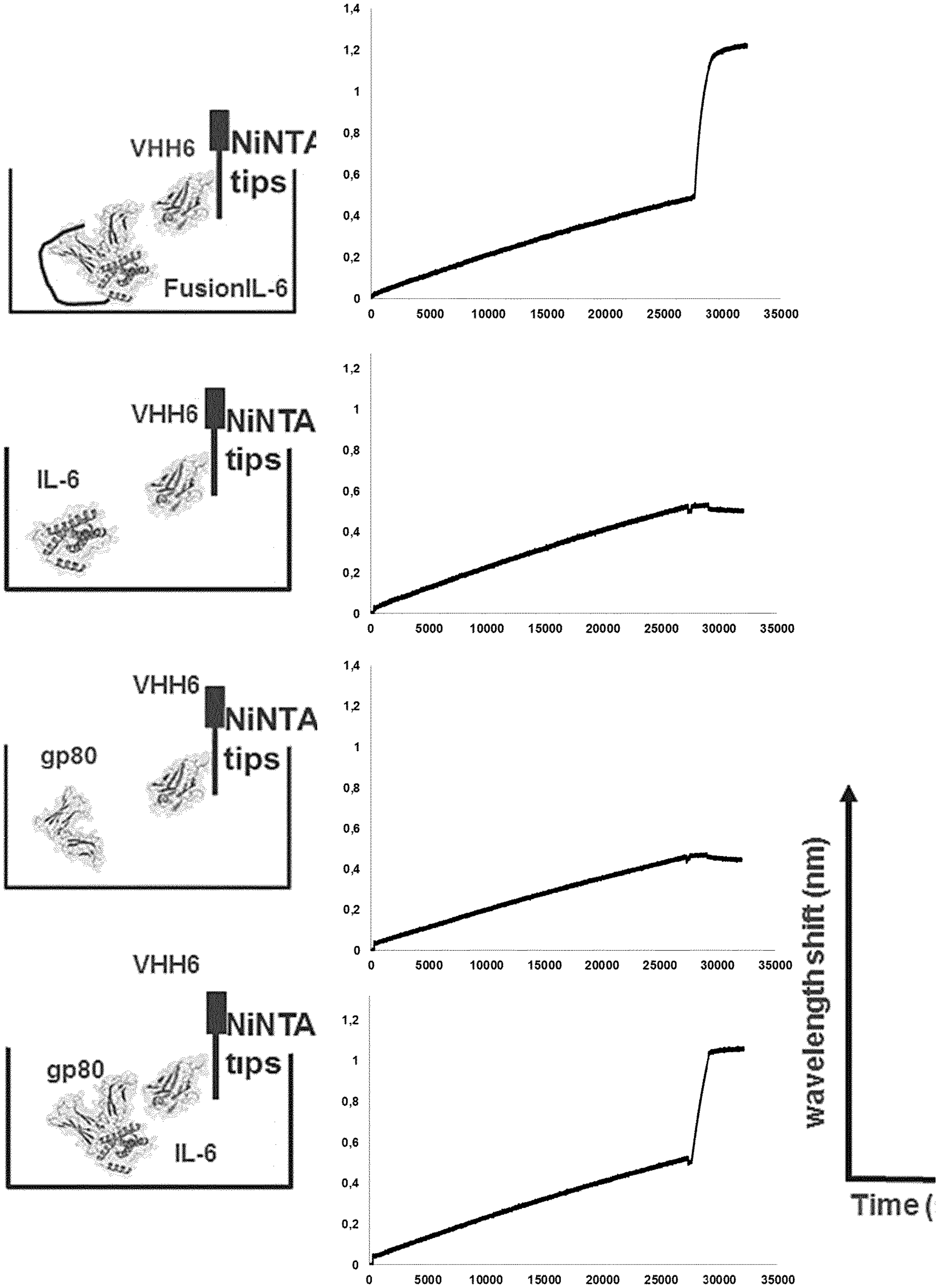
VHH6
NiNTA
tips
FusionIL-6
1,4
1,2
1
0,8
0,6
0,4
0,2
0
0
5000
10000
15000
20000
25000
30000
35000
VHH6
NiNTA
tips
IL-6
VHH6
NiNTA
tips
gp80
VHH6
NiNTA
tips
gp80
IL-6
wavelength shift (nm)
Time (

d

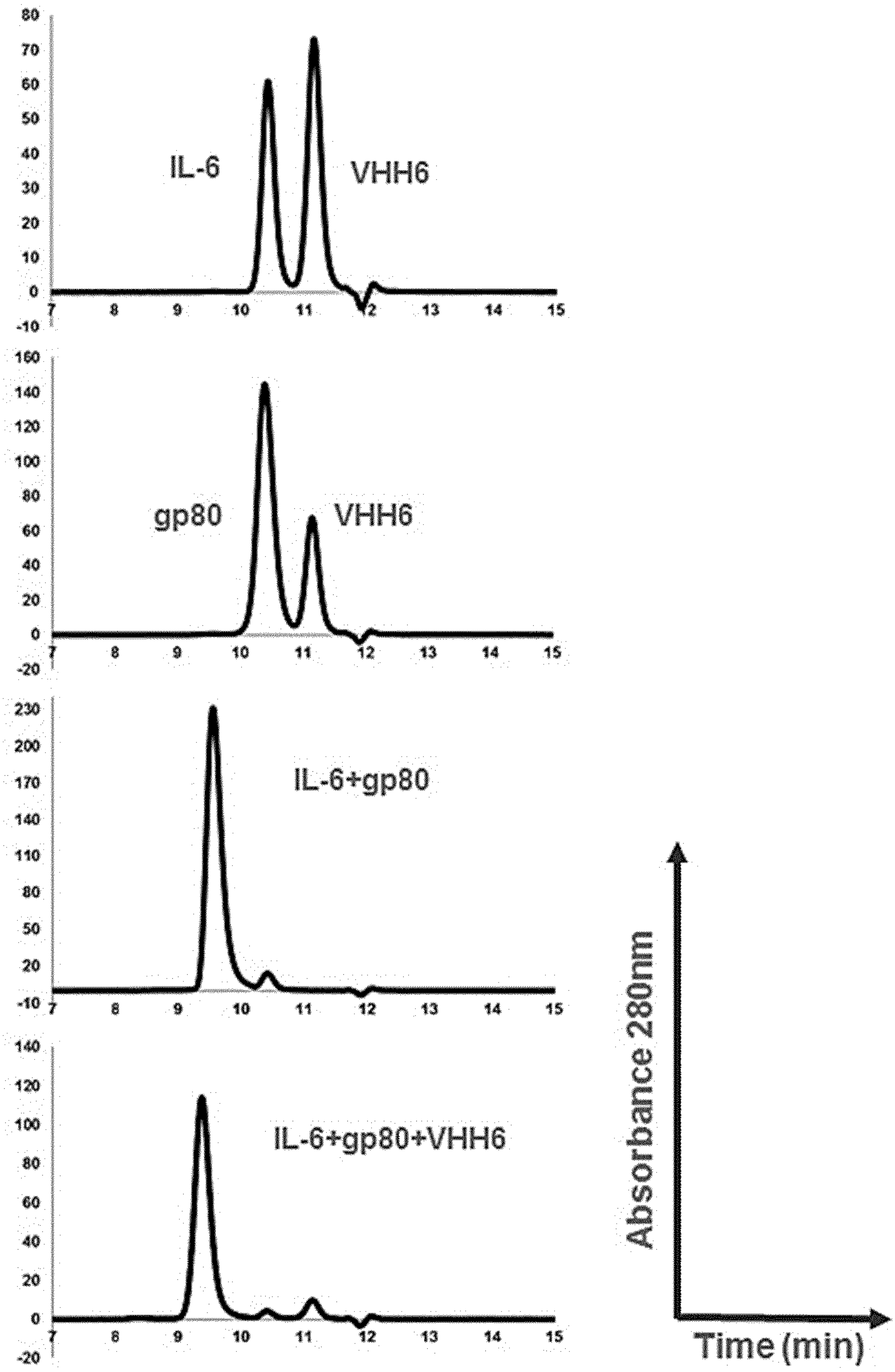
IL-6
VHH6
gp80
VHH6
IL-6+gp80
IL-6+gp80+VHH6
Absorbance 280nm
Time (min)

**Figure 8**

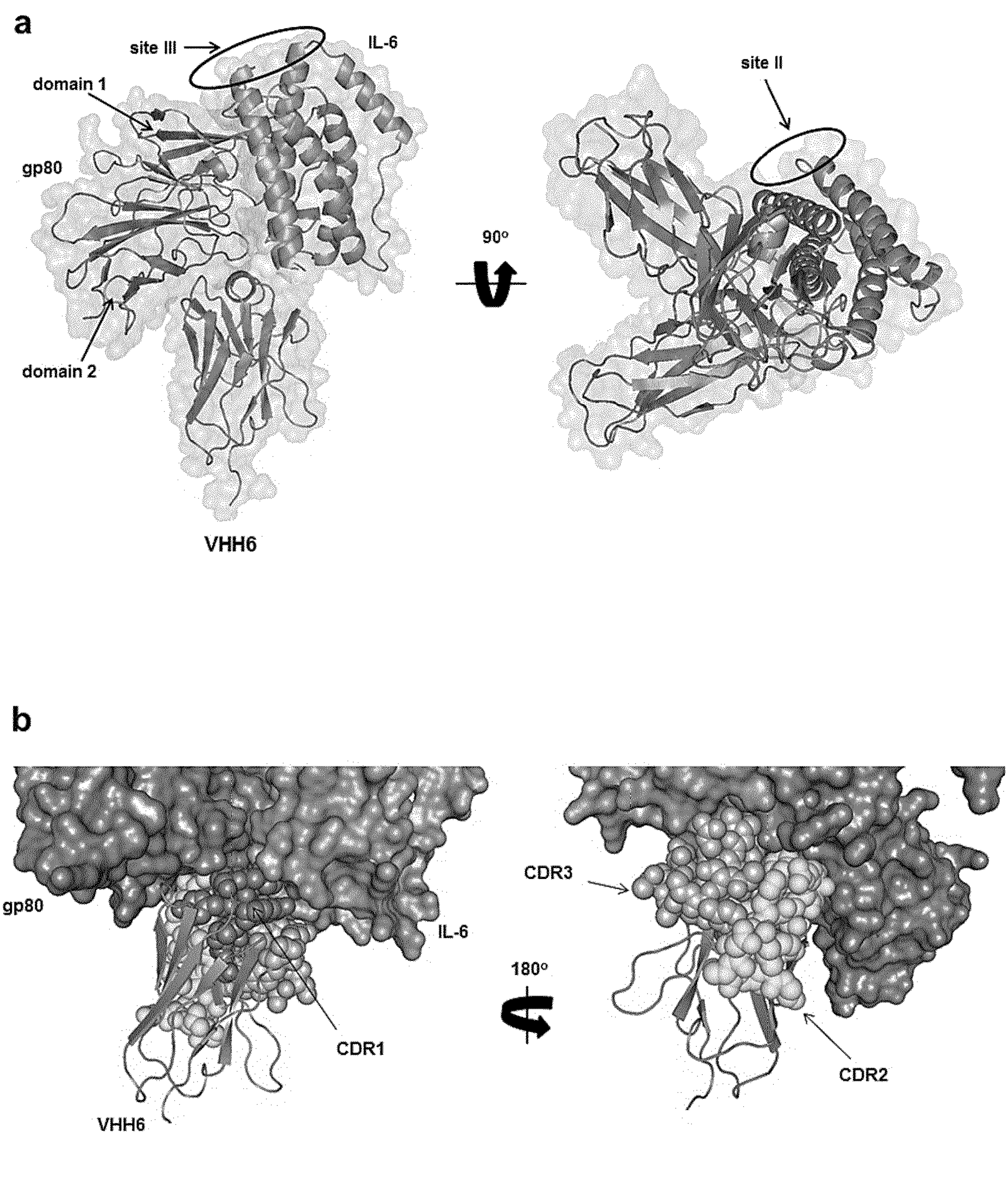
a
site III
IL-6
domain 1
gp80
site II
90°
domain 2
VHH6
b
gp80
IL-6
CDR3
180°
CDR1
CDR2
VHH6

c

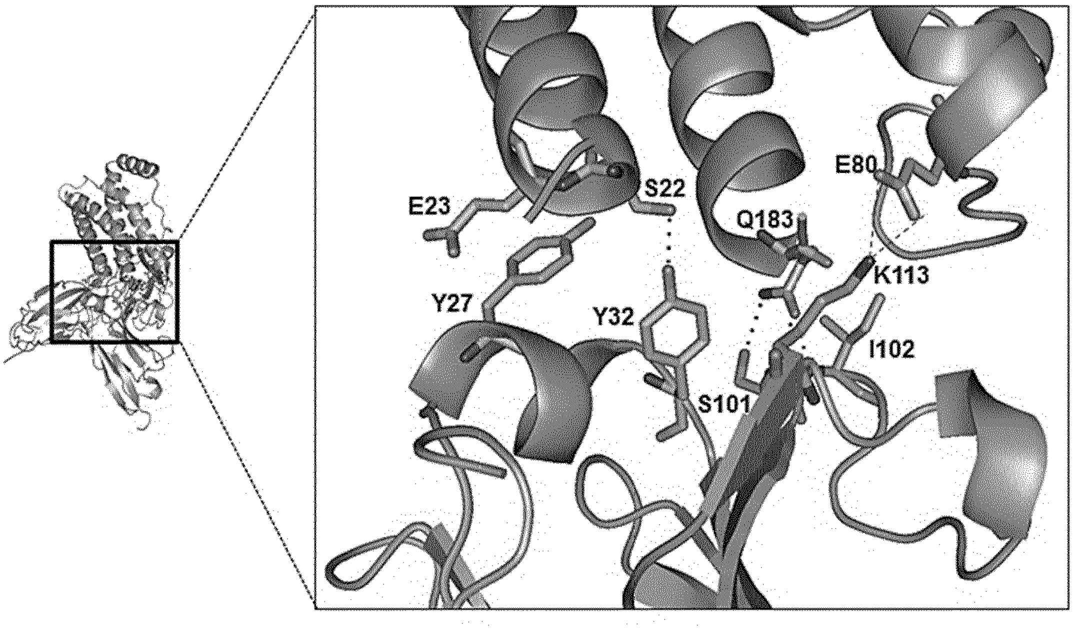
E23
S22
E80
Q183
K113
Y27
Y32
I102
S101

d

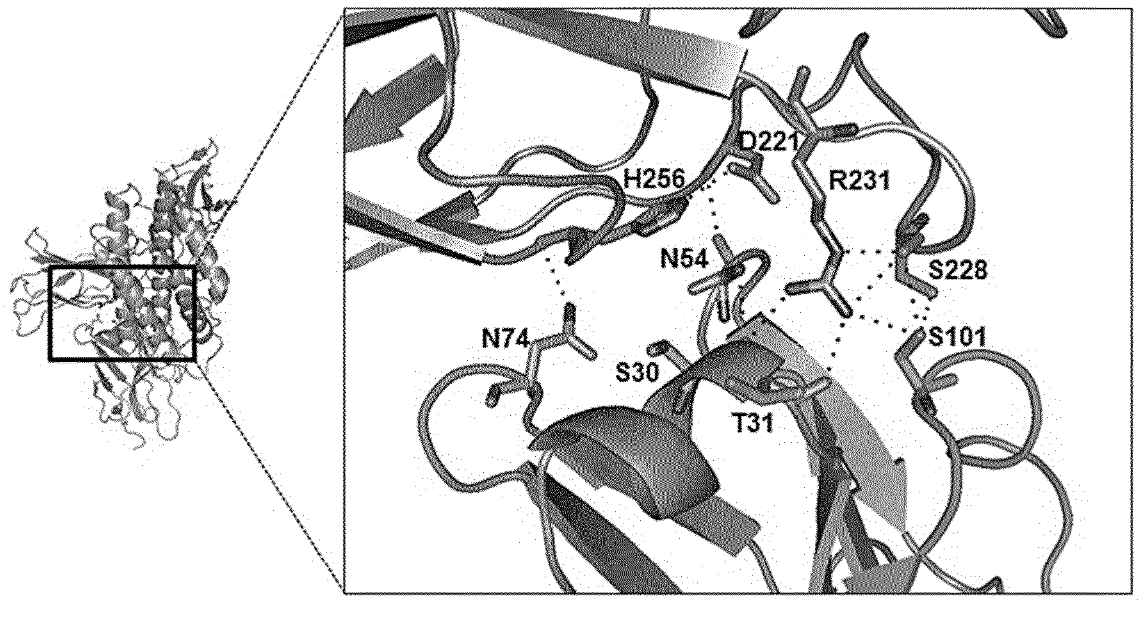
D221
H256
R231
N54
S228
N74
S101
S30
T31

**Figure 9**

**a**

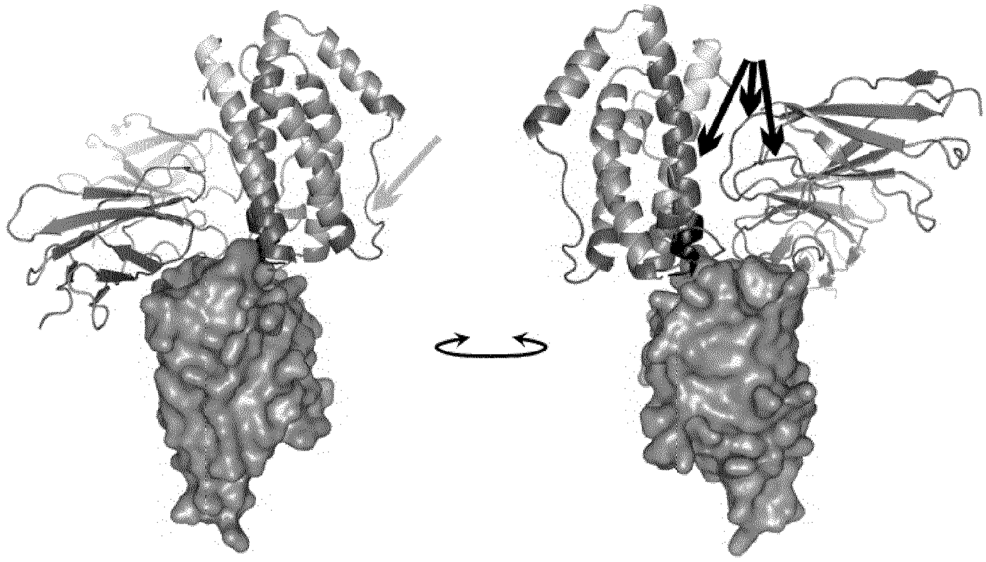

**b**

**IL-6**

1 **G**SAPVPPGED SKDVAAPHRQ PLT**SSERIDK** **Q**IRYILDGIS ALRKETCNKS
51 NMCESSKEAL AENNLNLPKM AE**KDGCFQSG** **FNE**ETCLVKI ITGLLEFEVY
101 LEYLQNRFES SEEQARAVQM STKVLIQF**LQ** **KKAKNLDAIT** **TPDPTTN**ASL
151 LTKLQAQNQW LQD**MTTHLIL** **RSFKEFLQSS** **LRALRQM**

**gp80**

17 GLAP RRCPAQEVAR GAGAGDVPPE EPQLSCFRKS PLSNVVCEWG
61 PRSTPSLTTK AVLLVRKFQN SPAEDFQEPC QYSQESQKFS CQLAV**PEGDS**
111 **SFY**IVSMCVA SSVGSKFSKT QTFQGAGILQ PDPPANITVT AVARNPRW**LS**
161 **VTWQD**PHSWN SSFYRLRFEL RYRAERSKTF TTW**MVKDLQH** **HAVIHD**AWSG
211 LRHVVQL**RAQ** **EEFGQGEWSE** WSPEAMGTPW TESRSPP

Figure 10

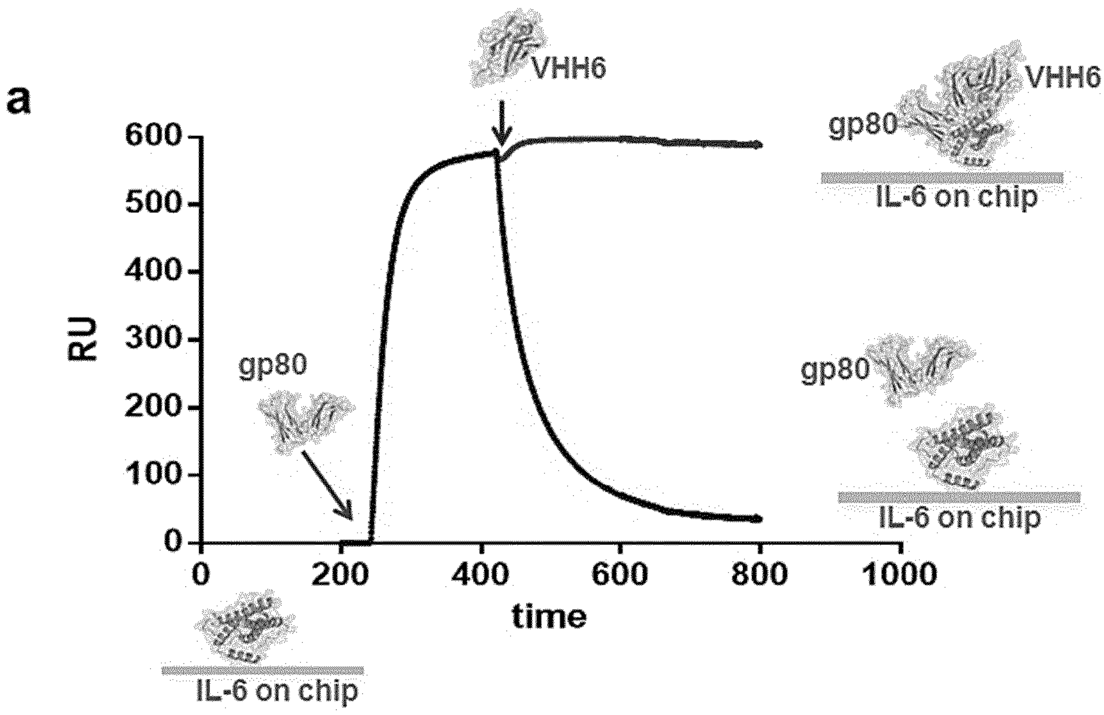
a
VHH6
gp80
IL-6 on chip
600
500
400
300
200
100
0
RU
0
200
400
600
800
1000
time
gp80
IL-6 on chip
IL-6 on chip

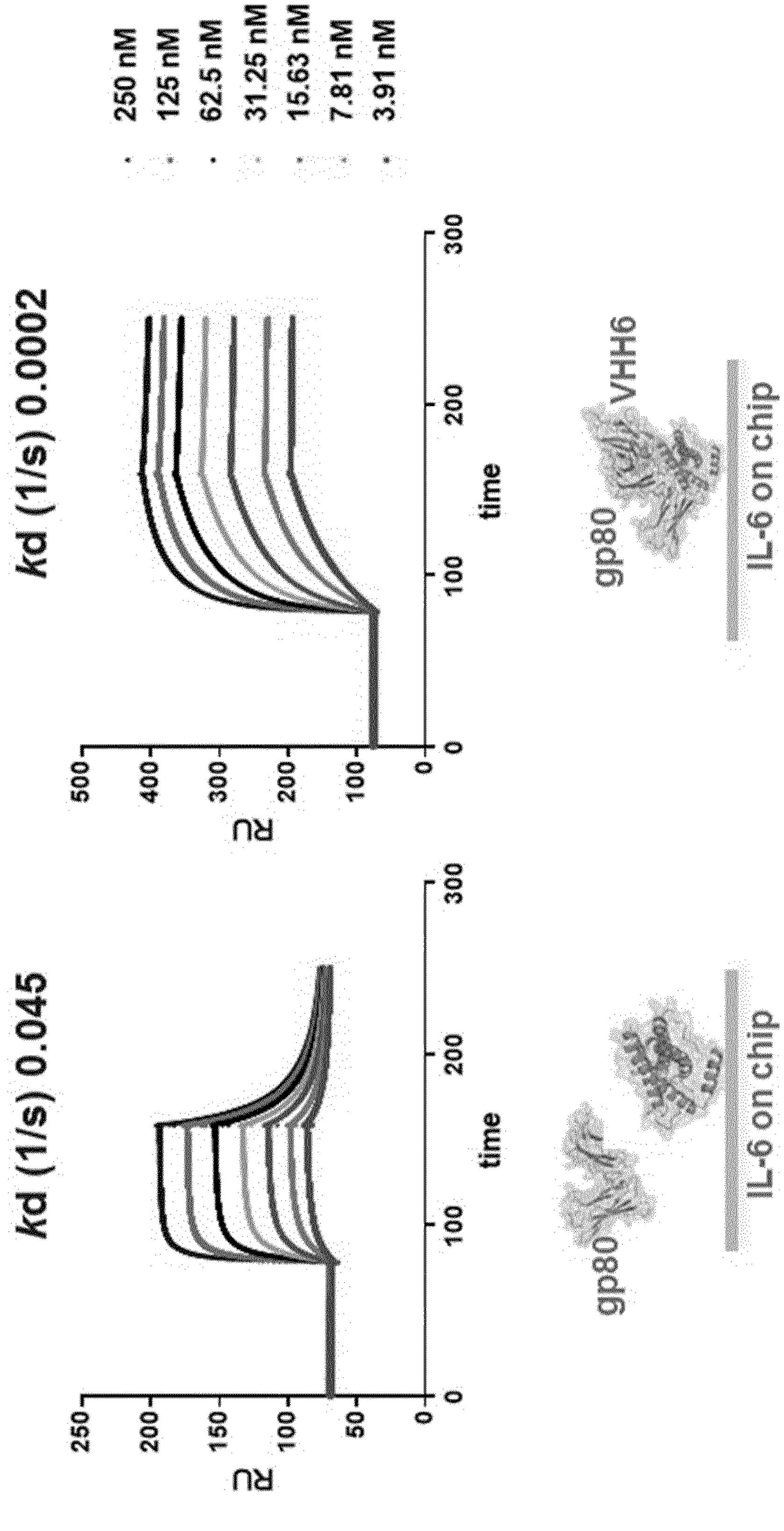
kd (1/s) 0.045
RU
250
200
150
100
50
0
time
0
100
200
300
gp80
IL-6 on chip
kd (1/s) 0.0002
RU
500
400
300
200
100
0
time
0
100
200
300
gp80
VHH6
IL-6 on chip
250 nM
125 nM
62.5 nM
31.25 nM
15.63 nM
7.81 nM
3.91 nM

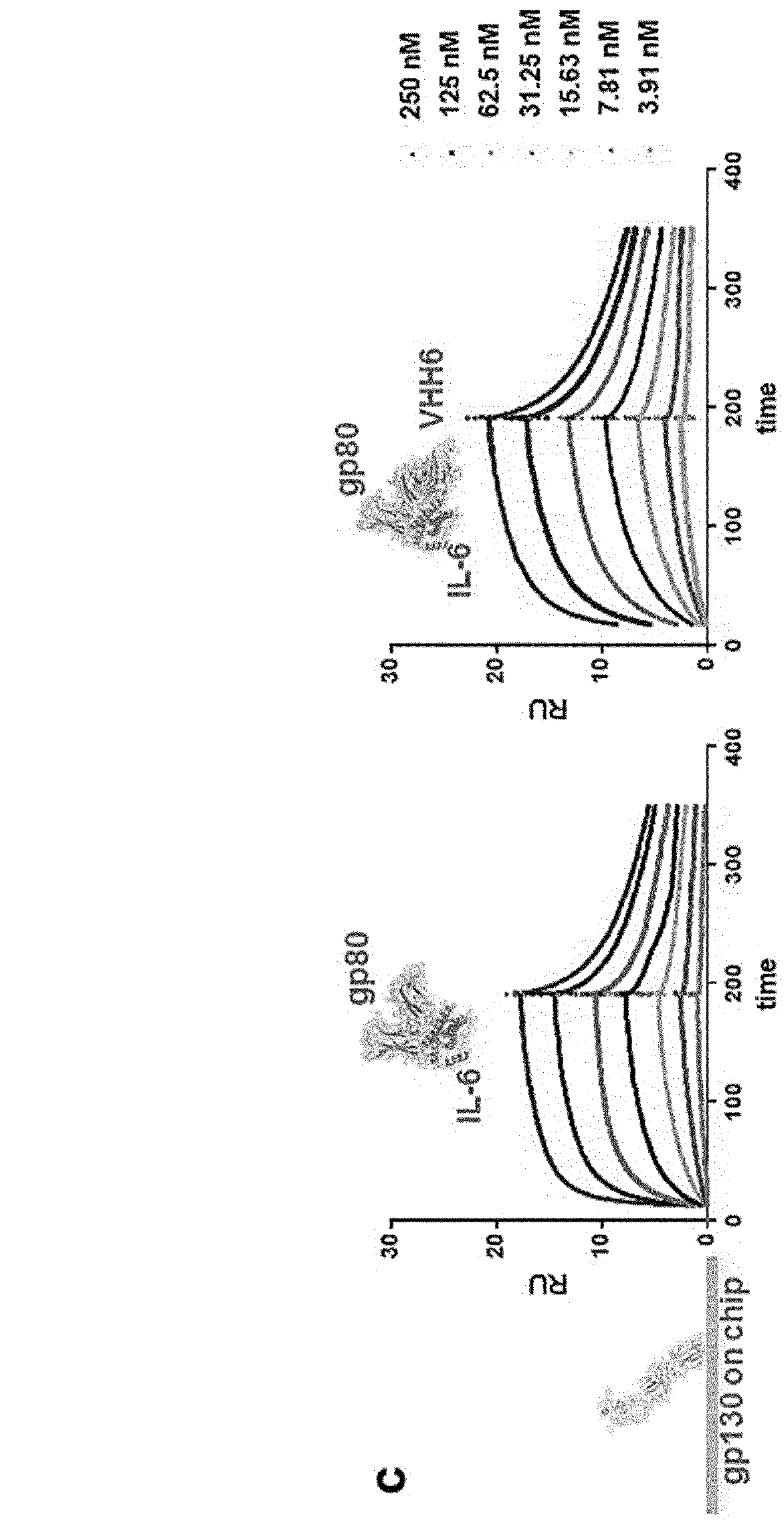
C
gp130 on chip
gp80
IL-6
RU
time
gp80
IL-6
VHH6
RU
time
250 nM
125 nM
62.5 nM
31.25 nM
15.63 nM
7.81 nM
3.91 nM

Figure 11

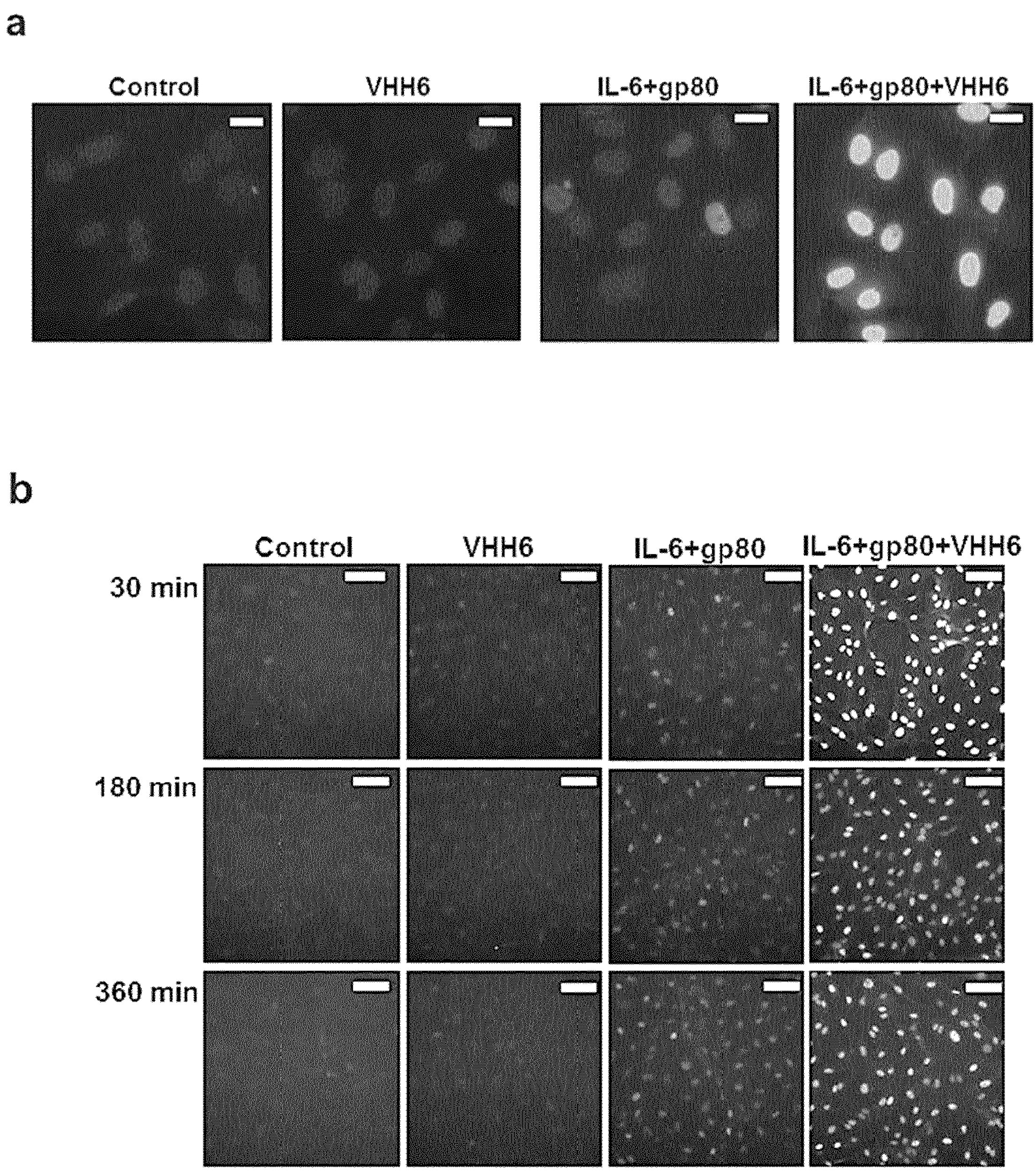
a
Control
VHH6
IL-6+gp80
IL-6+gp80+VHH6
b
Control
VHH6
IL-6+gp80
IL-6+gp80+VHH6
30 min
180 min
360 min

C

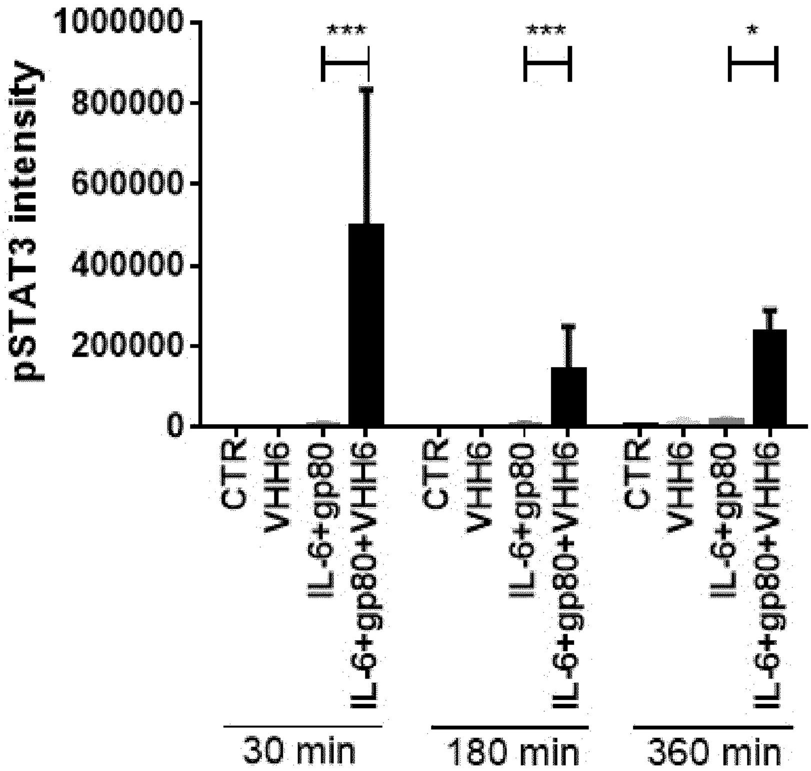
1000000
800000
600000
400000
200000
0
pSTAT3 intensity
***
***
*
CTR
VHH6
IL-6+gp80
IL-6+gp80+VHH6
30 min
180 min
360 min

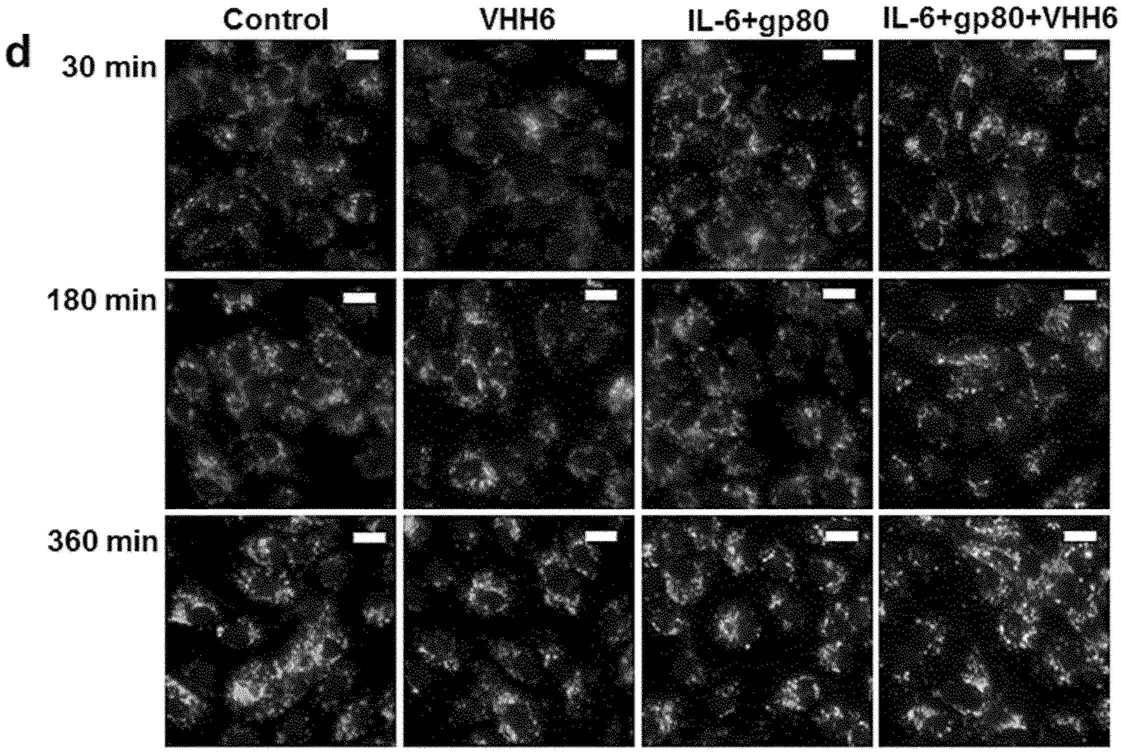
d
Control
VHH6
IL-6+gp80
IL-6+gp80+VHH6
30 min
180 min
360 min

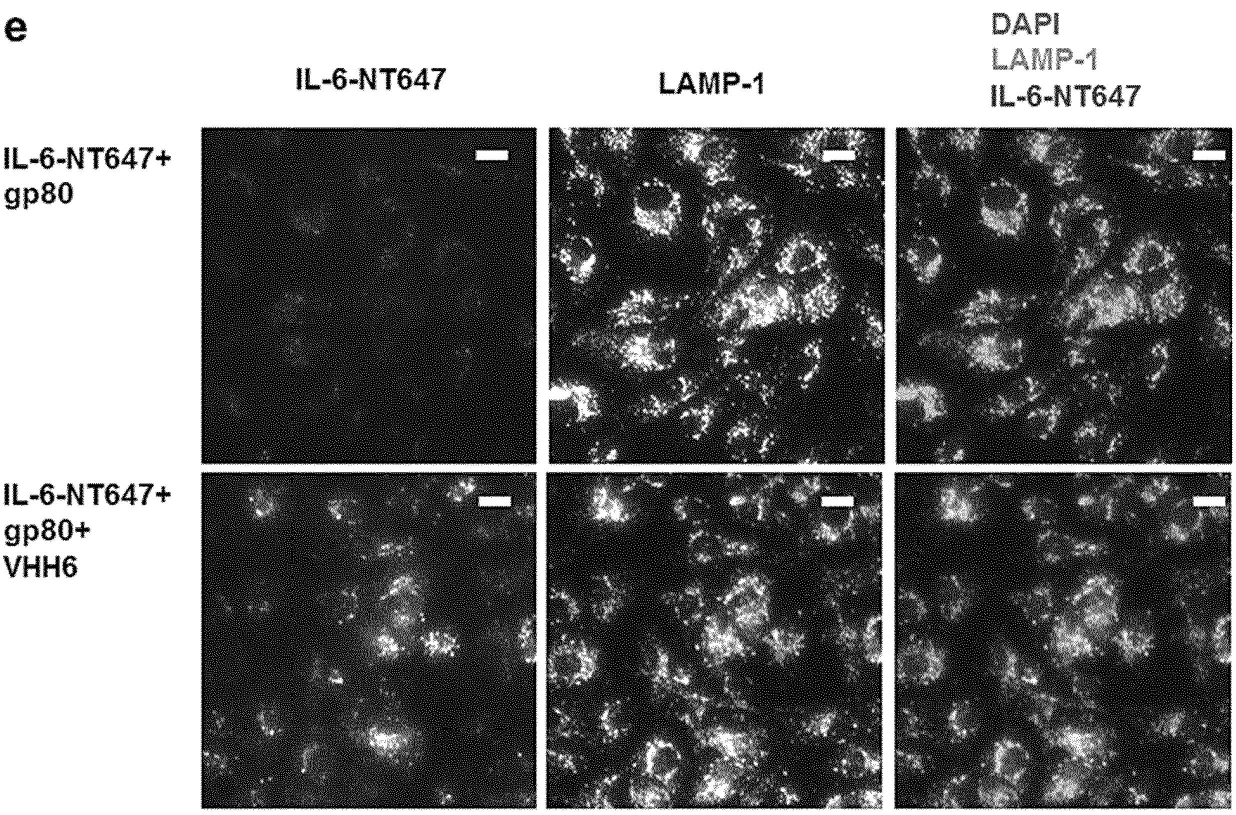
e
IL-6-NT647
LAMP-1
DAPI
LAMP-1
IL-6-NT647
IL-6-NT647+
gp80
IL-6-NT647+
gp80+
VHH6

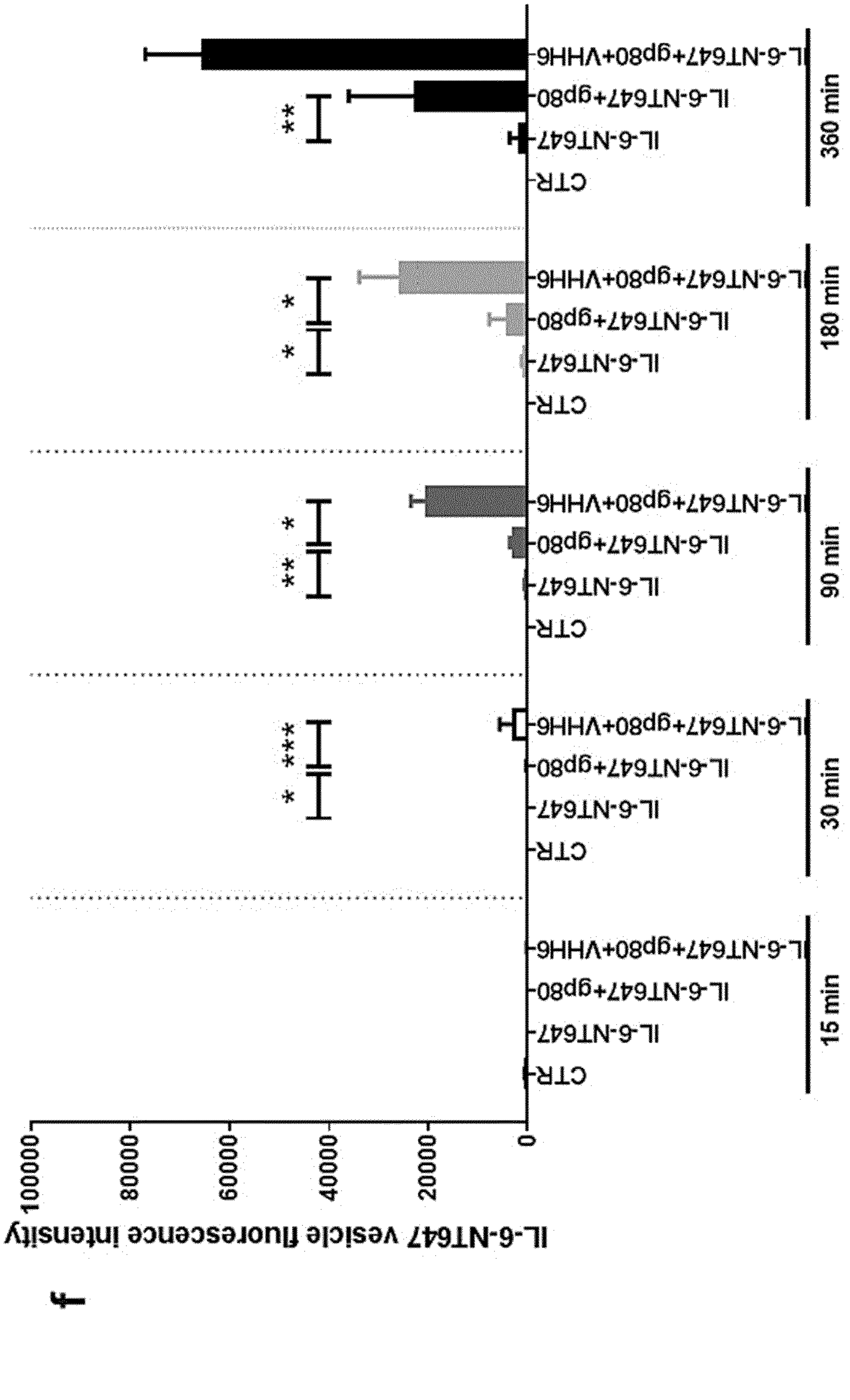
f
IL-6-NT647 vesicle fluorescence intensity
0
20000
40000
60000
80000
100000
CTR
IL-6-NT647
IL-6-NT647+gp80
IL-6-NT647+gp80+VHH6
15 min
30 min
90 min
180 min
360 min
*
**
***

Figure 12

a

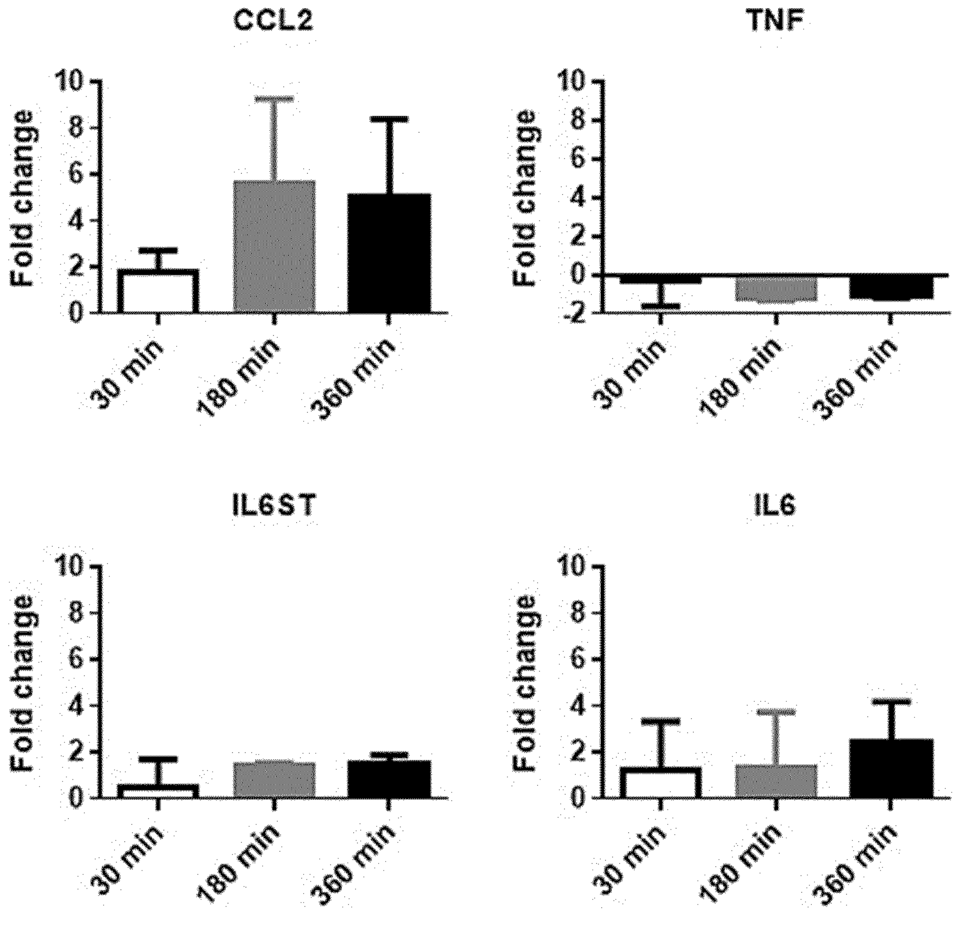
CCL2
TNF
IL6ST
IL6
Fold change
10
8
6
4
2
0
-2
30 min
180 min
360 min

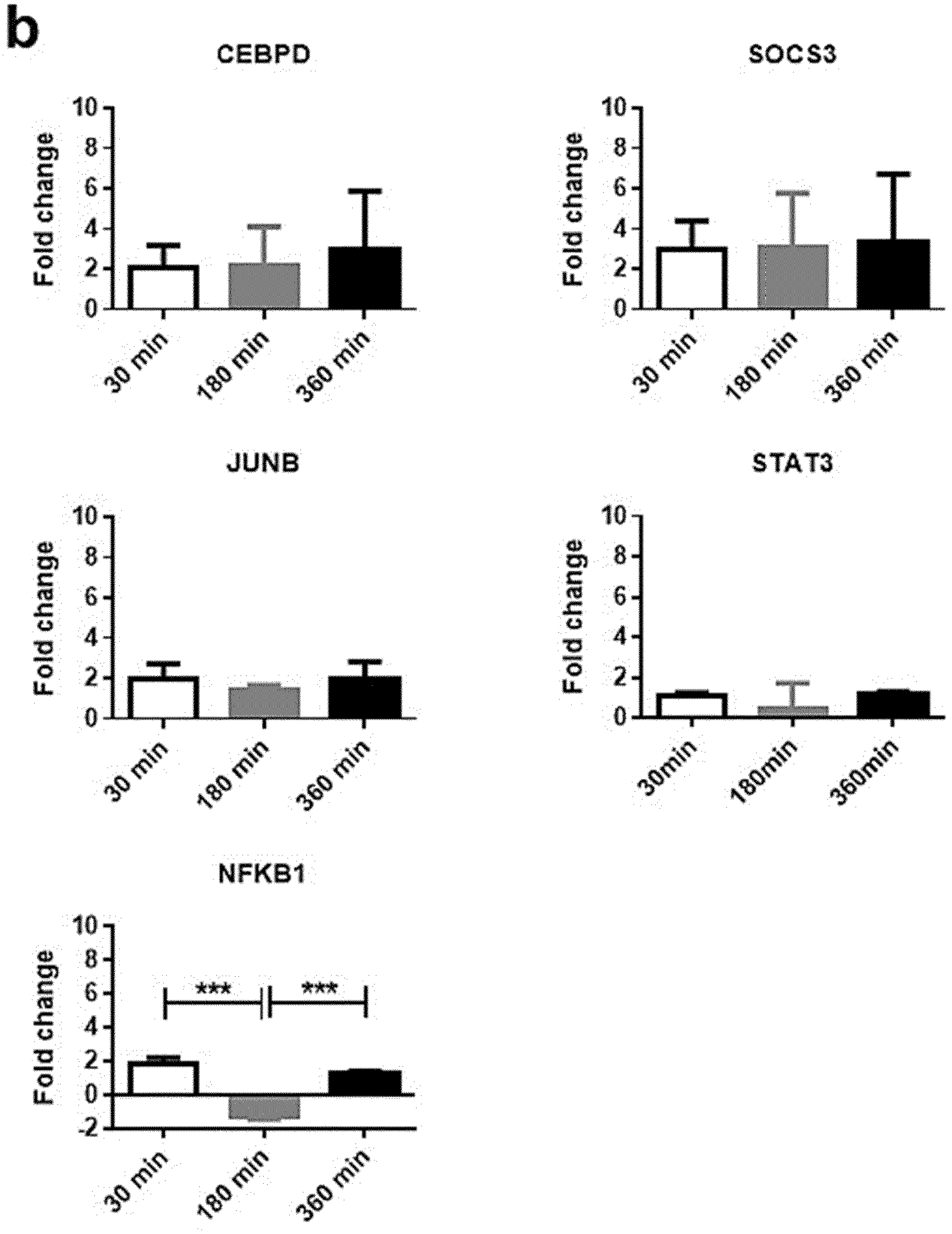
b
CEBPD
SOCS3
JUNB
STAT3
NFKB1
Fold change
10
8
6
4
2
0
-2
***
***
30 min
180 min
360 min
30min
180min
360min

C

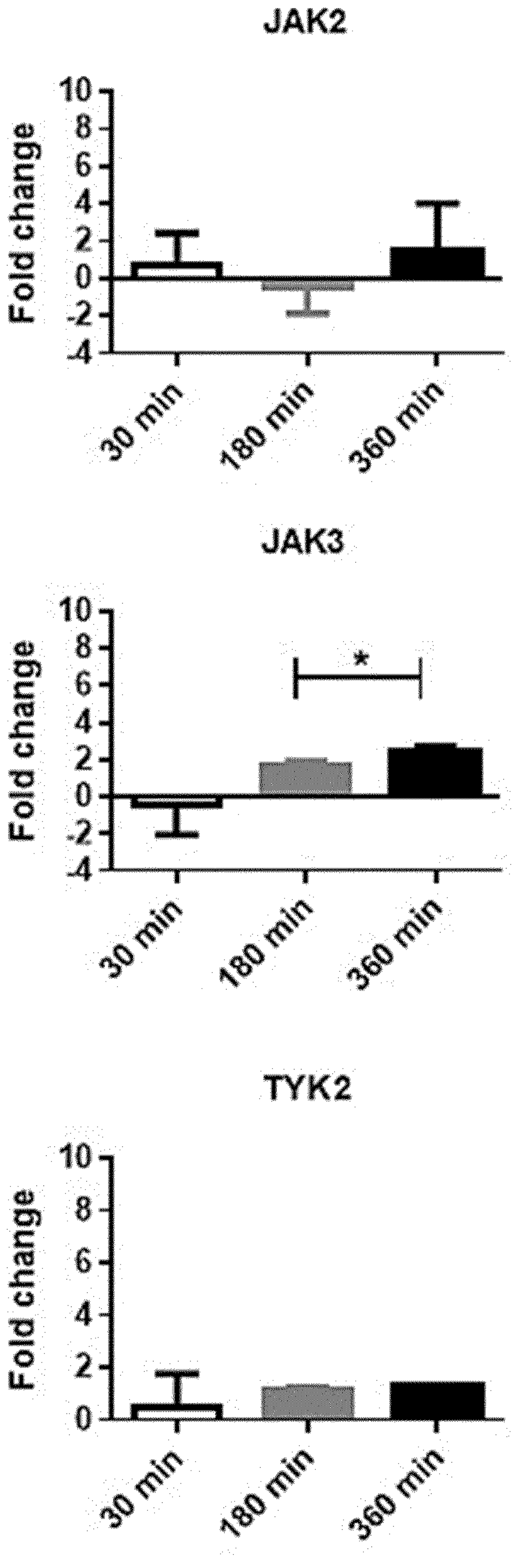
JAK2
Fold change
10
8
6
4
2
0
-2
-4
30 min
180 min
360 min
JAK3
Fold change
*
10
8
6
4
2
0
-2
-4
30 min
180 min
360 min
TYK2
Fold change
10
8
6
4
2
0
30 min
180 min
360 min

Figure 13

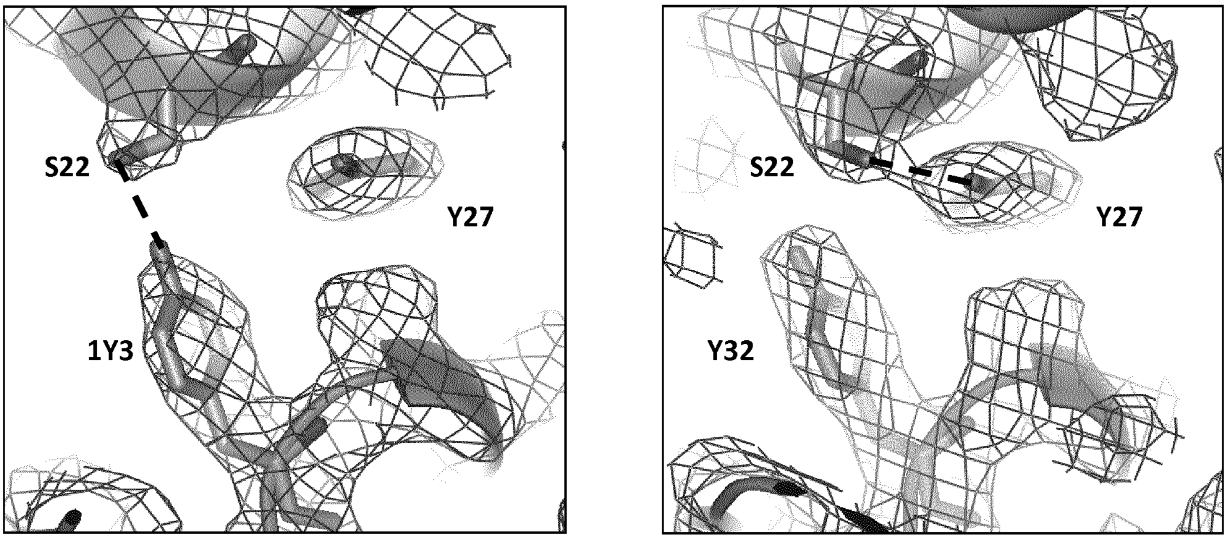


Figure 14

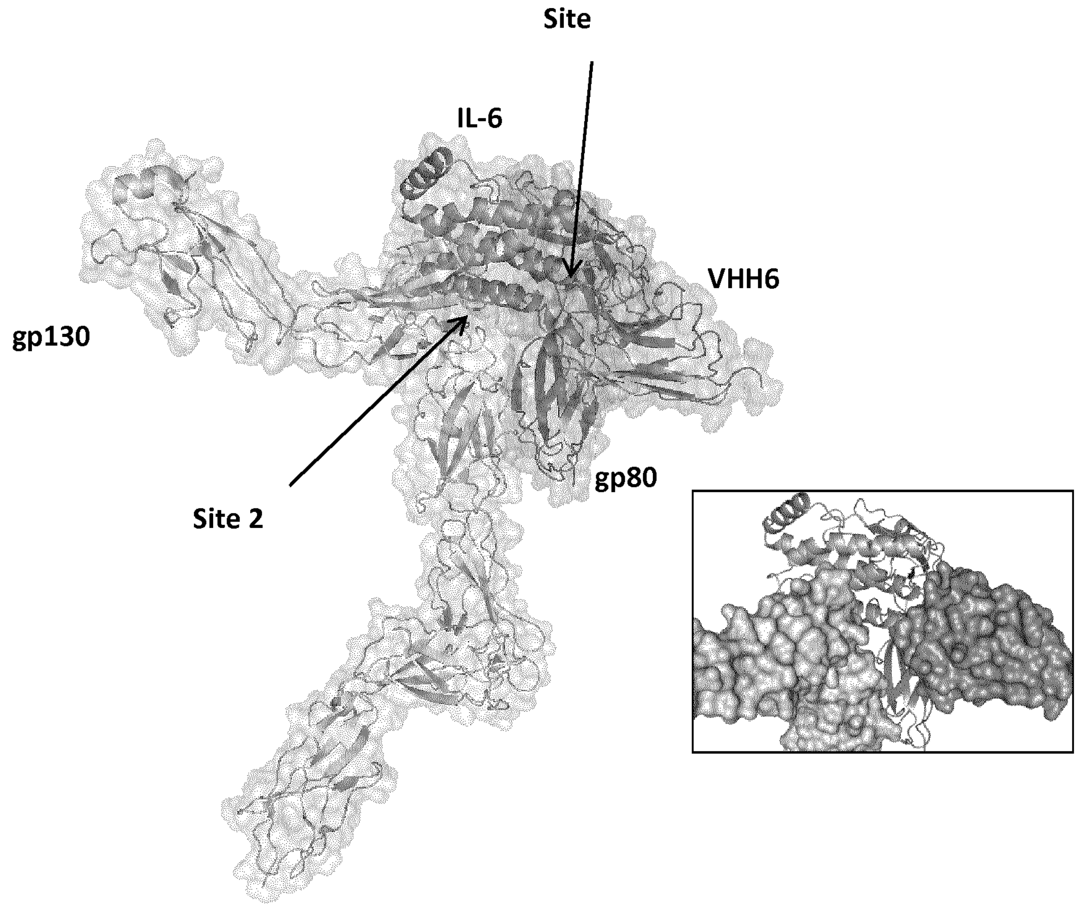

Figure 15

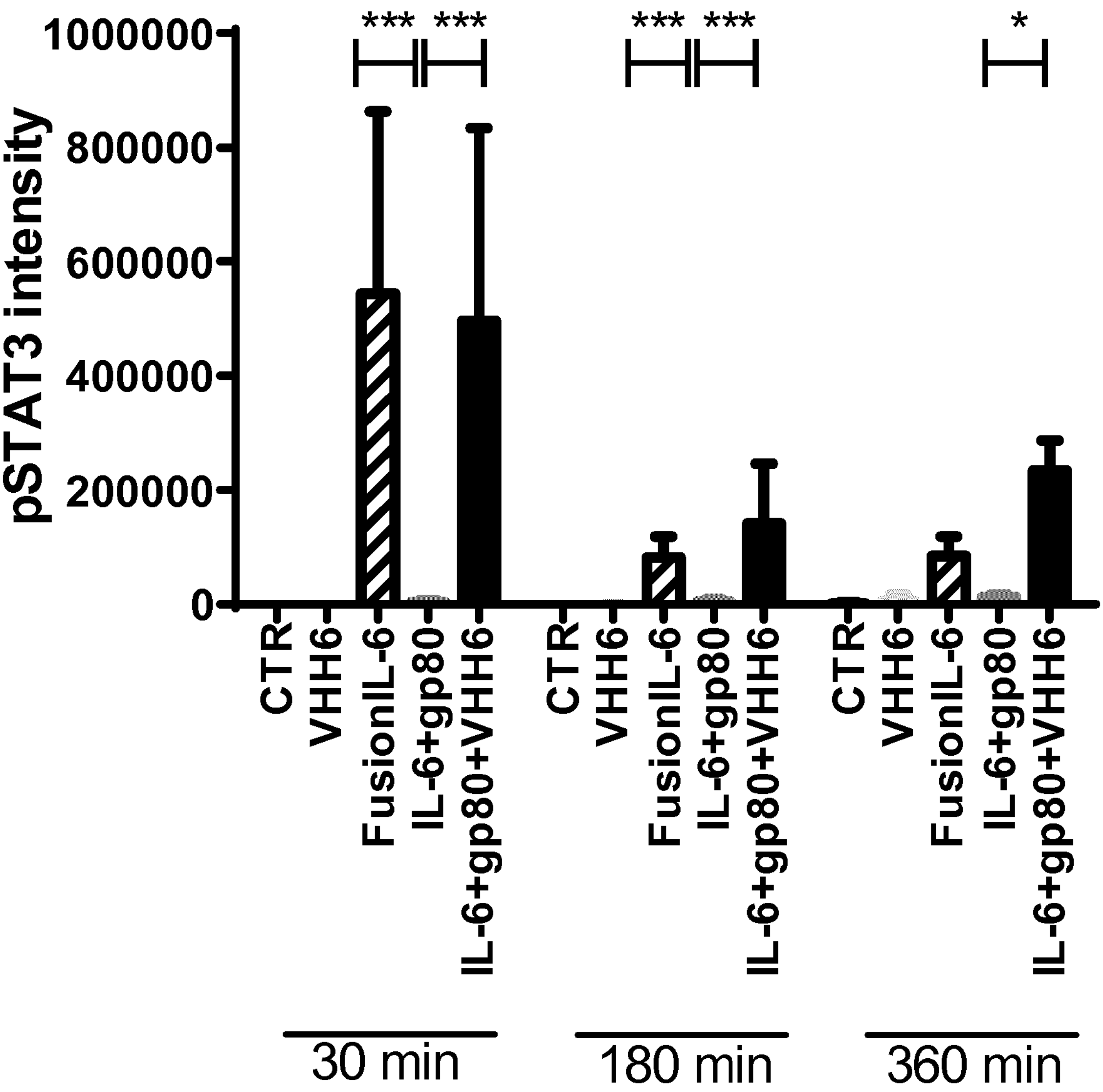
pSTAT3 intensity
1000000
800000
600000
400000
200000
0
*** ***
*** ***
*
CTR
VHH6
FusionIL-6
IL-6+gp80
IL-6+gp80+VHH6
30 min
180 min
360 min

Figure 16

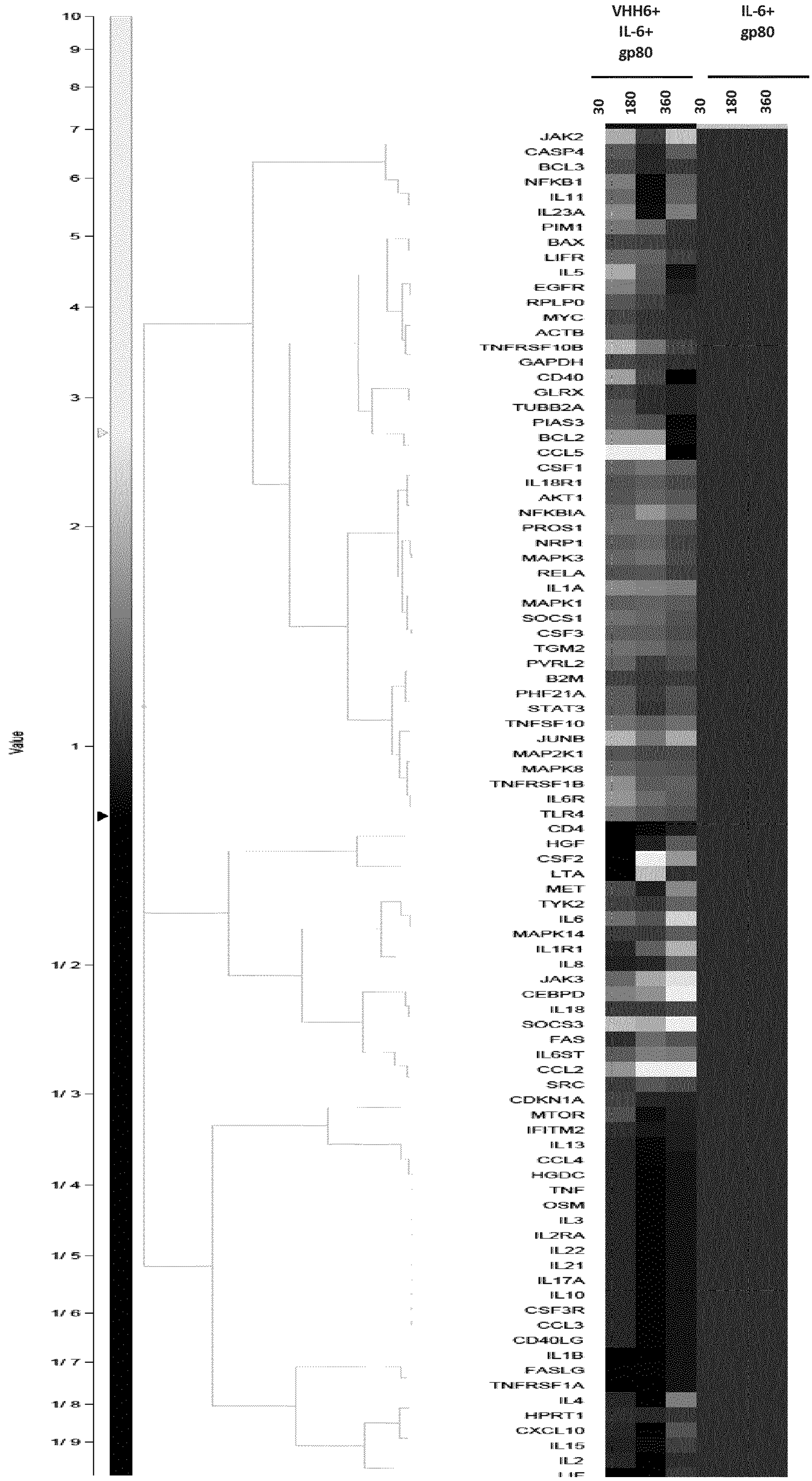
VHH6+
IL-6+
gp80
IL-6+
gp80
30
180
360
30
180
360
Value
10
9
8
7
6
5
4
3
2
1
1/2
1/3
1/4
1/5
1/6
1/7
1/8
1/9
JAK2
CASP4
BCL3
NFKB1
IL11
IL23A
PIM1
BAX
LIFR
IL5
EGFR
RPLP0
MYC
ACTB
TNFRSF10B
GAPDH
CD40
GLRX
TUBB2A
PIAS3
BCL2
CCL5
CSF1
IL18R1
AKT1
NFKBIA
PROS1
NRP1
MAPK3
RELA
IL1A
MAPK1
SOCS1
CSF3
TGM2
PVRL2
B2M
PHF21A
STAT3
TNFSF10
JUNB
MAP2K1
MAPK8
TNFRSF1B
IL6R
TLR4
CD4
HGF
CSF2
LTA
MET
TYK2
IL6
MAPK14
IL1R1
IL8
JAK3
CEBPD
IL18
SOCS3
FAS
IL6ST
CCL2
SRC
CDKN1A
MTOR
IFITM2
IL13
CCL4
HGDC
TNF
OSM
IL3
IL2RA
IL22
IL21
IL17A
IL10
CSF3R
CCL3
CD40LG
IL1B
FASLG
TNFRSF1A
IL4
HPRT1
CXCL10
IL15
IL2

Figure 17

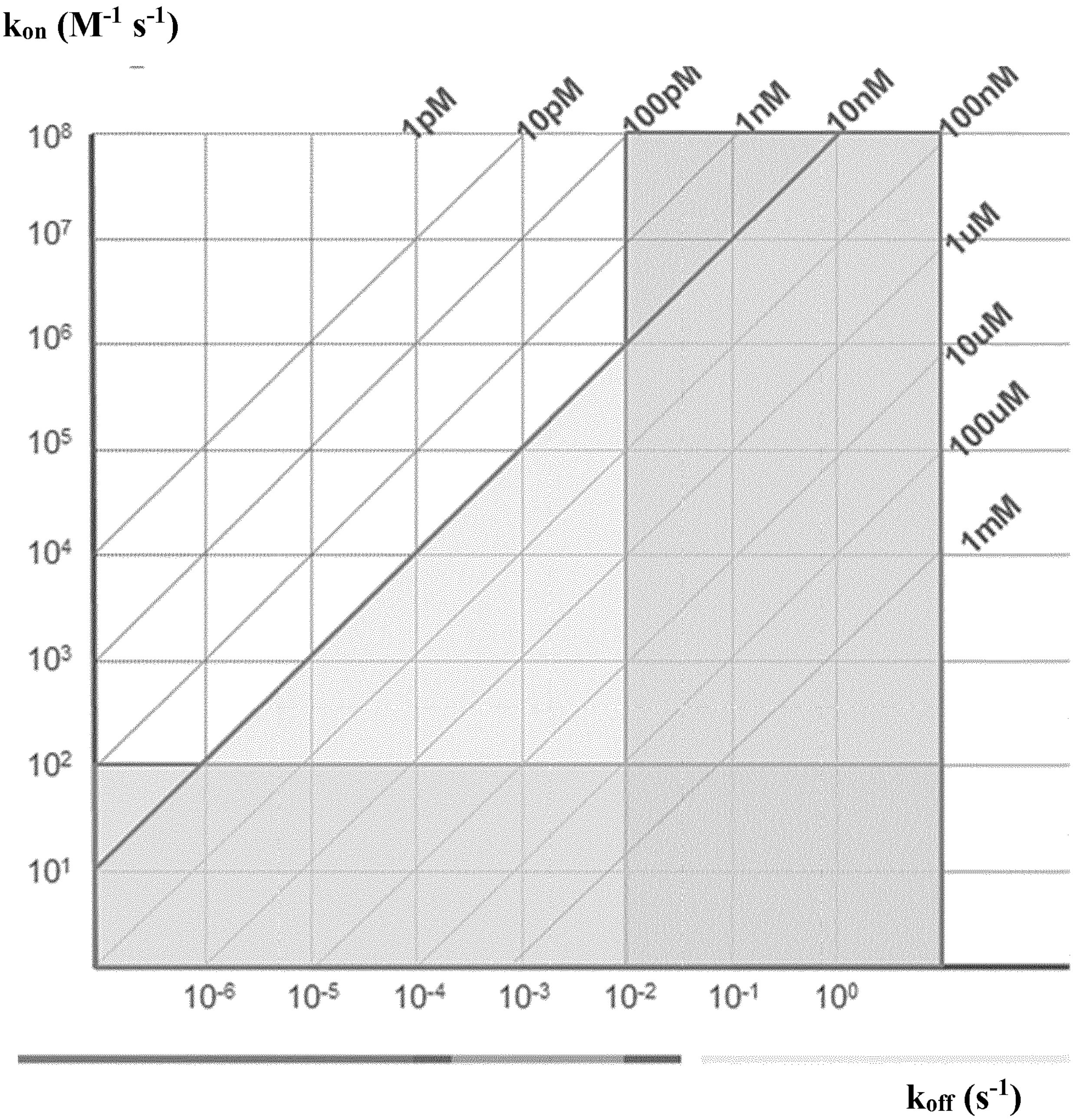
kon (M-1 s-1)
1pM
10pM
100pM
1nM
10nM
100nM
1uM
10uM
100uM
1mM
10^8
10^7
10^6
10^5
10^4
10^3
10^2
10^1
10^-6
10^-5
10^-4
10^-3
10^-2
10^-1
10^0
koff (s-1)

```
            1   5    10   15   20   25   30   35   40   45   50   55   60   65   70   75   80   85   90   95   100  105  110  115  120  125
VHH6        DVQFVESGGGSVHAGGSLRLNCATSGYIYSTYCMGWFRQAPGKEREGVAHIYTNSGRTYYADSVKGRFTISQDNAKNTVYLQMNSLKPEDTAIYYCAARPSIRCASFSATEYKDWGQGTQVTVSS
            |  |      |  ||       |  |  ||| | | |  |       |||| || |||||| |            |      |       ||     |    |                 ||||      |
IGHV3-74    EVQLVESGGGLVQPGGSLRLSCAASGFTFSSYWMHWVRQAPGKGLVWVSRINSDGSSTSYADSVKGRFTISRDNAKNTLYLQMNSLRAEDTAVYYCAR------------YFDYWGQGTLVTVSS
               |                    |            |       ||||  |             |      |                |
VHH6gH1     EVQFVESGGGLVQPGGSLRLSCATSGYIYSTYCMGWFRQAPGKEREGVAHIYTNSGRTYYADSVKGRFTISQDNAKNTVYLQMNSLRAEDTAVYYCAARPSIRCASFSATEYKDWGQGTLVTVSS
VHH6gH2     EVQFVESGGGLVQPGGSLRLSCATSGYIYSTYSMGWFRQAPGKEREGVAHIYTNSGRTYYADSVKGRFTISQDNAKNTVYLQMNSLRAEDTAVYYCAARPSIRSASFSATEYKDWGQGTLVTVSS
```

**Figure 18**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013104804 A
- WO 2004033485 A
- WO 2016012363 A
- WO 9202551 A
- WO 2004051268 A
- WO 2004106377 A
- WO 9002809 A
- WO 9110737 A
- WO 9201047 A
- WO 9218619 A
- WO 9311236 A
- WO 9515982 A
- WO 9520401 A
- US 5698426 A
- US 5223409 A
- US 5403484 A
- US 5580717 A
- US 5427908 A
- US 5750753 A
- US 5821047 A
- US 5571698 A
- US 5516637 A
- US 5780225 A
- US 5658727 A
- US 5733743 A
- US 5969108 A
- EP 0546073 A
- US 5545806 A
- US 5569825 A
- US 5625126 A
- US 5633425 A
- US 5661016 A
- US 5770429 A
- EP 0438474 A
- EP 0463151 A
- WO 2005003169 A
- WO 2005003170 A
- WO 2005003171 A
- WO 2009040562 A
- WO 9109967 A
- WO 9962534 A
- WO 2015181282 A
- WO 2016009030 A
- WO 2016009029 A
- GB 1617924 A

### Non-patent literature cited in the description

- **MAZOR et al.** *mAbs,* vol. 7, 461-469
- **WARD et al.** *The Journal of biological chemistry,* 1996, vol. 271, 20138-20144
- **BOULANGER et al.** *Science,* 2003, vol. 300, 2101-2104
- **MULLBERG et al.** *European journal of immunology,* 1993, vol. 23, 473-480
- **ROSE-JOHN et al.** *Journal of leukocyte biology,* 2006, vol. 80, 227-236
- **ROSE-JOHN et al.** *International journal of biological sciences,* 2012, vol. 8, 1237-1247
- **GALUN, E ; ROSE-JOHN, S.** *Methods in molecular biology,* 2013, vol. 982, 59-77
- **MIHARA et al.** *Clin Sci (Lond),* 2012, vol. 122, 143-159
- **HUNTER, C.A. ; JONES, S.A.** *Nature immunology,* 2015, vol. 16, 448-457
- **ROSE-JOHN et al.** *Expert opinion on therapeutic targets,* 2007, vol. 11, 613-624
- **CALABRESE, L.H. ; ROSE-JOHN, S.** Nature reviews. *Rheumatology,* 2014, vol. 10, 720-727
- **SHAW, S. et al.** Discovery and characterization of olokizumab: a humanized antibody targeting interleukin-6 and neutralizing gp130-signaling. *mAbs,* 2014, vol. 6, 774-782
- **ATREYA, R. et al.** *Nature medicine,* 2000, vol. 6, 583-588
- **NISHIMOTO, N. et al.** *Blood,* 2005, vol. 106, 2627-2632
- **MILAGRE, C.S. et al.** *Cancer research,* 2015, vol. 75, 1255-1264
- **DAMANSKA et al.** *Proc Nat Acad Sci USA,* 2011, 1314-1319
- **RUBINSTEIN et al.** *Int J of Cancer,* 1996, vol. 124 (1), 46-54
- **L. HANCOCK.** PhD Thesis. University of London, 2010
- **NOOREN ; THORNTON.** *The EMBO Journal,* 2003, vol. 22, 3486-3492
- **PERKINS et al.** *Structure,* 2010, vol. 18, 1233-1243
- **VONIGRADOVA ; QIN.** *Top Curr Chem,* 2012, vol. 326, 35-45

- Handbook of Experimental Immunology. Blackwell Scientific Publishers, 1986, vol. 4
- **KOHLER ; MILSTEIN.** *Nature,* 1975, vol. 256, 495-497
- **KOZBOR et al.** *Immunology Today,* 1983, vol. 4, 72
- **COLE et al.** Monoclonal Antibodies and Cancer Therapy. Alan R Liss, Inc, 1985, 77-96
- **BABCOOK, J et al.** *Proc. Natl. Acad. Sci. USA,* 1996, vol. 93 (15), 7843-78481
- **BRINKMAN et al.** *J. Immunol. Methods,* 1995, vol. 182, 41-50
- **AMES et al.** *J. Immunol. Methods,* 1995, vol. 184, 177-186
- **KETTLEBOROUGH et al.** *Eur. J. Immunol.,* 1994, vol. 24, 952-958
- **PERSIC et al.** *Gene,* 1997, vol. 187, 9-18
- **BURTON et al.** *Advances in Immunology,* 1994, vol. 57, 191-280
- **HOLLIGER ; HUDSON.** *Nature Biotech.,* 2005, vol. 23 (9), 1126-1136
- **ADAIR ; LAWSON.** *Drug Design Reviews - Online,* 2005, vol. 2 (3), 209-217
- **VERMA et al.** *Journal of Immunological Methods,* 1998, vol. 216, 165-181
- **VAUGHAN et al.** *Nature Biotechnology,* 1998, vol. 16, 535-539
- **KASHMIRI et al.** *Methods,* 2005, vol. 36, 25-34
- **RETTER et al.** *Nucl. Acids Res.,* 2005, vol. 33 (1), D671-D674
- **REICHMANN et al.** *Nature,* 1998, vol. 332, 323-324
- **HARRIS, RJ.** *Journal of Chromatography,* 1995, vol. 705, 129-134
- **WALKER.** The Proteomics Protocols Handbook. Humana Press, 2005, 571-607
- **DEVEREUX et al.** *Nucleic Acids Research,* 1984, vol. 12, 387-395
- **ALTSCHUL S.F.** *J Mol Evol,* 1993, vol. 36, 290-300
- **ALTSCHUL, S, F et al.** *J Mol Biol,* 1990, vol. 215, 403-10
- **HENIKOFF ; HENIKOFF.** *Proc. Natl. Acad. Sci. USA,* 1992, vol. 89, 10915-10919
- **KARLIN ; ALTSCHUL.** *Proc. Natl. Acad. Sci. USA,* 1993, vol. 90, 5873-5787
- Current Protocols in Molecular Biology. Wiley Interscience, 1999
- Maniatis Manual. Cold Spring Harbor Publishing
- **JUNGHANS et al.** *Cancer Res,* 1990, vol. 50, 1495-1502
- **HARLOW ; LANE.** Antibodies. Cold Spring Harbor Press
- **REINEKE.** *Methods Mol Biol,* 2004, vol. 248, 443-63
- **TOMER.** *Protein Science,* 2000, vol. 9, 487-496
- **GALUN.** *Methods in Molecular Biology,* 2013, vol. 982, 59-77
- **WUESTEFELD et al.** *The Journal of Biological Chemistry,* 2003, vol. 278, 11281-11288
- **BEST et al.** *Experimental and Molecular Pathology,* 2010, vol. 88, 7-14
- **FISCHER.** *Nature Biotechnology,* 1997, vol. 15, 142-145
- **GABAY et al.** *Arthritis Res Ther,* 2006, vol. 8 (2), S3
- **FISCHER.** *Eye,* 2017, vol. 31, 173-178
- **TADOKORO et al.** *PNAS,* 2014, vol. 111 (35), E3641-E3649
- **FISCHER, M. et al.** *I. Nature biotechnology,* 1997, vol. 15, 142-145
- **TICKLE, S. et al.** *JALA,* 2009, vol. 14, 303-307
- **LIGHTWOOD, D. et al.** *Journal of molecular biology,* 2013, vol. 425, 577-593
- A study of camelid antibodies reveal structural features of a cytokine. **HANCOCK, L.M.** PhD Thesis University of London. 2011
- **JERABEK-WILLEMSEN, M. ; WIENKEN et al.** *Assay and drug development technologies,* 2011, vol. 9, 342-353
- **ABDICHE et al.** *Journal of immunological methods,* 2012, vol. 382, 101-116
- **KABSCH, W.** *Xds. Acta crystallographica. Section D, Biological crystallography,* 2010, vol. 66, 125-132
- **MCCOY, A.J. et al.** *Journal of applied crystallography,* 2007, vol. 40, 658-674
- **BOULANGER M.J. et al.** *Science,* 2003, vol. 300, 2101-2104
- **BRUNGER, A.T. et al.** Crystallography & NMR system: A new software suite for macromolecular structure determination. *Acta crystallographica. Section D, Biological crystallography,* 1998, vol. 54, 905-921
- **BRUNGER, A.T.** Version 1.2 of the Crystallography and NMR system. *Nature protocols,* 2007, vol. 2, 2728-2733
- **EMSLEY, P. ; COWTAN, K.** Coot: model-building tools for molecular graphics. *Acta crystallographica. Section D, Biological crystallography,* 2004, vol. 60, 2126-2132
- **CHEN, V.B. et al.** MolProbity: all-atom structure validation for macromolecular crystallography. *Acta crystallographica. Section D, Biological crystallography,* 2010, vol. 66, 12-21
- **KRISSINEL, E ; HENRICK, K.** Inference of macromolecular assemblies from crystalline state. *Journal of molecular biology,* 2007, vol. 372, 774-797
- **DELANO W.** The PyMOL Molecular Graphics System. DeLano Scientific LLC, 2002

- **KONERMANN, L. ; PAN, J. ; LIU, Y.H.** Hydrogen exchange mass spectrometry for studying protein structure and dynamics. *Chemical Society reviews,* 2011, vol. 40, 1224-1234
- **BOULANGER, M.J. ; CHOW, D.C. ; BREVNOVA, E.E. ; GARCIA, K.C.** Hexameric structure and assembly of the interleukin-6/IL-6 alpha-receptor/gp130 complex. *Science,* 2003, vol. 300, 2101-2104
- **HEINRICH, P.C. et al.** Principles of interleukin (IL)-6-type cytokine signalling and its regulation. *The Biochemical journal,* 2003, vol. 374, 1-20
- **WANG, Y. ; VAN BOXEL-DEZAIRE, A.H. ; CHEON, H. ; YANG, J. ; STARK, G.R.** STAT3 activation in response to IL-6 is prolonged by the binding of IL-6 receptor to EGF receptor. *Proceedings of the National Academy of Sciences of the United States of America,* 2013, vol. 110, 16975-16980
- **ROMANO, M. et al.** Role of IL-6 and its soluble receptor in induction of chemokines and leukocyte recruitment. *Immunity,* 1997, vol. 6, 315-325
- **DISKIN, R. ; MARCOVECCHIO, P.M. ; BJORKMAN, P.J.** Structure of a clade C HIV-1 gp120 bound to CD4 and CD4-induced antibody reveals anti-CD4 polyreactivity. *Nature structural & molecular biology,* 2010, vol. 17, 608-613
- **IMPAGLIAZZO , A. et al.** A stable trimeric influenza hemagglutinin stem as a broadly protective immunogen. *Science,* 2015, vol. 349 (6254), 1301-1306
- **LEE, J.E. et al.** Structure of the Ebola virus glycoprotein bound to an antibody from a human survivor. *Nature,* 2008, vol. 454 (7201), 177-82
- **YU, H. ; PARDOLL, D. ; JOVE, R.** STATs in cancer inflammation and immunity: a leading role for STAT3. *Nature reviews. Cancer,* 2009, vol. 9, 798-809
- **KOCH, A.E. et al.** Enhanced production of monocyte chemoattractant protein-1 in rheumatoid arthritis. *The Journal of clinical investigation,* 1992, vol. 90, 772-779
- **HUEBER, W. et al.** Proteomic analysis of secreted proteins in early rheumatoid arthritis: anti-citrulline autoreactivity is associated with up regulation of proinflammatory cytokines. *Annals of the rheumatic diseases,* 2007, vol. 66, 712-719
- **STAMATOVIC, S.M. ; KEEP, R.F. ; KUNKEL, S.L. ; ANDJELKOVIC, A.V.** Potential role of MCP-1 in endothelial cell tight junction 'opening': signaling via Rho and Rho kinase. *Journal of cell science,* 2003, vol. 116, 4615-4628
- **DESAI, T.R. ; LEEPER, N.J. ; HYNES, K.L. ; GEWERTZ, B.L.** Interleukin-6 causes endothelial barrier dysfunction via the protein kinase C pathway. *The Journal of surgical research,* 2002, vol. 104, 118-123
- **BRAUN, D.A. ; FRIBOURG, M. ; SEALFON, S.C.** Cytokine response is determined by duration of receptor and signal transducers and activators of transcription 3 (STAT3) activation. *The Journal of biological chemistry,* 2013, vol. 288, 2986-2993
- **GALUN, E ; ROSE-JOHN, S.** The regenerative activity of interleukin-6. *Methods in molecular biology,* 2013, vol. 982, 59-77
- **WUESTEFELD, T. et al.** Interleukin-6/glycoprotein 130-dependent pathways are protective during liver regeneration. *The Journal of biological chemistry,* 2003, vol. 278, 11281-11288
- **BEST, D.H. ; BUTZ, G.M. ; COLEMAN, W.B.** Cytokine-dependent activation of small hepatocyte-like progenitor cells in retrorsine-induced rat liver injury. *Experimental and molecular pathology,* 2010, vol. 88, 7-14
- **FISCHER, M. et al.** I. A bioactive designer cytokine for human hematopoietic progenitor cell expansion. *Nature biotechnology,* 1997, vol. 15, 142-145
- **KABAT et al.** Sequences of proteins of immunological interest. 1987, vii-804

**SEQUENCE LISTING**

<110> UCB Biopharma SPRL

<120> PROTEINS AND USES

<130> PF0102-WO-PCT

<150> GB1617924.4
<151> 2016-10-24

<160> 154

<170> PatentIn version 3.5

<210> 1
<211> 125
<212> PRT
<213> Artificial Sequence

<220>

<223> VHH6

<400> 1

```
Asp Val Gln Phe Val Glu Ser Gly Gly Gly Ser Val His Ala Gly Gly
1               5                   10                  15

Ser Leu Arg Leu Asn Cys Ala Thr Ser Gly Tyr Ile Tyr Ser Thr Tyr
            20                  25                  30

Cys Met Gly Trp Phe Arg Gln Ala Pro Gly Lys Glu Arg Glu Gly Val
        35                  40                  45

Ala His Ile Tyr Thr Asn Ser Gly Arg Thr Tyr Tyr Ala Asp Ser Val
    50                  55                  60

Lys Gly Arg Phe Thr Ile Ser Gln Asp Asn Ala Lys Asn Thr Val Tyr
65                  70                  75                  80

Leu Gln Met Asn Ser Leu Lys Pro Glu Asp Thr Ala Ile Tyr Tyr Cys
                85                  90                  95

Ala Ala Arg Pro Ser Ile Arg Cys Ala Ser Phe Ser Ala Thr Glu Tyr
            100                 105                 110

Lys Asp Trp Gly Gln Gly Thr Gln Val Thr Val Ser Ser
        115                 120                 125
```

<210> 2
<211> 10
<212> PRT
<213> Artificial Sequence

<220>
<223> VHH6

<400> 2

```
Gly Tyr Ile Tyr Ser Thr Tyr Cys Met Gly
1               5                   10
```

<210> 3
<211> 17
<212> PRT
<213> Artificial Sequence

<220>
<223> VHH6 CDR2

<400> 3

His Ile Tyr Thr Asn Ser Gly Arg Thr Tyr Tyr Ala Asp Ser Val Lys
1               5                   10                  15

Gly

<210> 4
<211> 16
<212> PRT
<213> Artificial Sequence

<220>
<223> VHH6 CDR3

<400> 4

Arg Pro Ser Ile Arg Cys Ala Ser Phe Ser Ala Thr Glu Tyr Lys Asp
1               5                   10                  15

<210> 5
<211> 375
<212> DNA
<213> Artificial Sequence

<220>
<223> VHH6

<400> 5

gatgtgcagt tcgtggagtc tgggggaggc tcggtgcacg ctggagggtc tctaagactc 60

aactgtgcta cctctggata catttacagt acctattgca tgggatggtt ccgccaggct 120

ccagggaagg agcgtgaggg ggtcgcacat atttatacga atagtggacg cacatactat 180

gccgactccg tgaagggccg attcaccatc tcccaagaca acgccaagaa cacggtgtat 240

ctgcaaatga acagcctgaa acctgaggac acggccatct attactgtgc ggcccgtccc 300

agtattaggt gtgcttcctt cagcgccacc gaatataagg actggggcca ggggacccag 360

gtcaccgtct cctcg 375

<210> 6
<211> 30
<212> DNA
<213> Artificial Sequence

<220>
<223> VHH6

<400> 6
ggatacattt acagtaccta ttgcatggga 30

<210> 7
<211> 51
<212> DNA
<213> Artificial Sequence

<220>
<223> VHH6

<400> 7
catatttata cgaatagtgg acgcacatac tatgccgact ccgtgaaggg c 51

<210> 8
<211> 48
<212> DNA
<213> Artificial Sequence

<220>
<223> VHH6

<400> 8
cgtcccagta ttaggtgtgc ttccttcagc gccaccgaat ataaggac 48

<210> 9
<211> 187
<212> PRT
<213> Homo sapiens

<400> 9

```
Gly Ser Ala Pro Val Pro Pro Gly Glu Asp Ser Lys Asp Val Ala Ala
1               5                   10                  15

Pro His Arg Gln Pro Leu Thr Ser Ser Glu Arg Ile Asp Lys Gln Ile
            20                  25                  30

Arg Tyr Ile Leu Asp Gly Ile Ser Ala Leu Arg Lys Glu Thr Cys Asn
        35                  40                  45

Lys Ser Asn Met Cys Glu Ser Ser Lys Glu Ala Leu Ala Glu Asn Asn
    50                  55                  60

Leu Asn Leu Pro Lys Met Ala Glu Lys Asp Gly Cys Phe Gln Ser Gly
65                  70                  75                  80
```

```
Phe Asn Glu Glu Thr Cys Leu Val Lys Ile Ile Thr Gly Leu Leu Glu
                85                  90                  95

Phe Glu Val Tyr Leu Glu Tyr Leu Gln Asn Arg Phe Glu Ser Ser Glu
            100                 105                 110

Glu Gln Ala Arg Ala Val Gln Met Ser Thr Lys Val Leu Ile Gln Phe
        115                 120                 125

Leu Gln Lys Lys Ala Lys Asn Leu Asp Ala Ile Thr Thr Pro Asp Pro
    130                 135                 140

Thr Thr Asn Ala Ser Leu Leu Thr Lys Leu Gln Ala Gln Asn Gln Trp
145                 150                 155                 160

Leu Gln Asp Met Thr Thr His Leu Ile Leu Arg Ser Phe Lys Glu Phe
                165                 170                 175

Leu Gln Ser Ser Leu Arg Ala Leu Arg Gln Met
            180                 185
```

<210> 10
<211> 231
<212> PRT
<213> Homo sapiens

<400> 10

```
Gly Leu Ala Pro Arg Arg Cys Pro Ala Gln Glu Val Ala Arg Gly Ala
1               5                   10                  15

Gly Ala Gly Asp Val Pro Pro Glu Glu Pro Gln Leu Ser Cys Phe Arg
            20                  25                  30

Lys Ser Pro Leu Ser Asn Val Val Cys Glu Trp Gly Pro Arg Ser Thr
        35                  40                  45

Pro Ser Leu Thr Thr Lys Ala Val Leu Leu Val Arg Lys Phe Gln Asn
    50                  55                  60

Ser Pro Ala Glu Asp Phe Gln Glu Pro Cys Gln Tyr Ser Gln Glu Ser
65                  70                  75                  80

Gln Lys Phe Ser Cys Gln Leu Ala Val Pro Glu Gly Asp Ser Ser Phe
                85                  90                  95

Tyr Ile Val Ser Met Cys Val Ala Ser Ser Val Gly Ser Lys Phe Ser
            100                 105                 110
```

Lys Thr Gln Thr Phe Gln Gly Ala Gly Ile Leu Gln Pro Asp Pro Pro
115 120 125

Ala Asn Ile Thr Val Thr Ala Val Ala Arg Asn Pro Arg Trp Leu Ser
130 135 140

Val Thr Trp Gln Asp Pro His Ser Trp Asn Ser Ser Phe Tyr Arg Leu
145 150 155 160

Arg Phe Glu Leu Arg Tyr Arg Ala Glu Arg Ser Lys Thr Phe Thr Thr
165 170 175

Trp Met Val Lys Asp Leu Gln His His Ala Val Ile His Asp Ala Trp
180 185 190

Ser Gly Leu Arg His Val Val Gln Leu Arg Ala Gln Glu Glu Phe Gly
195 200 205

Gln Gly Glu Trp Ser Glu Trp Ser Pro Glu Ala Met Gly Thr Pro Trp
210 215 220

Thr Glu Ser Arg Ser Pro Pro
225 230

<210> 11
<211> 8
<212> PRT
<213> Artificial Sequence

<220>
<223> Fragment

<400> 11

Ser Ser Glu Arg Ile Asp Lys Gln
1 5

<210> 12
<211> 12
<212> PRT
<213> Artificial Sequence

<220>
<223> Protein fragment

<400> 12

Lys Asp Gly Cys Phe Gln Ser Cys Phe Asn Glu Glu
1 5 10

<210> 13
<211> 7

<212> PRT
<213> Artificial Sequence

<220>
<223> fragment

<400> 13

```
Leu Ser Val Thr Trp Gln Asp
1               5
```

<210> 14
<211> 13
<212> PRT
<213> Artificial Sequence

<220>
<223> fragment

<400> 14

```
Met Val Lys Asp Leu Gln His His Ala Val Ile His Asp
1               5                   10
```

<210> 15
<211> 6
<212> PRT
<213> Artificial Sequence

<220>
<223> fragment

<400> 15

```
Arg Ala Leu Arg Gln Met
1               5
```

<210> 16
<211> 18
<212> PRT
<213> Artificial Sequence

<220>
<223> fragment

<400> 16

```
Met Thr Thr His Leu Ile Leu Arg Ser Phe Lys Glu Phe Leu Gln Ser
1               5                   10                  15

Ser Leu
```

<210> 17
<211> 8
<212> PRT
<213> Artificial Sequence

<220>
<223> fragment

<400> 17

```
Pro Glu Gly Asp Ser Ser Phe Tyr
1               5
```

<210> 18
<211> 13
<212> PRT
<213> Artificial Sequence

<220>
<223> fragment

<400> 18

```
Arg Ala Gln Glu Glu Phe Gly Gln Gly Glu Trp Ser Glu
1               5                   10
```

<210> 19
<211> 18
<212> PRT
<213> Artificial Sequence

<220>
<223> fragment

<400> 19

```
Leu Gln Lys Lys Ala Lys Asn Leu Ala Ile Thr Thr Pro Asp Pro Thr
1               5                   10                  15

Thr Asn
```

<210> 20
<211> 10
<212> PRT
<213> Artificial Sequence

<220>
<223> fragment

<400> 20

```
Arg Lys Glu Thr Cys Asn Lys Ser Asn Met
1               5                   10
```

<210> 21
<211> 12
<212> PRT
<213> Artificial Sequence

<220>
<223> fragment

<400> 21

```
Ala Pro Val Pro Pro Gly Glu Asp Ser Lys Asp Val
1               5                   10
```

<210> 22
<211> 21
<212> PRT
<213> Artificial Sequence

<220>
<223> fragment

<400> 22

```
Pro Pro Gly Glu Asp Ser Lys Asp Val Ala Ala Pro His Arg Gln Pro
1               5                   10                  15

Leu Thr Ser Ser Glu
            20
```

<210> 23
<211> 11
<212> PRT
<213> Artificial Sequence

<220>
<223> fragment

<400> 23

```
Ser Ser Glu Arg Ile Asp Lys Gln Ile Arg Tyr
1               5                   10
```

<210> 24
<211> 10
<212> PRT
<213> Artificial Sequence

<220>
<223> fragment

<400> 24

```
Ser Glu Arg Ile Asp Lys Gln Ile Arg Tyr
1               5                   10
```

<210> 25
<211> 10
<212> PRT
<213> Artificial Sequence

<220>
<223> fragment

<400> 25

Arg Ile Asp Lys Gln Ile Arg Tyr Ile Leu
1 5 10

<210> 26
<211> 12
<212> PRT
<213> Artificial Sequence

<220>
<223> fragment

<400> 26

Ile Asp Lys Gln Ile Arg Tyr Ile Leu Asp Gly Ile
1 5 10

<210> 27
<211> 11
<212> PRT
<213> Artificial Sequence

<220>
<223> fragment

<400> 27

Ile Arg Tyr Ile Leu Asp Gly Ile Ser Ala Leu
1 5 10

<210> 28
<211> 12
<212> PRT
<213> Artificial Sequence

<220>
<223> fragment

<400> 28

Ala Leu Arg Lys Glu Thr Cys Asn Lys Ser Asn Met
1 5 10

<210> 29
<211> 11
<212> PRT
<213> Artificial Sequence

<220>
<223> fragment

<400> 29

Leu Arg Lys Glu Thr Cys Asn Lys Ser Asn Met
1 5 10

<210> 30
<211> 12
<212> PRT
<213> Artificial Sequence

<220>
<223> fragment

<400> 30

```
Arg Lys Glu Thr Cys Asn Lys Ser Asn Met Cys Glu
1               5                   10
```

<210> 31
<211> 21
<212> PRT
<213> Artificial Sequence

<220>
<223> fragment

<400> 31

```
Lys Glu Thr Cys Asn Lys Ser Asn Met Cys Glu Ser Ser Lys Glu Ala
1               5                   10                  15

Leu Ala Glu Asn Asn
            20
```

<210> 32
<211> 15
<212> PRT
<213> Artificial Sequence

<220>
<223> fragment

<400> 32

```
Glu Thr Cys Asn Lys Ser Asn Met Cys Glu Ser Ser Lys Glu Ala
1               5                   10                  15
```

<210> 33
<211> 24
<212> PRT
<213> Artificial Sequence

<220>
<223> fragment

<400> 33

Ser Ser Lys Glu Ala Leu Ala Glu Asn Asn Leu Asn Leu Pro Lys Met
1               5                   10                  15

Ala Glu Lys Asp Gly Cys Phe Gln
            20

<210> 34
<211> 11
<212> PRT
<213> Artificial Sequence

<220>
<223> fragment

<400> 34

Ser Lys Glu Ala Leu Ala Glu Asn Asn Leu Asn
1               5                   10

<210> 35
<211> 23
<212> PRT
<213> Artificial Sequence

<220>
<223> fragment

<400> 35

Ser Lys Glu Ala Leu Ala Glu Asn Asn Leu Asn Leu Pro Lys Met Ala
1               5                   10                  15

Glu Lys Asp Gly Cys Phe Gln
            20

<210> 36
<211> 11
<212> PRT
<213> Artificial Sequence

<220>
<223> fragment

<400> 36

Glu Ala Leu Ala Glu Asn Asn Leu Asn Leu Pro
1               5                   10

<210> 37
<211> 10
<212> PRT
<213> Artificial Sequence

<220>
<223> fragment

<400> 37

```
Ala Glu Asn Asn Leu Asn Leu Pro Lys Met
1               5                   10
```

<210> 38
<211> 17
<212> PRT
<213> Artificial Sequence

<220>
<223> fragment

<400> 38

```
Ala Glu Asn Asn Leu Asn Leu Pro Lys Met Ala Glu Lys Asp Gly Cys
1               5                   10                  15

Phe
```

<210> 39
<211> 22
<212> PRT
<213> Artificial Sequence

<220>
<223> fragment

<400> 39

```
Glu Asn Asn Leu Asn Leu Pro Lys Met Ala Glu Lys Asp Gly Cys Phe
1               5                   10                  15

Gln Ser Gly Phe Asn Glu
            20
```

<210> 40
<211> 8
<212> PRT
<213> Artificial Sequence

<220>
<223> fragment

<400> 40

```
Asn Asn Leu Asn Leu Pro Lys Met
1               5
```

<210> 41
<211> 11
<212> PRT
<213> Artificial Sequence

<220>
<223> fragment

<400> 41

```
Asn Asn Leu Asn Leu Pro Lys Met Ala Glu Lys
1               5                   10
```

<210> 42
<211> 23
<212> PRT
<213> Artificial Sequence

<220>
<223> fragment

<400> 42

```
Asn Asn Leu Asn Leu Pro Lys Met Ala Glu Lys Asp Gly Cys Phe Gln
1               5                   10                  15

Ser Gly Phe Asn Glu Glu Thr
            20
```

<210> 43
<211> 7
<212> PRT
<213> Artificial Sequence

<220>
<223> fragment

<400> 43

```
Ala Glu Lys Asp Gly Cys Phe
1               5
```

<210> 44
<211> 12
<212> PRT
<213> Artificial Sequence

<220>
<223> fragment

<400> 44

```
Lys Asp Gly Cys Phe Gln Ser Gly Phe Asn Glu Glu
1               5                   10
```

<210> 45
<211> 10
<212> PRT
<213> Artificial Sequence

<220>
<223> fragment

<400> 45

Gln Ser Gly Phe Asn Glu Glu Thr Cys Leu
1 5 10

<210> 46
<211> 8
<212> PRT
<213> Artificial Sequence

<220>
<223> fragment

<400> 46

Val Lys Ile Ile Thr Gly Leu Leu
1 5

<210> 47
<211> 9
<212> PRT
<213> Artificial Sequence

<220>
<223> fragment

<400> 47

Val Lys Ile Ile Thr Gly Leu Leu Glu
1 5

<210> 48
<211> 7
<212> PRT
<213> Artificial Sequence

<220>
<223> fragment

<400> 48

Ile Ile Thr Gly Leu Leu Glu
1 5

<210> 49
<211> 11
<212> PRT
<213> Artificial Sequence

<220>
<223> fragment

<400> 49

Leu Glu Tyr Leu Gln Asn Arg Phe Glu Ser Ser
1 5 10

<210> 50
<211> 11
<212> PRT

<213> Artificial Sequence

<220>
<223> fragment

<400> 50

```
Glu Tyr Leu Gln Asn Arg Phe Glu Ser Ser Glu
1               5                   10
```

<210> 51
<211> 12
<212> PRT
<213> Artificial Sequence

<220>
<223> fragment

<400> 51

```
Glu Tyr Leu Gln Asn Arg Phe Glu Ser Ser Glu Glu
1               5                   10
```

<210> 52
<211> 14
<212> PRT
<213> Artificial Sequence

<220>
<223> fragment

<400> 52

```
Glu Tyr Leu Gln Asn Arg Phe Glu Ser Ser Glu Glu Gln Ala
1               5                   10
```

<210> 53
<211> 22
<212> PRT
<213> Artificial Sequence

<220>
<223> fragment

<400> 53

```
Glu Tyr Leu Gln Asn Arg Phe Glu Ser Ser Glu Glu Gln Ala Arg Ala
1               5                   10                  15

Val Gln Met Ser Thr Lys
            20
```

<210> 54
<211> 11
<212> PRT
<213> Artificial Sequence

<220>
<223> fragment

<400> 54

Tyr Leu Gln Asn Arg Phe Glu Ser Ser Glu Glu
1 5 10

<210> 55
<211> 13
<212> PRT
<213> Artificial Sequence

<220>
<223> fragment

<400> 55

Tyr Leu Gln Asn Arg Phe Glu Ser Ser Glu Glu Gln Ala
1 5 10

<210> 56
<211> 11
<212> PRT
<213> Artificial Sequence

<220>
<223> fragment

<400> 56

Arg Phe Glu Ser Ser Glu Glu Gln Ala Arg Ala
1 5 10

<210> 57
<211> 7
<212> PRT
<213> Artificial Sequence

<220>
<223> fragment

<400> 57

Glu Ser Ser Glu Glu Gln Ala
1 5

<210> 58
<211> 11
<212> PRT
<213> Artificial Sequence

<220>
<223> fragment

<400> 58

Glu Ser Ser Glu Glu Gln Ala Arg Ala Val Gln
1 5 10

<210> 59
<211> 8
<212> PRT
<213> Artificial Sequence

<220>
<223> fragment

<400> 59

Glu Glu Gln Ala Arg Ala Val Gln

1 5

<210> 60
<211> 8
<212> PRT
<213> Artificial Sequence

<220>
<223> fragment

<400> 60

Val Gln Met Ser Thr Lys Val Leu
1 5

<210> 61
<211> 8
<212> PRT
<213> Artificial Sequence

<220>
<223> fragment

<400> 61

Val Gln Met Ser Thr Lys Val Leu
1 5

<210> 62
<211> 7
<212> PRT
<213> Artificial Sequence

<220>
<223> fragment

<400> 62

Lys Val Leu Ile Gln Phe Leu
1 5

<210> 63
<211> 13
<212> PRT
<213> Artificial Sequence

<220>
<223> fragment

<400> 63

```
Ile Gln Phe Leu Gln Lys Lys Ala Lys Asn Leu Asp Ala
1               5                   10
```

<210> 64
<211> 8
<212> PRT
<213> Artificial Sequence

<220>
<223> fragment

<400> 64

```
Leu Gln Lys Lys Ala Lys Asn Leu
1               5
```

<210> 65
<211> 10
<212> PRT
<213> Artificial Sequence

<220>
<223> fragment

<400> 65

```
Leu Gln Lys Lys Ala Lys Asn Leu Asp Ala
1               5                   10
```

<210> 66
<211> 19
<212> PRT
<213> Artificial Sequence

<220>
<223> fragment

<400> 66

```
Leu Gln Lys Lys Ala Lys Asn Leu Asp Ala Ile Thr Thr Pro Asp Pro
1               5                   10                  15

Thr Thr Asn
```

<210> 67
<211> 20
<212> PRT

<213> Artificial Sequence

<220>
<223> fragment

<400> 67

```
Leu Gln Lys Lys Ala Lys Asn Leu Asp Ala Ile Thr Thr Pro Asp Pro
1               5                   10                  15

Thr Thr Asn Ala
            20
```

<210> 68
<211> 22
<212> PRT
<213> Artificial Sequence

<220>
<223> fragment

<400> 68

```
Leu Gln Lys Lys Ala Lys Asn Leu Asp Ala Ile Thr Thr Pro Asp Pro
1               5                   10                  15

Thr Thr Asn Ala Ser Leu
            20
```

<210> 69
<211> 15
<212> PRT
<213> Artificial Sequence

<220>
<223> fragment

<400> 69

```
Lys Ala Lys Asn Leu Asp Ala Ile Thr Thr Pro Asp Pro Thr Thr
1               5                   10                  15
```

<210> 70
<211> 12
<212> PRT
<213> Artificial Sequence

<220>
<223> fragment

<400> 70

```
Ile Thr Thr Pro Asp Pro Thr Thr Asn Ala Ser Leu
1               5                   10
```

<210> 71

<211> 8
<212> PRT
<213> Artificial Sequence

<220>
<223> fragment

<400> 71

```
Leu Thr Lys Leu Gln Ala Gln Asn
1               5
```

<210> 72
<211> 8
<212> PRT
<213> Artificial Sequence

<220>
<223> fragment

<400> 72

```
Leu Thr Lys Leu Gln Ala Gln Asn
1               5
```

<210> 73
<211> 11
<212> PRT
<213> Artificial Sequence

<220>
<223> fragment

<400> 73

```
Leu Thr Lys Leu Gln Ala Gln Asn Gln Trp Leu
1               5                   10
```

<210> 74
<211> 13
<212> PRT
<213> Artificial Sequence

<220>
<223> fragment

<400> 74

```
Leu Thr Lys Leu Gln Ala Gln Asn Gln Trp Leu Gln Asp
1               5                   10
```

<210> 75
<211> 7
<212> PRT
<213> Artificial Sequence

<220>
<223> fragement

<400> 75

Gln Ala Gln Asn Gln Trp Leu
1 5

<210> 76
<211> 7
<212> PRT
<213> Artificial Sequence

<220>
<223> fragment

<400> 76

Gln Asp Met Thr Thr His Leu
1 5

<210> 77
<211> 9
<212> PRT
<213> Artificial Sequence

<220>
<223> fragment

<400> 77

Gln Asp Met Thr Thr His Leu Ile Leu
1 5

<210> 78
<211> 7
<212> PRT
<213> Artificial Sequence

<220>
<223> fragment

<400> 78

Met Thr Thr His Leu Ile Leu
1 5

<210> 79
<211> 7
<212> PRT
<213> Artificial Sequence

<220>
<223> fragment

<400> 79

Ile Leu Arg Ser Phe Lys Glu
1 5

<210> 80

<211> 8
<212> PRT
<213> Artificial Sequence

<220>
<223> fragment

<400> 80

Ile Leu Arg Ser Phe Lys Glu Phe
1 5

<210> 81
<211> 9
<212> PRT
<213> Artificial Sequence

<220>
<223> fragment

<400> 81

Ile Leu Arg Ser Phe Lys Glu Phe Leu
1 5

<210> 82
<211> 7
<212> PRT
<213> Artificial Sequence

<220>
<223> fragment

<400> 82

Arg Ser Phe Lys Glu Phe Leu
1 5

<210> 83
<211> 11
<212> PRT
<213> Artificial Sequence

<220>
<223> fragment

<400> 83

Arg Ser Phe Lys Glu Phe Leu Gln Ser Ser Leu
1 5 10

<210> 84
<211> 21
<212> PRT
<213> Artificial Sequence

<220>
<223> fragment

<400> 84

```
His Glu Asn Leu Tyr Phe Gln Gly Leu Ala Pro Arg Arg Cys Pro Ala
1               5                   10                  15

Gln Glu Val Ala Arg
            20
```

<210> 85
<211> 10
<212> PRT
<213> Artificial Sequence

<220>
<223> fragment

<400> 85

```
Gly Leu Ala Pro Arg Arg Cys Pro Ala Gln
1               5                   10
```

<210> 86
<211> 11
<212> PRT
<213> Artificial Sequence

<220>
<223> fragment

<400> 86

```
Gly Leu Ala Pro Arg Arg Cys Pro Ala Gln Glu
1               5                   10
```

<210> 87
<211> 13
<212> PRT
<213> Artificial Sequence

<220>
<223> fragment

<400> 87

```
Gly Leu Ala Pro Arg Arg Cys Pro Ala Gln Glu Val Ala
1               5                   10
```

<210> 88
<211> 19
<212> PRT
<213> Artificial Sequence

<220>
<223> fragment

<400> 88

Arg Cys Pro Ala Gln Glu Val Ala Arg Gly Ala Gly Ala Gly Asp Val
1 5 10 15

Pro Pro Glu

<210> 89
<211> 22
<212> PRT
<213> Artificial Sequence

<220>
<223> fragment

<400> 89

Ala Gln Glu Val Ala Arg Gly Ala Gly Ala Gly Asp Val Pro Pro Glu
1 5 10 15

Glu Pro Gln Leu Ser Cys
20

<210> 90
<211> 14
<212> PRT
<213> Artificial Sequence

<220>
<223> fragment

<400> 90

Gln Glu Val Ala Arg Gly Ala Gly Ala Gly Asp Val Pro Pro
1 5 10

<210> 91
<211> 12
<212> **PRT**
<213> **Artificial** Sequence

<220>
<223> fragment

<400> 91

Glu Val Ala Arg Gly Ala Gly Ala Gly Asp Val Pro
1 5 10

<210> 92
<211> 17
<212> PRT
<213> Artificial Sequence

<220>
<223> fragment

<400> 92

Val Ala Arg Gly Ala Gly Ala Gly Asp Val Pro Pro Glu Glu Pro Gln
1 5 10 15

Leu

<210> 93
<211> 15
<212> PRT
<213> Artificial Sequence

<220>
<223> fragment

<400> 93

Arg Gly Ala Gly Ala Gly Asp Val Pro Pro Glu Glu Pro Gln Leu

1 5 10 15

<210> 94
<211> 19
<212> PRT
<213> Artificial Sequence

<220>
<223> fragment

<400> 94

Asp Val Pro Pro Glu Glu Pro Gln Leu Ser Cys Phe Arg Lys Ser Pro
1 5 10 15

Leu Ser Asn

<210> 95
<211> 7
<212> PRT
<213> Artificial Sequence

<220>
<223> fragment

<400> 95

Pro Pro Glu Glu Pro Gln Leu
1 5

<210> 96
<211> 9
<212> PRT
<213> Artificial Sequence

<220>
<223> fragment

<400> 96

```
Phe Arg Lys Ser Pro Leu Ser Asn Val
1               5
```

<210> 97
<211> 19
<212> PRT
<213> Artificial Sequence

<220>
<223> fragment

<400> 97

```
Lys Ser Pro Leu Ser Asn Val Val Cys Glu Trp Gly Pro Arg Ser Thr
1               5                   10                  15

            Pro Ser Leu
```

<210> 98
<211> 13
<212> PRT
<213> Artificial Sequence

<220>
<223> fragment

<400> 98

```
Val Val Cys Glu Trp Gly Pro Arg Ser Thr Pro Ser Leu
1               5                   10
```

<210> 99
<211> 12
<212> PRT
<213> Artificial Sequence

<220>
<223> fragment

<400> 99

```
Val Cys Glu Trp Gly Pro Arg Ser Thr Pro Ser Leu
1               5                   10
```

<210> 100
<211> 15
<212> PRT
<213> Artificial Sequence

<220>
<223> fragment

<400> 100

Trp Gly Pro Arg Ser Thr Pro Ser Leu Thr Thr Lys Ala Val Leu
1 5 10 15

<210> 101
<211> 7
<212> PRT
<213> Artificial Sequence

<220>
<223> fragment

<400> 101

Thr Thr Lys Ala Val Leu Leu
1 5

<210> 102
<211> 12
<212> PRT
<213> Artificial Sequence

<220>
<223> fragment

<400> 102

Leu Val Arg Lys Phe Gln Asn Ser Pro Ala Glu Asp
1 5 10

<210> 103
<211> 11
<212> PRT
<213> Artificial Sequence

<220>
<223> fragment

<400> 103

Val Arg Lys Phe Gln Asn Ser Pro Ala Glu Asp
1 5 10

<210> 104
<211> 11
<212> PRT
<213> Artificial Sequence

<220>
<223> fragment

<400> 104

Gln Asn Ser Pro Ala Glu Asp Phe Gln Glu Pro
1 5 10

<210> 105
<211> 20
<212> PRT

<213> Artificial Sequence

<220>
<223> fragment

<400> 105

```
Ala Glu Asp Phe Gln Glu Pro Cys Gln Tyr Ser Gln Glu Ser Gln Lys
1               5                   10                  15

Phe Ser Cys Gln
            20
```

<210> 106
<211> 7
<212> PRT
<213> Artificial Sequence

<220>
<223> fragment

<400> 106

```
Asp Phe Gln Glu Pro Cys Gln
1               5
```

<210> 107
<211> 19
<212> PRT
<213> Artificial Sequence

<220>
<223> fragment

<400> 107

```
Cys Gln Tyr Ser Gln Glu Ser Gln Lys Phe Ser Cys Gln Leu Ala Val
1               5                   10                  15

Pro Glu Gly
```

<210> 108
<211> 18
<212> PRT
<213> Artificial Sequence

<220>
<223> fragment

<400> 108

```
Gln Tyr Ser Gln Glu Ser Gln Lys Phe Ser Cys Gln Leu Ala Val Pro
1               5                   10                  15

Glu Gly
```

<210> 109
<211> 11
<212> PRT
<213> Artificial Sequence

<220>
<223> fragment

<400> 109

```
Tyr Ser Gln Glu Ser Gln Lys Phe Ser Cys Gln
1               5                   10
```

<210> 110
<211> 7
<212> PRT
<213> Artificial Sequence

<220>
<223> fragment

<400> 110

```
Ser Gln Lys Phe Ser Cys Gln
1               5
```

<210> 111
<211> 14
<212> PRT
<213> Artificial Sequence

<220>
<223> fragment

<400> 111

```
Cys Gln Leu Ala Val Pro Glu Gly Asp Ser Ser Phe Tyr Ile
1               5                   10
```

<210> 112
<211> 10
<212> PRT
<213> Artificial Sequence

<220>
<223> fragment

<400> 112

```
Leu Ala Val Pro Glu Gly Asp Ser Ser Phe
1               5                   10
```

<210> 113
<211> 7
<212> PRT
<213> Artificial Sequence

<220>

<223> fragment

<400> 113

```
Pro Glu Gly Asp Ser Ser Phe
1               5
```

<210> 114
<211> 10
<212> PRT
<213> Artificial Sequence

<220>
<223> fragment

<400> 114

```
Cys Val Ala Ser Ser Val Gly Ser Lys Phe
1               5                   10
```

<210> 115
<211> 8
<212> PRT
<213> Artificial Sequence

<220>
<223> fragment

<400> 115

```
Ala Ser Ser Val Gly Ser Lys Phe
1               5
```

<210> 116
<211> 11
<212> PRT
<213> Artificial Sequence

<220>
<223> fragment

<400> 116

```
Leu Gln Pro Asp Pro Pro Ala Asn Ile Thr Val
1               5                   10
```

<210> 117
<211> 10
<212> PRT
<213> Artificial Sequence

<220>
<223> fragment

<400> 117

```
Ala Asn Ile Thr Val Thr Ala Val Ala Arg
1               5                   10
```

<210> 118
<211> 11
<212> PRT
<213> Artificial Sequence

<220>
<223> fragment

<400> 118

**Thr Ala Val Ala Arg Asn Pro Arg Trp Leu Ser**
**1 5 10**

<210> 119
<211> 8
<212> PRT
<213> Artificial Sequence

<220>
<223> fragment

<400> 119

**Ala Val Ala Arg Asn Pro Arg Trp**
**1 5**

<210> 120
<211> 7
<212> PRT
<213> Artificial Sequence

<220>
<223> fragment

<400> 120

**Val Ala Arg Asn Pro Arg Trp**
**1 5**

<210> 121
<211> 10
<212> **PRT**
<213> **Artificial** Sequence

<220>
<223> fragment

<400> 121

**Val Ala Arg Asn Pro Arg Trp Leu Ser Val**
**1 5 10**

<210> 122
<211> 11
<212> **PRT**
<213> **Artificial** Sequence

<220>

<223> fragment

<400> 122

Val Ala Arg Asn Pro Arg Trp Leu Ser Val Thr
1 5 10

<210> 123
<211> 12
<212> PRT
<213> Artificial Sequence

<220>
<223> fragment

<400> 123

Thr Trp Gln Asp Pro His Ser Trp Asn Ser Ser Phe
1 5 10

<210> 124
<211> 9
<212> PRT
<213> Artificial Sequence

<220>
<223> fragment

<400> 124

Asp Pro His Ser Trp Asn Ser Ser Phe
1 5

<210> 125
<211> 7
<212> PRT
<213> Artificial Sequence

<220>
<223> fragment

<400> 125

Tyr Arg Leu Arg Phe Glu Leu
1 5

<210> 126
<211> 11
<212> PRT
<213> Artificial Sequence

<220>
<223> fragment

<400> 126

Glu Leu Arg Tyr Arg Ala Glu Arg Ser Lys Thr
1 5 10

<210> 127
<211> 9
<212> PRT
<213> Artificial Sequence

<220>
<223> fragment

<400> 127

Arg Tyr Arg Ala Glu Arg Ser Lys Thr
1               5

<210> 128
<211> 14
<212> PRT
<213> Artificial Sequence

<220>
<223> fragment

<400> 128

Ser Lys Thr Phe Thr Thr Trp Met Val Lys Asp Leu Gln His
1               5                   10

<210> 129
<211> 17
<212> PRT
<213> Artificial Sequence

<220>
<223> fragment

<400> 129

Met Val Lys Asp Leu Gln His His Ala Val Ile His Asp Ala Trp Ser
1               5                   10                  15

Gly

<210> 130
<211> 24
<212> PRT
<213> Artificial Sequence

<220>
<223> fragment

<400> 130

Met Val Lys Asp Leu Gln His His Ala Val Ile His Asp Ala Trp Ser
1               5                   10                  15

Gly Leu Arg His Val Val Gln Leu
            20

<210> 131
<211> 9
<212> PRT
<213> Artificial Sequence

<220>
<223> fragment

<400> 131

```
Ser Gly Leu Arg His Val Val Gln Leu
1               5
```

<210> 132
<211> 7
<212> PRT
<213> Artificial Sequence

<220>
<223> fragment

<400> 132

```
Leu Arg His Val Val Gln Leu
1               5
```

<210> 133
<211> 8
<212> PRT
<213> Artificial Sequence

<220>
<223> fragment

<400> 133

```
Arg His Val Val Gln Leu Arg Ala
1               5
```

<210> 134
<211> 10
<212> PRT
<213> Artificial Sequence

<220>
<223> fragment

<400> 134

```
Arg Ala Gln Glu Glu Phe Gly Gln Gly Glu
1               5                   10
```

<210> 135
<211> 11
<212> PRT
<213> Artificial Sequence

<220>

<223> fragment

<400> 135

Arg Ala Gln Glu Glu Phe Gly Gln Gly Glu Trp
1 5 10

<210> 136
<211> 19
<212> PRT
<213> Artificial Sequence

<220>
<223> fragment

<400> 136

Glu Glu Phe Gly Gln Gly Glu Trp Ser Glu Trp Ser Pro Glu Ala Met
1 5 10 15

Gly Thr Pro

<210> 137
<211> 17
<212> PRT
<213> Artificial Sequence

<220>
<223> fragment

<400> 137

Gly Gln Gly Glu Trp Ser Glu Trp Ser Pro Glu Ala Met Gly Thr Pro
1 5 10 15

Trp

<210> 138
<211> 8
<212> PRT
<213> Artificial Sequence

<220>
<223> fragment

<400> 138

Trp Ser Glu Trp Ser Pro Glu Ala
1 5

<210> 139
<211> 15
<212> PRT
<213> Artificial Sequence

<220>
<223> fragment

<400> 139

Trp Ser Glu Trp Ser Pro Glu Ala Met Gly Thr Pro Trp Thr Glu
1 5 10 15

<210> 140
<211> 20
<212> PRT
<213> Artificial Sequence

<220>
<223> fragment

<400> 140

Trp Ser Glu Trp Ser Pro Glu Ala Met Gly Thr Pro Trp Thr Glu Ser
1 5 10 15

Arg Ser Pro Pro 20

<210> 141
<211> 7
<212> PRT
<213> Artificial Sequence

<220>
<223> fragment

<400> 141

Ser Glu Trp Ser Pro Glu Ala
1 5

<210> 142
<211> 14
<212> PRT
<213> Artificial Sequence

<220>
<223> fragment

<400> 142

Ser Glu Trp Ser Pro Glu Ala Met Gly Thr Pro Trp Thr Glu
1 5 10

<210> 143
<211> 19
<212> PRT
<213> Artificial Sequence

<220>
<223> fragment

<400> 143

Ser Glu Trp Ser Pro Glu Ala Met Gly Thr Pro Trp Thr Glu Ser Arg
1               5                   10                  15

Ser Pro Pro

<210> 144
<211> 12
<212> PRT
<213> Artificial Sequence

<220>
<223> fragment

<400> 144

Trp Ser Pro Glu Ala Met Gly Thr Pro Trp Thr Glu
1               5                   10

<210> 145
<211> 15
<212> PRT
<213> Artificial Sequence

<220>
<223> fragment

<400> 145

Pro Glu Ala Met Gly Thr Pro Trp Thr Glu Ser Arg Ser Pro Pro
1               5                   10                  15

<210> 146
<211> 7
<212> PRT
<213> Artificial Sequence

<220>
<223> fragment

<400> 146

Met Gly Thr Pro Trp Thr Glu
1               5

<210> 147
<211> 125
<212> PRT
<213> Artificial Sequence

<220>
<223> hVHH6

<400> 147

Glu Val Gln Phe Val Glu Ser Gly Gly Gly Leu Val Gln Pro Gly Gly
1               5                   10                  15

Ser Leu Arg Leu Ser Cys Ala Thr Ser Gly Tyr Ile Tyr Ser Thr Tyr
            20                  25                  30

Cys Met Gly Trp Phe Arg Gln Ala Pro Gly Lys Glu Arg Glu Gly Val
        35                  40                  45

Ala His Ile Tyr Thr Asn Ser Gly Arg Thr Tyr Tyr Ala Asp Ser Val
    50                  55                  60

Lys Gly Arg Phe Thr Ile Ser Gln Asp Asn Ala Lys Asn Thr Val Tyr
65                  70                  75                  80

Leu Gln Met Asn Ser Leu Arg Ala Glu Asp Thr Ala Val Tyr Tyr Cys
                85                  90                  95

Ala Ala Arg Pro Ser Ile Arg Cys Ala Ser Phe Ser Ala Thr Glu Tyr
            100                 105                 110

Lys Asp Trp Gly Gln Gly Thr Leu Val Thr Val Ser Ser
        115                 120                 125

<210> 148
<211> 125
<212> PRT
<213> Artificial Sequence

<220>
<223> hVHH6

<400> 148

```
Glu Val Gln Phe Val Glu Ser Gly Gly Gly Leu Val Gln Pro Gly Gly
1               5                   10                  15

Ser Leu Arg Leu Ser Cys Ala Thr Ser Gly Tyr Ile Tyr Ser Thr Tyr
            20                  25                  30

Ser Met Gly Trp Phe Arg Gln Ala Pro Gly Lys Glu Arg Glu Gly Val
        35                  40                  45

Ala His Ile Tyr Thr Asn Ser Gly Arg Thr Tyr Tyr Ala Asp Ser Val
    50                  55                  60

Lys Gly Arg Phe Thr Ile Ser Gln Asp Asn Ala Lys Asn Thr Val Tyr
65                  70                  75                  80

Leu Gln Met Asn Ser Leu Arg Ala Glu Asp Thr Ala Val Tyr Tyr Cys
                85                  90                  95

Ala Ala Arg Pro Ser Ile Arg Ser Ala Ser Phe Ser Ala Thr Glu Tyr
            100                 105                 110

Lys Asp Trp Gly Gln Gly Thr Leu Val Thr Val Ser Ser
        115                 120                 125
```

<210> 149
<211> 10
<212> PRT
<213> Artificial Sequence

<220>
<223> hVHH6

<400> 149

```
Gly Tyr Ile Tyr Ser Thr Tyr Cys Met Gly
1               5                   10
```

<210> 150
<211> 10
<212> PRT
<213> Artificial Sequence

<220>
<223> hVHH6

<400> 150

```
Gly Tyr Ile Tyr Ser Thr Tyr Ser Met Gly
1               5                   10
```

<210> 151
<211> 17

<212> PRT
<213> Artificial Sequence

<220>
<223> hVHH6

<400> 151

His Ile Tyr Thr Asn Ser Gly Arg Thr Tyr Tyr Ala Asp Ser Val Lys
1               5                   10                  15

Gly

<210> 152
<211> 16
<212> PRT
<213> Artificial Sequence

<220>
<223> hVHH6

<400> 152

Arg Pro Ser Ile Arg Cys Ala Ser Phe Ser Ala Thr Glu Tyr Lys Asp
1               5                   10                  15

<210> 153
<211> 16
<212> PRT
<213> Artificial Sequence

<220>
<223> hVHH6

<400> 153

Arg Pro Ser Ile Arg Ser Ala Ser Phe Ser Ala Thr Glu Tyr Lys Asp
1               5                   10                  15

<210> 154
<211> 113
<212> PRT
<213> Homo sapiens

<400> 154

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Trp Met His Trp Val Arg Gln Ala Pro Gly Lys Gly Leu Val Trp Val
        35                  40                  45

Ser Arg Ile Asn Ser Asp Gly Ser Ser Thr Ser Tyr Ala Asp Ser Val
    50                  55                  60

Lys Gly Arg Phe Thr Ile Ser Arg Asp Asn Ala Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Tyr Phe Asp Tyr Trp Gly Gln Gly Thr Leu Val Thr Val Ser
            100                 105                 110

Ser
```